# EUROPEAN PATENT APPLICATION

(11) **EP 4 317 142 A1**
(43) Date of publication of application: **07.02.2024**
(21) Application number: 22774119.6
(22) Date of filing: 17.03.2022
(51) Int. Cl.: C07D 401/04, C07D 405/04, C07D 487/04, A61K 31/496, A61K 31/4427, A61P 3/10

(54) **THIOPHENE GLP-1 RECEPTOR AGONIST AND USE THEREOF**

(30) Priority: 22.03.2021 CN 202110302981; 15.07.2021 CN 202110798187; 18.08.2021 CN 202110947386
(71) Applicant: Hangzhou Zhongmeihuadong Pharmaceutical Co., Ltd., GongShu District HangZhou, Zhejiang 310011 (CN)
(72) Inventor: ZHAI, Wenqiang, HangZhou, Zhejiang 310011 (CN); ZHANG, Zhimin, HangZhou, Zhejiang 310011 (CN); GUO, Liubin, HangZhou, Zhejiang 310011 (CN); LIU, Dongzhou, HangZhou, Zhejiang 310011 (CN); PAN, Hao, HangZhou, Zhejiang 310011 (CN)
(74) Representative: Nederlandsch Octrooibureau
(86) International application number: PCT/CN2022/081466
(87) International publication number: WO 2022/199458

(57) **Abstract**

Provided are a series of thiophene GLP-I receptor agonist com-pounds, a preparation method therefor and the pharmaceutical use thereof. The compounds can be used for preparing drugs for treating or preventing GLP-1-mediateddiseases and related diseases.

## Description

### Field of the Invention

The invention relates to the pharmaceutical field, in particular to a GLP-1 receptor agonist compound and a method for preparing the same, as well as the use of the compound in the preparation of a medicament for the treatment or prevention of GLP-1 mediated diseases and related diseases.

### Background of the Invention

Diabetes is a chronic comprehensive disease mainly characterized by glucose metabolism disorder due to absolute or relative deficiency of insulin or decreased sensitivity of target cells to insulin, and can be divided into type 1 diabetes and type 2 diabetes. Among them, type 2 diabetes is an adult-onset diabetes, which is an endocrine disease mainly characterized by chronic increase in blood sugar level due to insulin resistance and/or inadequate insulin secretion. Patients with type 2 diabetes account for more than 90% of diabetic patients. According to the global mapping of diabetes, there are about 425 million diabetic patients around the world in 2017, among which Chinese diabetic patients rank first with a number of about 114.4 million. It is estimated that by 2045, there will be 629 million diabetic patients around the world. It can be seen that diabetes is a very common chronic disease all over the world.

At present, drugs used to treat type 2 diabetes mainly include the following kinds of drugs: insulin secretagogues, metformins, α-glycosidase inhibitors, insulin sensitizers, sodium-glucose cotransporter 2 inhibitors, dipeptidyl peptidase 4 (DPP-4) inhibitors, GLP-1 receptor agonists, insulins and analogues thereof, among which, insulins and GLP-1 receptor agonists are ones of the most effective drugs for treating diabetes. Insulin formulations are still the most widely used diabetes drugs all over the world, and about 30-40% of patients with type 2 diabetes finally need insulins. GLP-1 formulations mainly include exenatide, liraglutide, somalutide, etc., and are suitable for patients with type 2 diabetes whose blood sugar cannot be fully controlled by the combination of metformin and sulfonylurea, and so on. However, current insulin formulations and GLP-1 formulations are substantially polypeptides and injectable formulations. There are still many limitations in administration even for oral somarutide. Thus, it is still necessary to further develop small molecule GLP-1 receptor agonists.

GLP-1 stimulates insulin secretion in a glucose dependent manner, and inhibits glucagon secretion in a glucose dependent manner, so there is no risk of hypoglycemia. GLP-1 can increase the production of insulin by β cells and improve the response of β cells to glucose. GLP-1 may delay gastric emptying and reduce food intake, and therefore can lead to weight loss. In addition, GLP-1 also has the unique effect of cardiovascular benefits. GLP-1 receptor agonists are used in the transitional stage between oral hypoglycemic drugs and insulins in clinical practice, can be used in combination with other drugs, and have become the fastest growing hypoglycemic drugs in the past five years and will be the most promising hypoglycemic drugs in the future.

Other conditions associated with type 2 diabetes include diabetic nephropathy, diabetic complications of the eye (diabetic retinopathy, diabetes-related uveitis, diabetic cataract), diabetic foot, diabetic cardiovascular complications, diabetic cerebrovascular disease, diabetic neuropathy, obesity, and hypertension.

GLP-1 receptor agonists, as very potential drugs, are marketed mostly in forms for administration by injection at present. Oral small molecule GLP-1 receptor agonists can improve patient compliance, representing the development trend of GLP-1 receptor agonists in the future. At present, the research and development progress of known small molecule GLP-1 receptor agonists are as follows:
WO2009111700A2 discloses a series of oxadiazoanthracenes as GLP-1 receptor agonist compounds; WO2010114824A1 discloses substituted azoanthracene derivatives as GLP-1 receptor agonist compounds; WO2017078352A1 discloses a series of clohexene derivatives as GLP-1 receptor agonist compounds; KR1020180101671A discloses a series of heteroaryl substituted pyridine[1,2-a]imidazole derivatives as GLP-1 receptor agonist compounds; WO2018056453A1 discloses a series of pyrazolopyridine derivatives as GLP-1 receptor agonist compounds; and WO2018109607A1 discloses a series of GLP-1 receptor agonist compounds similar to the present invention. With respect to the compounds disclosed in WO2018109607A1, there are several similar patent applications for GLP-1 receptor agonist compounds, such as WO2020103815A1, WO2020207474A1, WO2021018023A1, etc.

### Summary of the Invention

The invention provides a series of compounds as represented by Formula I: 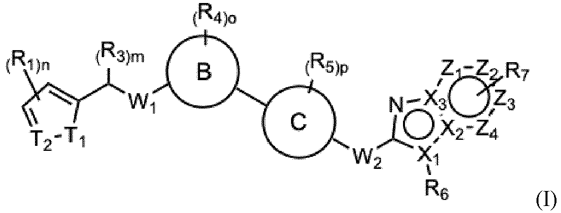
and pharmaceutically acceptable salts thereof,
wherein
T₁ and T₂ are each independently selected from the group consisting of CH₂, NH, O, and S;
W₁ is selected from the group consisting of O, S, CH₂, and NH;
W₂ is selected from the group consisting of O, NH, CH₂, and CR_{y};
Z₁, Z₂, Z₃, and Z₄ are each independently selected from the group consisting of CH, N, or C;
X₁, X₂, and X₃ are each independently selected from the group consisting of CH, N, or C, and at most two of X₁, X₂, and X₃ are N;
ring B is selected from the group consisting of benzene ring or 5- to 7-membered heteroaromatic ring;
ring C is selected from the group consisting of benzene ring, 4 to 8-membered heterocyclic ring, 5 to 10-membered spiro ring, 5 to 10-membered bridged ring, and 5- to 7-membered heteroaromatic ring;
R₁ is independently selected from the group consisting of R₂, -carbonyl-R₂, -carbonyl-amino-R₂, -sulphonyl-R₂, -amido-R₂, -phosphoroso-Rz, -amino-R₂, and -O-R₂, wherein the R₂, amino, amido, sulphonyl, and phosphoroso in R₁ may be optionally substituted 1 to 3 times by asubstituent(s) independently selected from Rₓ;
R₂ is independently selected from the group consisting of hydrogen, oxo, halogen, -C₁₋₆ alkyl, -C₂₋₆ alkenyl, -C₂₋₆ alkynyl, -C₁₋₆ alkoxy, -C₁₋₆ cycloalkoxy, cyano, 3- to 8-membered cycloalkyl, 3- to 8-membered heterocyclyl, phenyl, and 5- to 8-membered heteroaryl, wherein the halogen, alkyl, alkenyl, alkynyl, amino, alkoxy, cycloalkoxy, cycloalkyl, heterocyclyl, phenyl, and heteroaryl in R₂ may be optionally substituted 1 to 3 times by a substituent(s) independently selected from Rₓ;
R₃ is independently selected from the group consisting of hydrogen, oxo, halogen, -CN, -C₁₋₆ alkyl, -C₂₋₆ alkenyl, -C₂₋₆ alkynyl, -C₁₋₆ alkoxy, amino, amido, sulfonyl, sulfonamido, -OH, -C₃₋₈ cycloalkyl, 3- to 8 membered heterocyclyl, 6- to 10 membered aryl, and 5- to 8 membered heteroaryl, wherein R₃ may be optionally substituted 1 to 3 times by a substituent(s) independently selected from R_{y}, where valency permits;
R₄ is independently selected from the group consisting of hydrogen, halogen, -C₁₋₃ alkyl, -C₁₋₃ haloalkyl, -C₁₋₃ alkoxy, cyano, hydroxyl, amino, amido, sulfonyl, and sulfonamido;
R₅ is independently selected from the group consisting of hydrogen, halogen, hydroxy, -CN, -C₁₋₃ alkyl, -C₁₋₃ alkoxy, and -C₁₋₃ cycloalkyl, wherein the alkyl, alkoxy, and cycloalkyl in R₅ may be optionally substituted 1 to 3 times by halogen, hydroxyl, -NR_{z}, -CN, -C₁₋₃ alkyl, -C₁₋₃ alkoxy, -C₁₋₃ cycloalkyl, where valency permits;
R₆ is selected from the group consisting of -R_{z}, -O-R_{z}, -S-R_{z}, -C₁₋₃ alkyl, -C₁₋₃ alkylene-R_{z}, -C₀₋₃ alkylene-amino-R_{z}, -C₀₋₃ alkylene-carbonyl-R_{z}, -C₀₋₃ alkylene-amido-R_{z}, -C₀₋₃ alkylene-sulfonyl-R_{z}, -C₀₋₃ alkylene-phosphoryl-R_{z}, and -C₀₋₃ alkylene-sulfonamido-R_{z}, wherein the alkyl, amino, amido, sulfonyl, sulfonamido, and phosphoryl in R₆ may be optionally substituted 1 to 3 times by halogen or one time by R_{w}, where valency permits;
R₇ is selected from the group consisting of -COOH, -C(R_{y})ₙ₀-COOH, -N(R_{z})ₙ₀-COOH, -SOz-COOH, and -SO₂-NH-COOH, wherein the R_{y} in -C(R_{y})ₙ₀- may be attached to C in the form of a backbone and/or a branched chain, the R_{z} in -N(R_{z})ₙ₀- may be attached to N in the form of a backbone and/or a side chain, wherein n₀ is an integer selected from 0, 1 or 2; when n₀ is 2, two R_{y} or R_{z} may be further cyclized to form a 3- to 8-membered carbocyclic or heterocyclic ring;
n is an integer selected from 0, 1, 2 or 3;
m is an integer selected from 0, 1 or 2;
o is an integer selected from 0, 1, 2, 3 or 4;
p is an integer selected from 0, 1, 2, 3 or 4;
when n is more than or equal to 2, any two R₁ may be further cyclized into a 3- to 8-membered carbocyclic ring, aromatic ring, heterocyclic ring or heteroaromatic ring, wherein the formed carbocyclic ring and heterocyclic ring may be optionally substituted 1 to 3 times by C₁₋₃ alkyl, C₁₋₃ haloalkyl, halogen, cyano, C₁₋₃ alkoxy, where valency permits;
when m is 2, two R₃ may be further cyclized into a 3- to 8-membered carbocyclic ring or heterocyclic ring;
when m is 1 or 2, R₁ and R₃ may be further cyclized into a 3- to 8-membered carbocyclic ring or heterocyclic ring;
when p is greater than or equal to 2, any two R₅ may be further cyclized with the ring C to form a 6- to 10-membered spiro ring or bridged ring, wherein the formed spiro ring and bridged ring may be optionally substituted 1 to 3 times by C₁₋₃ alkyl, C₁₋₃ haloalkyl, halogen, cyano, or C₁₋₃ alkoxy where valency permits;
when o is not 0 and p is not 0, any R₄ and R₅ may be further cyclized to form a 5- to 8-membered ring, wherein the formed ring may be optionally substituted 1 to 3 times by C₁₋₃ alkyl, C₁₋₃ haloalkyl, halogen, cyano, oxo, C₁₋₃ alkoxy, where valence permits;
R_{w} is independently selected from the group consisting of -CN, -CH₂CN, -C₁₋₃ alkyl, -OH, -C₁₋₃ alkoxy, amido, sulfonyl, sulfonamido, -NH₂, and -NH-C₁₋₃ alkyl, wherein the alkyl in R_{w} may be optionally substituted 1 to 3 times by C₁₋₃ alkyl, C₁₋₃ haloalkyl, halogen, cyano, oxo, C₁₋₃ alkoxy, where valence permits;
Rₓ is independently selected from the group consisting of hydrogen, halogen, oxo, C₁₋₆ alkoxy, cyano, hydroxyl, carboxyl, amino, amido, sulfonyl, sulfonamido, -C₁₋₆ alkyl, -C₂₋₆ alkenyl, -C₂₋₆ alkynyl, -C₃₋₆ cycloalkyl, 3- to 6-membered heterocyclyl, 6- to 8-membered aryl, and 5- to 8-membered heteroaryl, wherein the alkyl, alkoxy, cycloalkyl, heterocyclyl, aryl, and heteroaryl in Rₓ may be optionally substituted 1 to 3 times by C₁₋₃ alkyl, C₁₋₃ haloalkyl, halogen, cyano, oxo, C₁₋₃ alkoxy, or one time by hydroxy, where valency permits;
R_{y} is independently selected from the group consisting of hydrogen, halogen, oxo, -C₁₋₃ alkoxy, cyano, hydroxyl, amino, carboxyl, amido, sulfonyl, sulfonamido, -C₁₋₆ alkyl, -C₂₋₆ alkenyl, -C₂₋₆ alkynyl, C₃₋₆ cycloalkyl, 3- to 6-membered heterocyclyl, and 5- to 6-membered heteroaryl, wherein the alkyl, alkenyl, alkynyl, amido, amido, alkoxy, cycloalkyl, heterocyclyl and heteroaryl in R_{y} may be optionally substituted 1 to 3 times by C₁₋₃ alkyl, C₁₋₃ haloalkyl, halogen, cyano, oxo, C₁₋₃ alkoxy, where valence permits; and
R_{z} is independently selected from the group consisting of hydrogen, C₁₋₃ alkyl, C₁₋₃ alkoxy, C₃₋₆ cycloalkyl, 3- to 6-membered heterocyclyl, aryl, and 5- to 6-membered heteroaryl, wherein R_{z} may be optionally substituted 1 to 3 times by C₁₋₃ alkyl, C₁₋₃ haloalkyl, halogen, cyano, oxo, C₁₋₃ alkoxy, 3- to 6-member heterocyclyl, where valence permits.

In a specific embodiment, the ring B may be further selected from the group consisting of 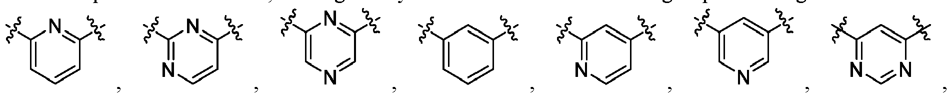 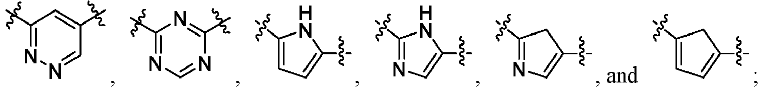 preferably, the ring B is 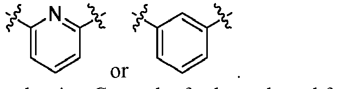 in a specific embodiment, the ring C may be further selected from the group consisting of 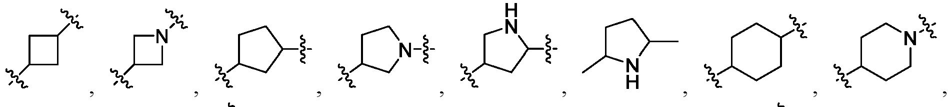 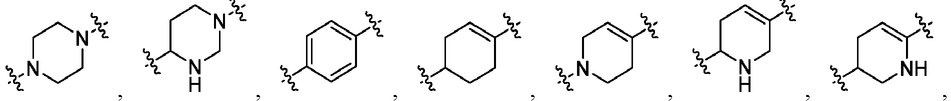 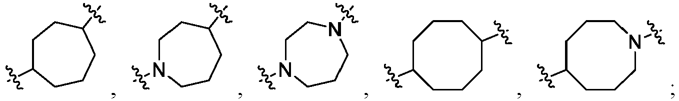 preferably, the ring C is

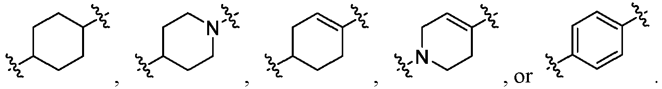

Further, the present invention provides a compounds as represented by Formula I-2: 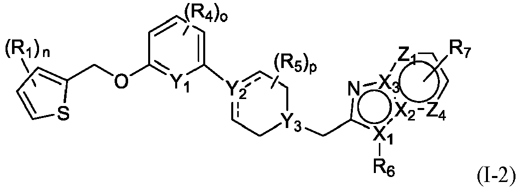 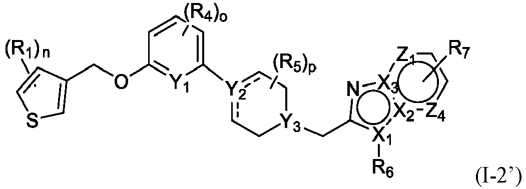
and pharmaceutically acceptable salts thereof,
wherein
------ represents the presence or absence of a chemical bond;
Z₁ and Z₄ are each independently selected from the group consisting of CH and N;
X₁, X₂ and X₃ are each independently selected from the group consisting of CH, N or C, and at most two of X₁, X₂ and X₃ are N;
Y₁ is selected from the group consisting of CH or N;
Y₂ is selected from the group consisting of CH, N or C;
Y₃ is selected from the group consisting of CH or N;
R₁ is independently selected from the group consisting of R₂, -carbonyl-R₂, -carbonyl-amino-R₂, -sulphonyl-R₂, -amido-R₂, -phosphoroso-Rz, -amino-R₂, and -O-R₂, wherein the R₂, amino, amido, sulphonyl, and phosphoroso in R₁ may be optionally substituted 1 to 3 times by a substituent(s) independently selected from Rₓ;
R₂ is independently selected from the group consisting of hydrogen, oxo, halogen, -C₁₋₆ alkyl, -C₂₋₆ alkenyl, -C₂₋₆ alkynyl, -C₁₋₆ alkoxy, -C₁₋₆ cycloalkoxy, cyano, 3- to 8-membered cycloalkyl, 3- to 8-membered heterocyclyl, phenyl, and 5- to 8-membered heteroaryl, wherein the halogen, alkyl, alkenyl, alkynyl, amino, alkoxy, cycloalkoxy, cycloalkyl, heterocyclyl, phenyl, and heteroaryl in R₂ may be optionally substituted 1 to 3 times by a substituent(s) independently selected from Rₓ;
R₃ is independently selected from the group consisting of hydrogen, oxo, halogen, -CN, -C₁₋₆ alkyl, -C₂₋₆ alkenyl, -C₂₋₆ alkynyl, -C₁₋₆ alkoxy, amino, amido, sulfonyl, sulfonamido, -OH, -C₃₋₈ cycloalkyl, 3- to 8-membered heterocyclyl, 6- to 10-membered aryl, and 5- to 8-membered heteroaryl, wherein R₃ may be optionally substituted 1 to 3 times by a substituent(s) independently selected from R_{y}, where valency permits;
R₄ is independently selected from the group consisting of hydrogen, halogen, -C₁₋₃ alkyl, -C₁₋₃ haloalkyl, -C₁₋₃ alkoxy, cyano, hydroxyl, amino, amido, sulfonyl, and sulfonamido;
R₅ is independently selected from the group consisting of hydrogen, halogen, hydroxy, -CN, -C₁₋₃ alkyl, -C₁₋₃ alkoxy, and -C₁₋₃ cycloalkyl, wherein the alkyl, alkoxy, and cycloalkyl in R₅ may be optionally substituted 1 to 3 times by halogen, hydroxyl, -NR_{z}, -CN, -C₁₋₃ alkyl, -C₁₋₃ alkoxy, -C₁₋₃ cycloalkyl, where valency permits;
R₆ is selected from the group consisting of -R_{z}, -O-R_{z}, -S-R_{z}, -C₁₋₃ alkyl, -C₁₋₃ alkylene-R_{z}, -C₀₋₃ alkylene-amino-R_{z}, -C₀₋₃ alkylene-carbonyl-R_{z}, -C₀₋₃ alkylene-amido-R_{z}, -C₀₋₃ alkylene-sulfonyl-R_{z}, -C₀₋₃ alkylene-phosphoryl-R_{z}, and -C₀₋₃ alkylene-sulfonamido-R_{z}, wherein the alkyl, amino, amido, sulfonyl, sulfonamido, and phosphoryl in R₆ may be optionally substituted 1 to 3 times by halogen or one time by R_{w}, where valency permits;
R₇ is selected from the group consisting of -COOH, -C(R_{y})ₙ₀-COOH, -N(R_{z})ₙ₀-COOH, -SOz-COOH, and -SO₂-NH-COOH, wherein the R_{y} in -C(R_{y})ₙ₀- may be attached to C in the form of a backbone and/or a side chain, the R_{z} in -N(R_{z})ₙ₀- may be attached to N in the form of a backbone and/or a side chain, wherein n₀ is an integer selected from 0, 1 or 2; when n₀ is 2, two R_{y} or R_{z} may be further cyclized into a 3- to 8-membered carbocyclic ring or heterocyclic ring;
n is an integer selected from 0, 1, 2 or 3;
o is an integer selected from 0, 1, 2, 3 or 4;
p is an integer selected from 0, 1, 2, 3 or 4;
when n is more than or equal to 2, any two R₁ may be further cyclized to form a 3- to 8-membered carbocyclic ring, aromatic ring, heterocyclic ring or heteroaromatic ring, wherein the formed carbocyclic ring and heterocyclic ring may be optionally substituted 1 to 3 times by C₁₋₃ alkyl, C₁₋₃ haloalkyl, halogen, cyano, C₁₋₃ alkoxy, where valence permits;
when p is more than or equal to 2, any two R₅ may be further cyclized with the ring C to form a 6- to 10-membered spiro ring or bridged ring, wherein the formed spiro ring and bridged ring may be optionally substituted 1 to 3 times by C₁₋₃ alkyl, C₁₋₃ haloalkyl, halogen, cyano, C₁₋₃ alkoxy, where valence permits;
when o is not 0 and p is not 0, any R₄ and R₅ may be further cyclized to form a 5- to 8-membered ring, wherein the formed ring may be optionally substituted 1 to 3 times by C₁₋₃ alkyl, C₁₋₃ haloalkyl, halogen, cyano, oxo, C₁₋₃ alkoxy, where valence permits;
R_{w} is independently selected from the group consisting of -CN, -CH₂CN, -C₁₋₃ alkyl, -OH, -C₁₋₃ alkoxy, amido, sulfonyl, sulfonamido, -NH₂, and -NH-C₁₋₃ alkyl, wherein the alkyl in R_{w} may be optionally substituted 1 to 3 times by C₁₋₃ alkyl, C₁₋₃ haloalkyl, halogen, cyano, C₁₋₃ alkoxy, where valence permits;
Rₓ is independently selected from the group consisting of hydrogen, halogen, oxo, C₁₋₆ alkoxy, cyano, hydroxyl, carboxyl, amino, amido, sulfonyl, sulfonamido, -C₁₋₆ alkyl, -C₂₋₆ alkenyl, -C₂₋₆ alkynyl, -C₃₋₆ cycloalkyl, 3- to 6-membered heterocyclyl, 6- to 8-membered aryl, and 5- to 8-membered heteroaryl, wherein the alkyl, alkoxy, cycloalkyl, heterocyclyl, aryl, and heteroaryl in Rₓ may be optionally substituted 1 to 3 times by halogen or optionally substituted 0 to 1 time by hydroxyl, where valence permits;
R_{y} is independently selected from the group consisting of hydrogen, halogen, oxo, -C₁₋₃ alkoxy, cyano, hydroxyl, amino, carboxyl, amido, sulfonyl, sulfonamido, -C₁₋₆ alkyl, -C₂₋₆ alkenyl, -C₂₋₆ alkynyl, C₃₋₆ cycloalkyl, and 3- to 6-membered heterocyclyl, wherein the alkyl, alkoxy, cycloalkyl, and heterocyclyl in R_{y} may be optionally substitute 1 to 3 times by halogen, where valence permits; and
R_{z} is independently selected from the group consisting of hydrogen, C₁₋₃ alkyl, C₁₋₃ alkoxy, C₃₋₆ cycloalkyl, 4- to 6-membered heterocyclyl, 5- to 6-membered aryl, or 5- to 6-membered heteroaryl, wherein R_{z} may be optionally substituted 1 to 3 times by halogen, cyano, C₁₋₃ alkyl, C₁₋₃ alkoxy, C₃₋₆ cycloalkyl, 3- to 6-membered heterocyclyl, where valence permits.
In a specific embodiment, when o is not 0 and p is not 0, any adjacent R₄ and R₅ may be further cyclized to form a 5- to 8-membered ring, wherein the 5- to 8-membered ring comprises C₅₋₆ carbocyclic ring, 5- to 8-membered heterocyclic ring, benzene ring, and 5- to 8-member heteroaromatic ring, and the formed ring can be optionally substituted 1 to 3 times by alkyl, haloalkyl, halogen, cyano, alkoxy, wherein valence permits.

In a specific embodiment, when o is not 0 and p is not 0, any adjacent R₄ and R₅ may be further cyclized to form a 5- to 8-membered ring which may be selected from the group consisting of 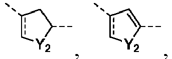 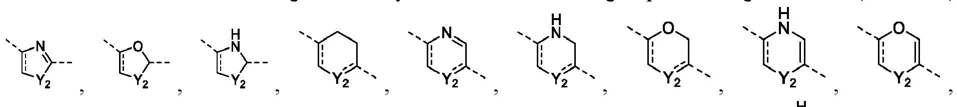 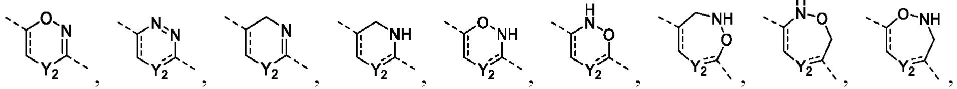 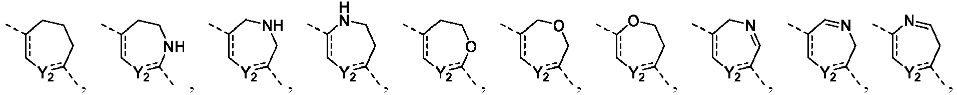 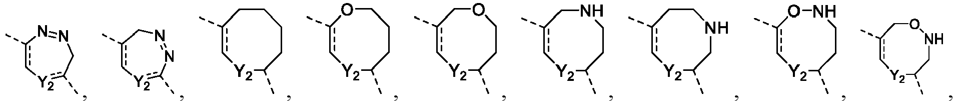 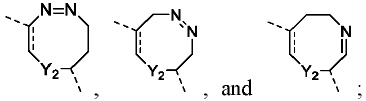 wherein the formed 5- to 8-membered ring may be optionally substituted 1 to 3 times by C₁₋₃ alkyl, C₁₋₃ haloalkyl, halogen, cyano, oxo, C₁₋₃ alkoxy, where valence permits.

In a specific embodiment, when o is not 0 and p is not 0, any adjacent R₄ and R₅ may be further cyclized to form a 5- to 8-membered ring which is preferably 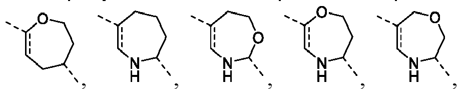 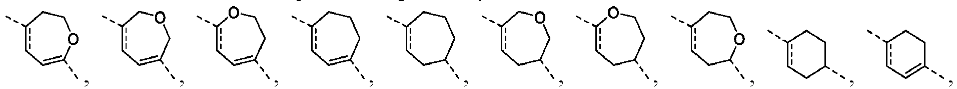 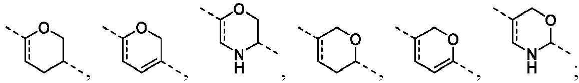 wherein the formed 5- to 8-membered ring may be optionally substituted 1 to 3 times by C₁₋₃ alkyl, C₁₋₃ haloalkyl, halogen, cyano, oxo, C₁₋₃ alkoxy, where valence permits.

In a specific embodiment, when o is not 0 and p is not 0, any adjacent R₄ and R₅ may be further cyclized to form a 5- to 8-membered ring which may be selected from the group consisting of 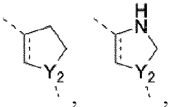 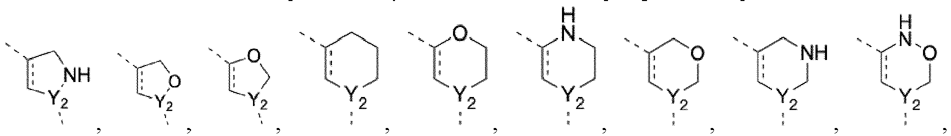 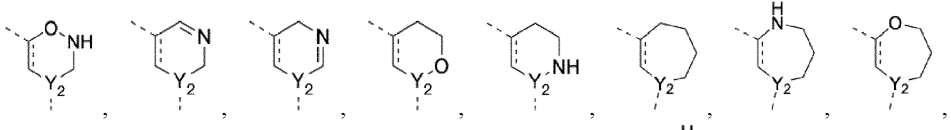 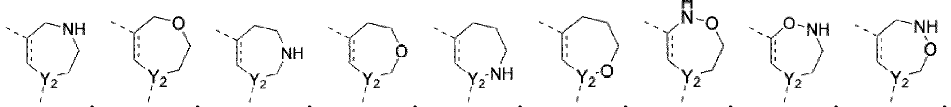 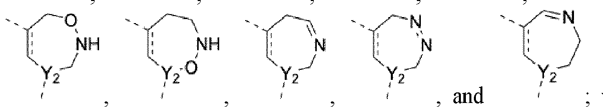 wherein the formed 5- to 8-membered ring may be optionally substituted 1 to 3 times by C₁₋₃ alkyl, C₁₋₃ haloalkyl, halogen, cyano, oxo, C₁₋₃ alkoxy, where valence permits.

In a specific embodiment, the structural unit 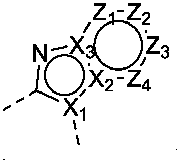 may be further selected from the group consisting of

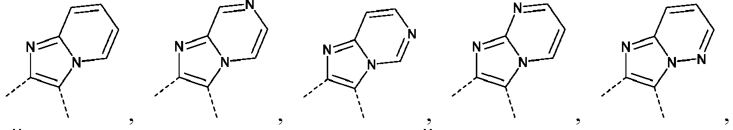

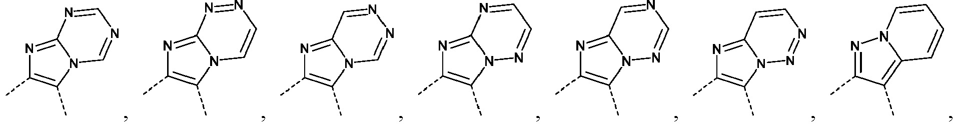

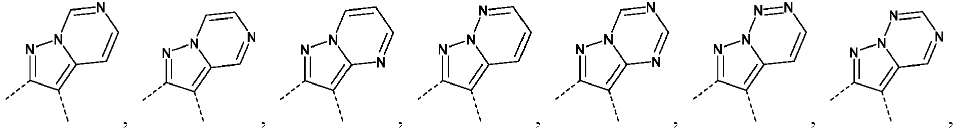

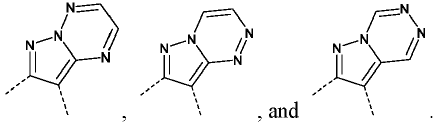

In a specific embodiment, the compound of Formula I according to the present invention may have the following subformula, 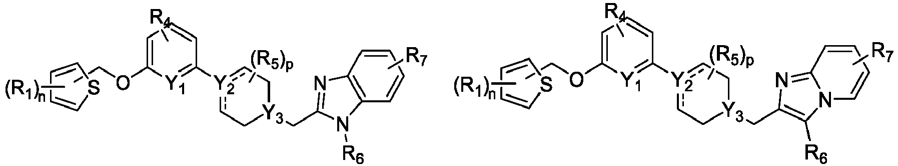 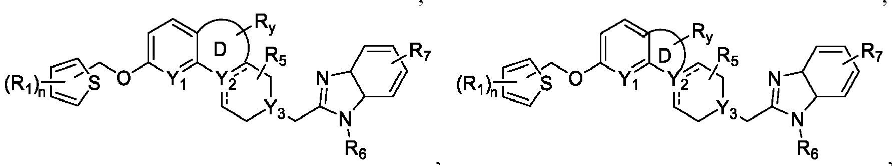 or

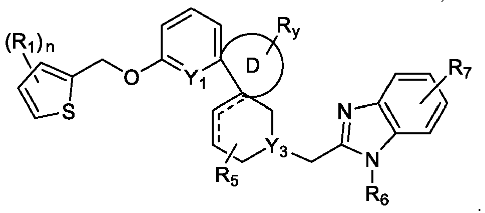

In a specific embodiment, said n is selected from 1, 2 or 3.

In a specific embodiment, said p is selected from 0, 1 or 2.

In a specific embodiment, said R₁ may be further independently selected from the group consisting of -F, -Cl, -CN, -OCH₃, -OCH₂CH₃, -O-cyclopropyl, -CH₃, -CH₂CH₃, -CH₂CH₂CH₃, -(CH)₂CH₃, -COCH₃, -CONH₂, -CF₃, -CHF₂, -CH₂F, -CH₂CH₂F, -CO-cyclopropyl, 5- to 6-membered heterocyclyl, and 5- to 6-membered heteroaryl.

In a specific embodiment, said R₃ may be further selected from the group consisting of -F, -Cl, -CH₃, -OCH₃, -NH₂, -OH, -CH₂CH₃, -CH₂OH, -NHCH₃, -COCH₃, -SO₂CH₃, -OCH₂CH₃, -CF₃, -CHF₂, -CH₂F, isopropyl, cyclopropyl, and fluorocyclopropyl.

In a specific embodiment, said R_{y} may be further selected from the group consisting of -F, -Cl, methyl, ethyl, trifluoromethyl, difluoromethyl, fluoromethyl, fluoroethyl, methoxy, amino, hydroxyl, propyl, isopropyl, cyclopropyl, and cyclobutyl.

In a specific embodiment, said R_{y} in -C(R_{y})ₙ₀-COOH of R₇ may be attached to C in the form of a backbone and/or a side chain; when R_{y} is attached to C of R₇ in the form of a backbone, R_{y} exists in the form of a corresponding divalent group; and when R_{y} is attached to C of R₇ in the form of a side chain, R_{y} exists in the form of a corresponding saturated group.

In a specific embodiment, said R_{z} in -N(R_{z})ₙ₀-COOH of R₇ may be attached to N in the form of a backbone and/or a side chain; when R_{z} is attached to C of R₇ in the form of a backbone, R_{z} exists in the form of a corresponding divalent form; and when R_{y} is attached to N of R₇ in the form of a side chain, R_{z} exists in the form of a corresponding saturated group.

In a specific embodiment, when said R_{y} in -C(R_{y})ₙ-COOH of R₇ is methyl, said "attached to C in the form of a backbone" means being attached in the structure of 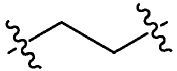 (i.e., R₇ is -CH₂- in this case), and said "attached to C in the form of a side chain" means being attached in the structure of 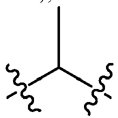 (i.e., R₇ is -CH₃ in this case).

In a specific embodiment, said R₆ is selected from the group consisting of -R_{z}, -O-R_{z}, -S-R_{z}, -C₁₋₃ alkylene-R_{z}, -C₀₋₃ alkylene-amino-R_{z}, and -C₀₋₃ alkylene-carbonyl-R_{z}, wherein the alkyl, amino, amido, sulfonyl, sulfonamido and phosphoryl in R₆ may be optionally substituted 1 to 3 times by halogen or one time by R_{w}, where valence permits.

In a specific embodiment, said R_{z} may be further selected from the group consisting of methyl, ethyl, isopropyl, cyclopropyl, cyclobutyl, methoxy, ethoxy. 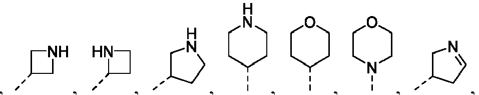 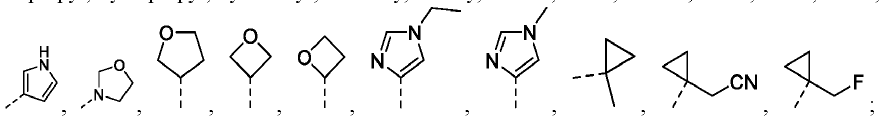 R_{z} may be optionally substituted 1 to 3 times by halogen, cyano, C₁₋₃ alkyl, C₁₋₃ alkoxy, C₃₋₆ cycloalkyl, 3- to 6-membered heterocyclyl, where valency permits.

In a specific embodiment, said R₁ may be further independently selected from the group consisting of -F, -Cl, -CN, -OCH₃, -OCH₂CH₃, -CH₃, -CH₂CH₃, -COCH₃, -CONH₂, -CF₃, -CHF₂, -CH₂F, -CH₂CH₂F, -CO-cyclopropyl, 5- to 6-membered heterocyclyl, and 5- to 6-membered heteroaryl.

In a specific embodiment, said R₂ may be further independently selected from the group consisting of -H, -CH₃, -CHF₂, -CH₂F, -CF₃, -CH₂CH₃, -CH₂CH₂F, -NH₂, cyclopropyl, 5- to 6-membered heterocyclyl, and 5-to 6-membered heteroaryl.

In a specific embodiment, said R₃ may be further selected from the group consisting of -F, -Cl, -CH₃, -OCH₃, -NH₂, -OH, -CH₂CH₃, -CH₂OH, -NHCH₃, -COCH₃, -SO₂CH₃, -OCH₂CH₃, -CF₃, -CHF₂, -CH₂F, isopropyl, cyclopropyl, and fluorocyclopropyl.

In a specific embodiment, said R₄ may be further selected from the group consisting of -CN, -CH₃, -OH, -CH₂OH, -CH₂OCH₃, -OCH₃, -NH₂, -NHCH₃, -COCH₃, -OCH₂CH₃.

In a specific embodiment, said R₅ is selected from the group consisting of -F, -Cl, -CN, -CH₃, -CH₂CH₃, -CF₃, -CHF₂, -CH₂F, -CHzOH, -OH, -CH₂OCH₃, -OCH₃, -CHzCHzOH, -CH₂CH₂OCH₃, isopropyl or cyclopropyl.

In a specific embodiment, said R₇ is selected from the group consisting of -COOH, -CH₂COOH, -CH₂CH₂COOH, and -CH(CH₃)COOH, wherein said R₇ may be optionally substituted 1 to 3 times by halogen, where valence permits.

In a specific embodiment, the present invention provides a series of compounds which are independently selected from one of the following compounds or any combination thereof: 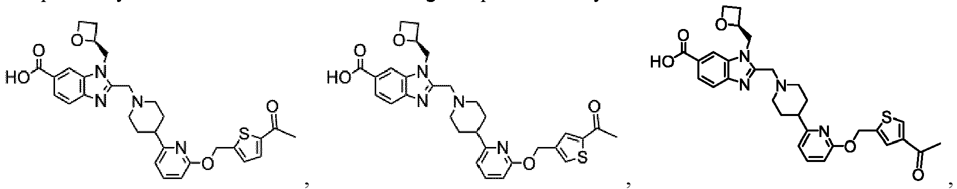 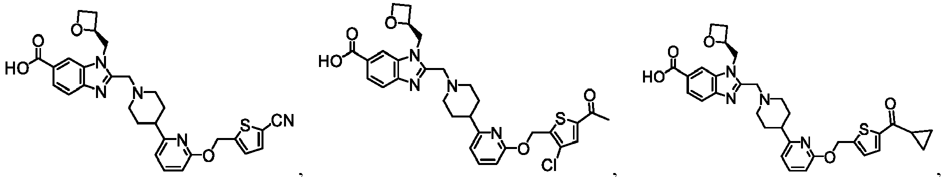 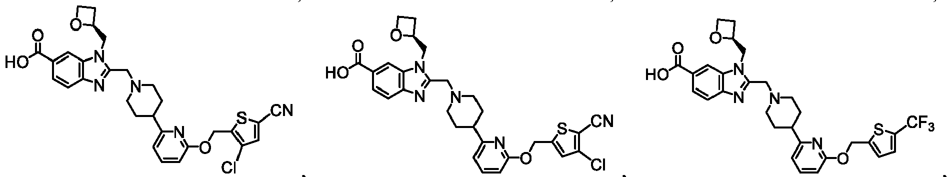 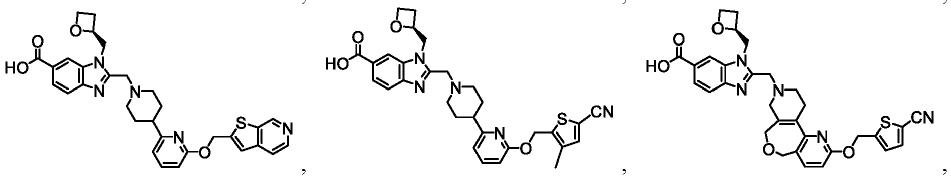 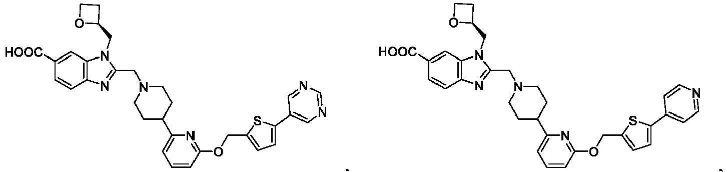 and pharmaceutically acceptable salts thereof.

In a specific embodiment, the present invention provides a series of compounds which are independently selected from one of the following compounds or any combination thereof: 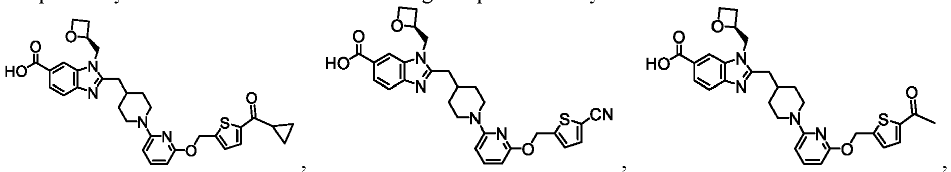 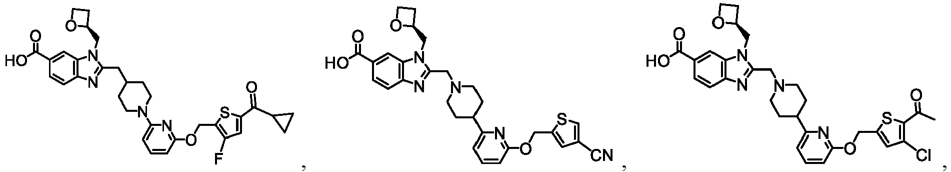 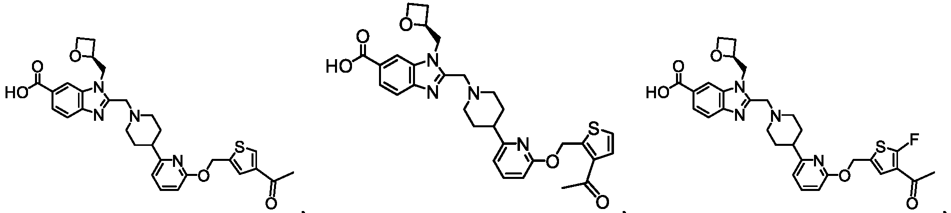 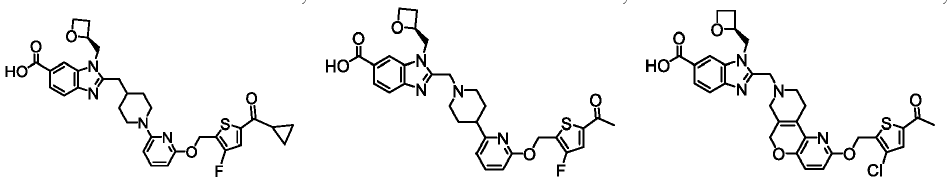 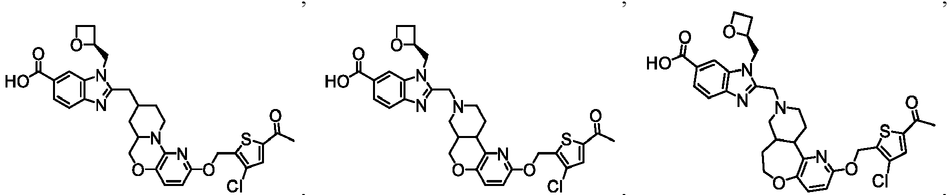 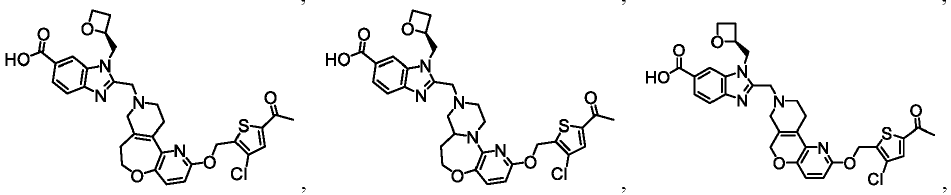 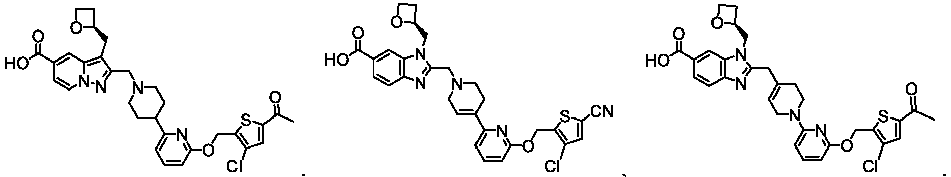 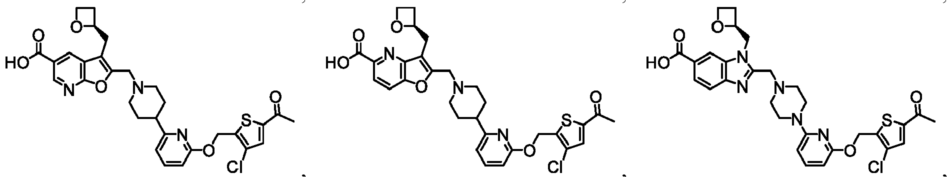 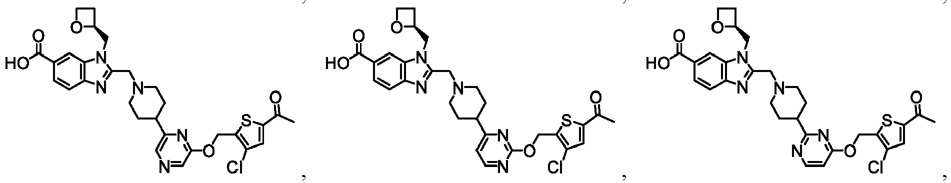 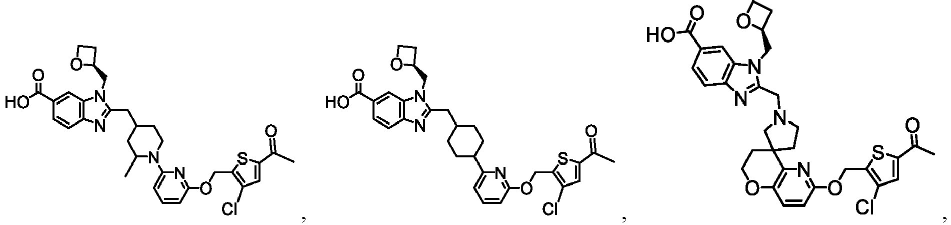 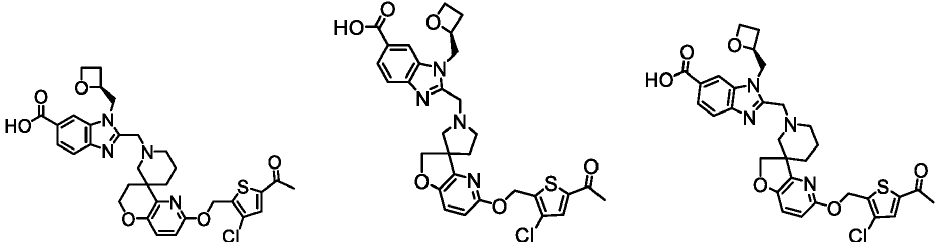 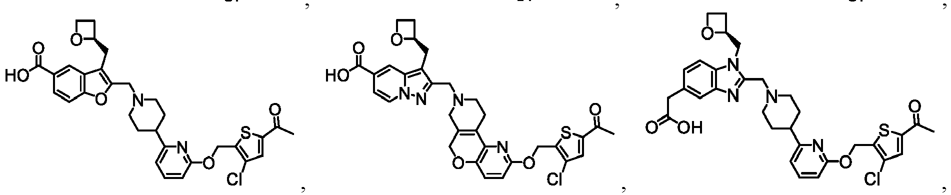 and pharmaceutically acceptable salts thereof.

The compound and the pharmaceutically acceptable salt thereof provided in the present invention may be used in therapy alone or in combination with at least one other therapeutic agent.

The present invention provides a pharmaceutical composition comprising the compound of Formula I and a pharmaceutically acceptable salt thereof, with one, two or more other therapeutically active ingredients.

The present invention still provides a pharmaceutical formulation comprising the compound of Formula I and a pharmaceutically acceptable salt thereof, with one, two or more pharmaceutical carriers; the pharmaceutical formulation may be in any clinically acceptable dosage form of the formulation.

The compound of the present invention and the pharmaceutically acceptable salt thereof can be formed into solid dosage forms such as capsules, tablets, pills, lozenges, dragees, granules, powders, ointments, creams, drops or the like; or the compound of the present invention and the pharmaceutically acceptable salt thereof can be in liquid dosage forms such as elixirs, syrups, emulsions, dispersants, suspensions, solutions, sprays or the like.

The pharmaceutical carrier and/or pharmaceutical diluent suitable for the pharmaceutical composition or pharmaceutical formulation of the present invention may be any conventional carrier and/or diluent in the field of pharmaceutical formulations.

The pharmaceutically acceptable salt of the present invention includes an acid addition salt and a base salt.

The pharmaceutically acceptable salt of the present invention may be present in a non-solvated or solvated form.

The present invention also provides a use of the compound of Formula I and the pharmaceutically acceptable salt thereof in the preparation of a medicament for treating and/or metabolism-related diseases, wherein the metabolism-related diseases comprise GLP-1-mediated diseases and related diseases, including but not limited to diabetes, hyperglycemia, insulin resistance, glucose intolerance, diabetic nephropathy, diabetic neuropathy, diabetic retinopathy, adipocyte dysfunction, obesity, dyslipidemia, hyperinsulinemia and the like; wherein the diabetes includes, but is not limited to T1D and/or T2DM, idiopathic T1D, early onset T2D, latent autoimmune diabetes, adolescent atypical diabetes, gestational diabetes and the like.

The present invention also provides a method for the treatment of a disease, comprising administering a therapeutically effective amount of the compound of Formula I and a pharmaceutically acceptable salt thereof to a patient in need thereof, wherein the disease is a GLP-1 mediated disease or a related disease, which includes, but is not limited to diabetes, hyperglycemia, insulin resistance, glucose intolerance, diabetic nephropathy, diabetic neuropathy, diabetic retinopathy, adipocyte dysfunction, obesity, dyslipidemia, hyperinsulinemia and the like; wherein the diabetes includes, but is not limited to T1D and/or T2DM, idiopathic T1D, early onset T2D, latent autoimmune diabetes, adolescent atypical diabetes, gestational diabetes and the like.

The compounds of Formula I and the pharmaceutically acceptable salt thereof provided in the present invention have excellent GLP-1 receptor agonistic activity and thus are useful for the treatment and/or prevention of GLP-1-mediated diseases and related diseases.

The compounds of Formula I and the pharmaceutically acceptable salt thereof provided in the present invention have excellent GLP-1 receptor agonistic activity and thus are useful for the treatment and/or prevention of GLP-1-mediated diseases and related diseases.

The present invention also provides a use of the above-described compound or pharmaceutically acceptable salt thereof in the preparation of a GLP-1 receptor agonist-related medicament.

In some embodiments of the present invention, the GLP-1 receptor agonist-related medicament is used for treating type 2 diabetes, type 1 diabetes, and obesity.

The compounds described in the present invention are named according to the chemical structural formula. Where the name of a compound is inconsistent with the chemical structural formula of the same compound, the chemical structural formula shall prevail.

In the present invention, unless defined otherwise, all scientific and technical terms used herein have the same meaning as those commonly understood by a person skilled in the art. Nevertheless, definitions of some terms are provided below for a better understanding of the present invention. Where the definitions and interpretations of the terms provided herein differ from those commonly understood by a person skilled in the art, the definitions and interpretations of the terms provided herein shall prevail.

The compound and pharmaceutically acceptable salt thereof provided in the present invention may be present in a chiral form, i.e., in S-configuration or R-configuration. The compound and pharmaceutically acceptable salt thereof provided in the present invention may be present in an achiral form. When the structure of a compound described in the present invention is exemplified by one configuration, it is intended that the other configuration or the achiral form thereof is disclosed as well.

The compound described in the present invention comprises a stereoisomer of the compound. The stereoisomer described in the present invention means that when the compound as shown by Formula I has a asymmetric carbon atom, enantiomers will exist; when the compound has a carbon-carbon double bond or cyclic structure, cis-trans isomers will exist; when a ketone or oxime is present in the compound, tautomers will exist. As one particular embodiment, stereoisomers described in the present invention include, but are not limited to, enantiomers, diastereomers, racemic isomers, cis-trans isomers, tautomers, geometric isomers, epimers, and mixtures thereof.

The compound of the present invention may exist in specific geometric or stereoisomer forms. All of such compounds are contemplated in the present invention, including *cis* and *trans* isomers, (-)- and (+)-enantiomers, (R)- and (S)-enantiomers, diastereomers, (D)-isomers, (L)-isomers, and the racemic mixtures and other mixtures thereof, such as enantiomer- or diastereomer-enriched mixtures, all of these mixture falling within the scope of the invention. Additional asymmetric carbon atoms may be present in substituents such as alkyl. All these isomers and mixtures thereof are included within the scope of the present invention.

Unless otherwise stated, the terms "enantiomers" or "optically active isomers" refer to stereoisomers that are mirror images of one another.

Unless otherwise stated, the terms *"cis-trans* isomers" or "geometrical isomers" occur due to the inability to freely rotate of a double bond or a single bond of ring-forming carbon atoms.

Unless otherwise stated, the term "diastereomers" refer to stereoisomers that have two or more chiral centers and are not mirror images of one another.

Unless otherwise stated, "(+)" denotes right-handed, "(-)" denotes left-handed, and "(±)" denotes racemic.

Unless otherwise stated, the wedged solid bond ( 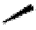 ) and wedged dashed bond ( 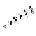 ) are used to denote the absolute configuration of a stereocenter, the straight solid bond ( 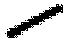 ) and straight dashed bond ( 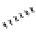 ) are used to indicate that the stereocenter is an absolute configuration, but it is not sure whether it is a wedged solid bond ( 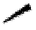 ) or a wedged dashed bond ( 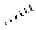 ). The wavy line ( 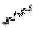 ) is used to denote a wedged solid bond ( 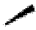 ) or wedged dashed bond ( 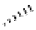 ), or the wavy line ( 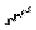 ) is used to denote a straight solid bond ( 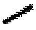 ) or straight dashed bond ( 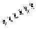 ).

Optically active (R)- and (S)-isomers and *D* and *L* isomers can be prepared by chiral synthesis or chiral reagents or other conventional techniques. If one enantiomer of a compound of the invention is desired, it can be prepared by asymmetric synthesis or the derivatization with a chiral auxiliary reagent, in which the obtained diastereomeric mixture is separated and the auxiliary group is cleaved to provide the pure desired enantiomer. Alternatively, when a basic functional group (e.g., amino) or an acidic functional group (e.g., carboxyl) in a molecule, the molecule can be reacted with a suitable optically active acid or base to form salts of the diastereomers, which can then be subjected to diastereomeric resolution by conventional techniques known to those skilled in the art, followed by recovery of pure enantiomers. In addition, enantiomers and diastereomers are usually separated by chromatography using a chiral fixed phase and optionally combined with chemical derivatization (e.g., producing a carbamate from an amine).

The compound of the present invention may contain an unnatural proportion of atom isotopes on one or more of the atoms constituting the compound. For example, the compound may be labeled with radioisotopes, such as tritium (³H), iodine-125 (¹²⁵I) or C-14 (¹⁴C). For another example, deuterated drugs may be prepared by replacing hydrogen with deuterium, in which a deuterium-carbon bond is stronger than the bond formed between an ordinary hydrogen and carbon. Deuterated drugs have such advantages as reduced toxicity and side effects, enhanced stability and therapeutic effects, prolonged biological half-life and the like, compared to non-deuterated drugs. The transformation of all isotopic constituents of the compound of the invention, whether radioactive or not, is included within the scope of the invention.

The term "pharmaceutically acceptable" herein means that those compounds, materials, compositions and/or dosage forms, within the limits of sound medical judgment, are suitable for use in contact with human and animal tissues without excessive toxicity, irritation, allergic reactions, or other issues or complications, and are commensurate with a reasonable benefit/risk ratio.

The term "pharmaceutically acceptable salt" used herein refers to a salt of the compound of the invention, which is prepared from the compound with a specific substituent of the present invention and a relatively non-toxic acid or base. Where the compounds of the invention contain relatively acidic functional groups, base addition salts can be obtained by contacting such compounds with a sufficient amount of a base in a pure solution or in a suitable inert solvent. Pharmaceutically acceptable base addition salts include sodium, potassium, calcium, ammonium, organic amine or magnesium salts or the like. Where the compounds of the invention contain relatively basic functional groups, acid addition salts can be obtained by contacting such compounds with a sufficient amount of an acid in a pure solution or in a suitable inert solvent. Examples of pharmaceutically acceptable acid addition salts include salts of inorganic acids, including, for example, hydrochloric acid, hydrobromic acid, nitric acid, carbonic acid, bicarbonate, phosphoric acid, monohydrogen phosphate, dihydrogen phosphate, sulfuric acid, bisulfate, hydroiodate, and phosphorous acid; and salts of organic acids, including, for example, acetic acid, propionic acid, isobutyric acid, maleic acid, malonic acid, benzoic acid, succinic acid, octandioic acid, fumaric acid, lactic acid, mandelic acid, phthalic acid, benzenesulfonic acid, p-toluenesulfonic acid, citric acid, tartaric acid, and methanesulfonic acid; and also salts of amino acids (such as arginine), as well as salts of organic acids such as glucuronic acid. Some specific compounds of the invention contain both basic and acidic functional groups, and are allowed to be converted to any base or acid addition salt.

The pharmaceutically acceptable salts of the invention can be synthesized by conventional chemical methods from parent compounds containing acid or base groups. Typically, such salts are prepared by reacting these compounds in a form of free acids or bases with a stoichiometric amount of an appropriate base or acid in water or an organic solvent or a mixture of both.

The term "optional" or "optionally" as used herein means that a subsequently described event or condition may, but not necessarily, occur and that the description includes cases in which the event or condition occurs and cases in which the event or condition does not occur.

The term "substituted" as used herein means that any one or more hydrogen atoms on a particular atom are substituted by a substituent which may include heavy hydrogen and variants of hydrogen, as long as the valence state of the particular atom is normal and the substituted compound is stable. When the substituent is oxygen (i.e., = O), it means that two hydrogen atoms are substituted. Oxygen substitution does not occur on aromatic groups. The term "optionally substituted" refers to being substituted or unsubstituted. Unless otherwise specified, the kind and number of the substituent may be arbitrary on a chemically achievable basis.

The term "optionally substituted" as used herein refers to both "substituted" and "unsubstituted".

When any variable (e.g., R) occurs more than once in the constitution or structure of a compound, it is defined independently of one another in each case. Therefore, for example, if a group is substituted by 0-2 R, said group may be optionally substituted by at most two R, and R in each case has independent options. Furthermore, combinations of substituents and/or variants thereof are permitted only if such combinations will result in stable compounds.

When the number of a linking group is 0, such as-(CRR)₀-, it means that the linking group is a single bond.

When the number of a substituent is 0, it means that the substituent does not exist, for example, -A-(R)₀ indicates that the structure is actually -A.

When a substituent is vacant, it means that the substituent does not exist, for example, when X is vacant in A-X, it means that this structure is actually A.

When one of the variables is selected from a single bond, it means that the two groups to which it is linked are directly connected, for example, when L in A-L-Z represents a single bond, it means that this structure is actually A-Z.

When the bond of a substituent may be cross-linked to two or more atoms in a ring, such substituent may be bonded to any atom in the ring. For example, the structural unit 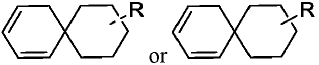 means that the substituent R may substitute any position on the cyclohexyl or cyclohexadiene. When it is not specified by which atom in a recited substituent it is attached to the substituted group, the substituent may be bonded by any of its atoms. For example, the pyridyl group as a substituent may be attached to the substituted group by any of the carbon atoms in the pyridine ring.

Where the linking orientation of a recited linking group is not indicated, the linking orientation thereof is arbitrary. For example, where the linking group L in 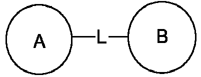 is -M-W- the -M-W- can either connect ring A and ring B in the same orientation as the reading order from left to right to form 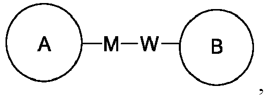 or connect ring A and ring B in the opposite orientation to the reading order from left to right to form 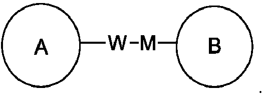 Combinations of the linking groups, substituents and/or variants thereof are permitted only if such combinations may result in stable compounds.

Unless otherwise specified, when a group has one or more linkable sites, any one or more sites of the group may be linked to other groups by a chemical bond. When the site to which the chemical bond is linked is not specified and there exist H atoms at a linkable site, the number of the H atoms at the linkable site when linked to the chemical bond will decrease correspondingly with the number of the linking chemical bond to become a group with a corresponding valence number. The chemical bond between the site and other groups may be represented by a straight solid bond ( 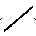 ), a straight dashed bond ( 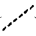 ), or a wavy line ( 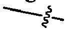 ). For example, the straight solid bond in -OCH₃ indicates connection to other groups through the oxygen atom in the group; the straight dashed bonds in the group 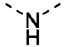 indicate connection to other groups through both ends of the nitrogen atom in the group; the wavy lines in 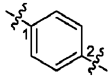 indicate connection to other groups through carbon atoms at positions 1 and 2 in the phenyl group; 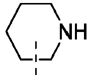 indicates that any linkable site on the piperidine group can be connected to other groups through one chemical bond, at least including the four connection manners: 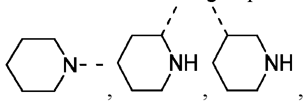 and 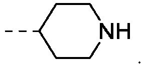 Even if an H atom is shown on -N- 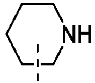 still includes the 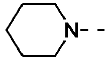 group in such a connection manner which causes a corresponding reduction by one of H on this site when connected with one chemical bond, resulting in a corresponding monovalent piperidyl.

Unless otherwise specified, the number of atoms in a ring is usually defined as the number of the member s of the ring. For example, a "5- to 7-membered ring" refers to a "ring" of 5 to 7 atoms in a cyclic arrangement.

The term "halogen atom" as used herein means a fluorine atom, a chlorine atom, a bromine atom, an iodine atom, and the like. Preferably, the halogen atoms as substituents on the aryl groups in the present invention are fluorine and chlorine atoms. Preferably, the halogen atoms as substituents on the alkyl groups in the present invention are fluorine and chlorine atoms. C₁₋₆ alkyl groups with a halogen atom as a substituent include, but are not limited to, fluoromethyl, difluoromethyl, trifluoromethyl, chloromethyl, pentafluoroethyl, 2-fluoroethyl, 2,2,2-trifluoroethyl, 2-chloroethyl, heptafluoropropyl, 3,3,3-trifluoropropyl, 2,3-dichloropropyl, 1-fluoro-3-bromopropyl, 4-bromobutyl, 3,3,3,4,4-pentafluorobutyl, 4,4-dichlorobutyl, 5-iodopentyl, 5,5-difluoropentyl, 6-chlorohexyl, and 6,6,6-trifluorohexyl.

The term "C₁₋₆ alkyl" as used herein is a straight or branched alkyl group having 1 to 6 carbons, including but not limited to, methyl, ethyl, n-propyl, isopropyl, n-butyl, isobutyl, sec-butyl, tert-butyl, 1-methylpropyl, n-amyl, isoamyl, 2-methylbutyl, 1,1-dimethylpropyl, 1-ethylpropyl, n-hexyl, 4-methylpentyl, and 2-ethylbutyl.

The term "C₁₋₆ alkoxy" as used herein means a C₁₋₆ alkyl-O- group, including but not limited to methoxy, ethoxy, n-propoxy, isopropoxy, n-butoxy, isobutoxy, sec-butoxy, tert-butoxy, 1-methylpropoxy, n-amyloxy, isoamyloxy, 2-methylbutoxy, 1,1-dimethylpropoxy, 1-ethylpropoxy, n-hexyloxy, 4-methylamyloxy, and 2-ethylbutoxy.

The term "aryl" as used herein refers to a 6- to 14-membered all-carbon monocyclic or fused polycyclic (i.e., rings sharing a pair of adjacent carbon atoms) group having a conjugated π-electron system, preferably a 6- to 10-membered ring, such as phenyl and naphthyl, more preferably phenyl. The aryl ring may be fused to heteroaryl, heterocyclyl or cycloalkyl rings, including benzo 3- to 8-membered cycloalkyl and benzo 3- to 8-membered heterocyclyl, wherein the heterocyclyl is a heterocyclic group containing 1 to 3 nitrogen atoms, oxygen atoms and sulfur atoms; or further including a ternary nitrogen-containing fused ring containing a benzene ring.

The term "heteroaryl" as used herein refers to a heteroaromatic system comprising 1 to 4 heteroatoms and 5 to 14 ring atoms, wherein the heteroatom is selected from the group consisting of oxygen, sulfur and nitrogen. The heteroaryl group is preferably 5- to 10-membered, more preferably 5- or 6-membered, such as imidazolyl, furyl, thienyl, thiazolyl, pyrazolyl, oxazolyl, pyrrolyl, triazolyl, tetrazolyl, pyridyl, pyrimidine, thiadiazole, pyrazinyl, preferably triazolyl, thienyl, imidazolyl, pyrazolyl, oxazolyl, pyrimidine or thiazolyl. The heteroaryl ring may be fused to an aryl, heterocyclyl or cycloalkyl ring, wherein the ring attached to the parent structure is a heteroaryl ring, including but not limited to

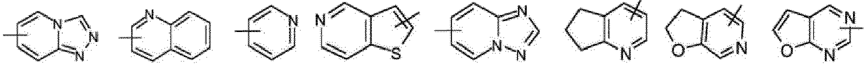

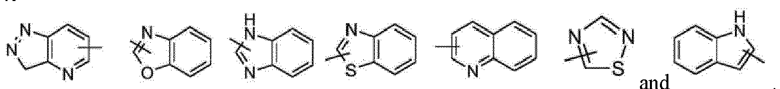

The heteroaryl group may be optionally substituted or unsubstituted. When substituted, the substituent may be preferably one or more groups independently selected from the group consisting of alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxyl, heterocycloalkoxyl, cycloalkylthio, heterocycloalkylthio, carboxyl, or carboxylic ester groups.

Unless otherwise specified, the terms "5- to 6-membered heteroaromatic ring" and "5- to 6-membered heteroaryl" as used herein can be used interchangeably. The term "5-6-membered heteroaryl" means a monocyclic group having a conjugated π-electron system which consists of 5 to 6 ring atoms, of which 1, 2, 3 or 4 ring atoms are heteroatoms independently selected from O, S and N and the remainder are carbon atoms, wherein the nitrogen atom is optionally quaternized, and nitrogen and sulfur heteroatoms may be optionally oxidized (i.e., NO and S(O)ₚ, p being 1 or 2). The 5- to 6-membered heteroaryl may be connected to the rest of the molecule through a heteroatom or carbon atom. The 5- to 6-membered heteroaryl comprises 5-membered and 6-membered heteroaryl groups. Examples of the 5- to 6-membered heteroaryl group include, but are not limited to, pyrrolyl (including N-pyrrolyl, 2-pyrrolyl, 3-pyrrolyl, etc.), pyrazolyl (including 2-pyrazolyl, and 3-pyrazolyl), imidazolyl (including N-imidazolyl, 2-imidazolyl, 4-imidazolyl, and 5-imidazolyl), oxazolyl (including 2-oxazolyl, 4-oxazolyl, and 5-oxazolyl), triazolyl (1H-1,2,3-triazolyl, 2H-1,2,3-triazolyl, 1H-1,2,4-triazolyl, and 4H-1,2,4-triazolyl), tetrazolyl, isoxazolyl (3-isoxazolyl, 4-isoxazolyl, and 5-isoxazolyl), thiazolyl (including 2-thiazolyl, 4-thiazolyl, and 5-thiazolyl), furyl (including 2-furyl, and 3-furyl), thienyl (including 2-thienyl, and 3-thienyl), pyridyl (including 2-pyridyl, 3-pyridyl, and 4-pyridyl), pyrazinyl or pyrimidinyl (including 2-pyrimidinyl and 4-pyrimidinyl).

The term "alkoxy" as used herein refers to -O-(alkyl) and -O-(unsubstituted cycloalkyl), wherein the alky is as defined above. Representative examples of alkoxy include methoxy, ethoxy, propoxy, butoxy, cyclopropoxy, cyclobutoxy, cyclopentyloxy, and cyclohexyloxy. The alkoxy group may be substituted or unsubstituted, and when substituted, the substituent may be preferably one or more groups independently selected from the group consisting of alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxyl, heterocycloalkoxyl, cycloalkylthio, heterocycloalkylthio, carboxyl, or carboxylic ester groups.

The term "haloalkyl" as used herein refers to an alkyl group substituted with one or more halogens.

The term "3- to 8-membered heterocyclyl" used herein means a non-aromatic cyclic group comprising one or more heteroatoms selected from nitrogen, oxygen and sulfur atoms, which may be fully saturated or partially unsaturated. The ring can be a 3- to 8-membered monocyclic ring, bicyclic ring or spiro ring, including but not limited to, oxetanyl, azetidinyl, piperazinyl, piperidinyl, morpholinyl, thiomorpholinyl, pyrrolidinyl, tetrahydropyranyl, tetrahydrofuryl, oxazolidinyl, thiazolidinyl, imidazolidinyl, pyrazolidinyl, thianyl, oxanyl, oxathianyl, dihydroindolyl, dihydroisoindolyl, tetrahydrodihydroindolyl, quinuclidinyl, azepinyl, and the like.

The heterocyclic ring may be fused to an aryl, heteroaryl or cycloalkyl ring, where the ring attached to the parent structure is the heterocyclyl group. Non-limiting examples include

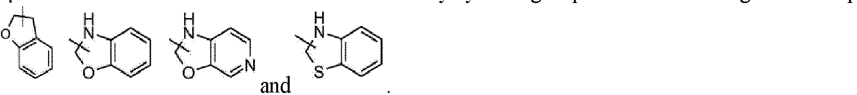

The term "C₃₋₈ cycloalkyl" as used herein means a monovalent group obtained by removing any single hydrogen atom from a cyclic saturated aliphatic hydrocarbon having 3 to 8 carbons, i.e., a cycloalkyl group having 3 to 8 carbons, including, but not limited to cyclopropyl, cyclobutyl, cyclopentyl, cyclohexyl, cycloheptyl, and cyclooctyl. When two groups together form a C₃₋₈ cycloalkyl ring, the resultant group may be divalent, such as cyclopropane-1,1-diyl, cyclobutane-1,1-diyl, cyclopentane-1,1-diyl, cyclohexane-1,1-diyl, cycloheptane-1,1-diyl, and cyclooctane-1,1-diyl. In addition, the cycloalkane ring, carbocyclic ring or cyclic hydrocarbon in the cycloalkyl group may be a cross-linked ring.

The term "fused ring" as used herein refers to a 5- to 20-membered all-carbon polycyclic group, wherein each ring in the system shares a pair of adjacent carbon atoms with other rings in the system, wherein one or more rings may contain one or more double bonds, but there is no ring having a completely conjugated π-electron system. The fused ring is preferably 6- to 14-membered, more preferably 7- to 10-membered. Depending on the number of ring components, the fused ring can be divided into a bicyclic, tricyclic, tetracyclic or polycyclic fused cycloalkyl group, preferably a bicyclic or tricyclic alkyl, more preferably a 5-membered/5-membered or 5-membered/6-membered bicyclic alkyl, including but not limited to

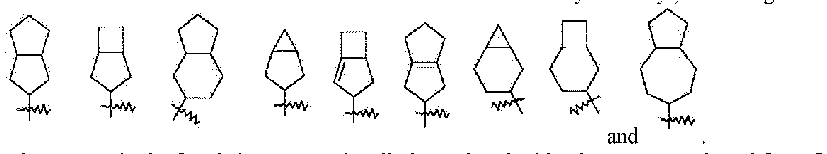

The carbon atoms in the fused ring may optionally be replaced with a heteroatom selected from O, S, and N, i.e., a "fused heterocycle" is also included herein.

The term "fused heterocycle" as used herein refers to an 5- to 20-membered polycyclic heterocyclic group, wherein each ring in the system shares a pair of adjacent atoms with other rings in the system, wherein one or more rings may contain one or more double bonds, but there is no ring having a completely conjugated π-electron system; and one or more of the ring atoms are heteroatoms selected from N, O or S(O)ₜ (wherein t is an integer of 0 to 2), and the remaining ring atoms are carbon. The fused heterocycle is preferably 6- to 14-membered, more preferably 7- to 10-membered. Depending on the number of ring components, the fused heterocycle can be divided into a bicyclic, tricyclic, tetracyclic or polycyclic fused heterocyclic group, preferably a bicyclic or tricyclic fused heterocyclyl, more preferably a 5-membered/3-membered, 5-membered/4-membered or 5-membered/5-membered bicyclic fused heterocvclvl. The fused heterocycle includes but is not limited to:

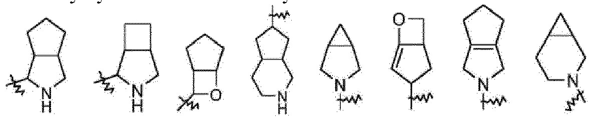

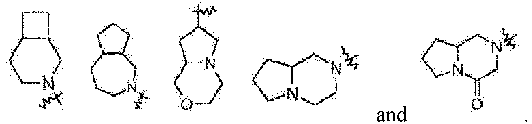

The term "bridged ring" as used herein refers to a 5- to 20-membered all-carbon polycyclic group, wherein any two rings in the system shares a pair of non-adjacent carbon atoms. The bridged ring may contain one or more double bonds, but there is no ring having a completely conjugated π-electron system. The bridged ring is preferably 6- to 14-membered, more preferably 7- to 10-membered. Depending on the number of ring components, the bridged ring can be divided into bicyclic, tricyclic, tetracyclic or polycyclic bridged ring groups, preferably a bicyclic, tricyclic or tetracyclic bridged ring group, more preferably a bicyclic or tricyclic bridged ring group. The bridged ring includes but is not limited to:

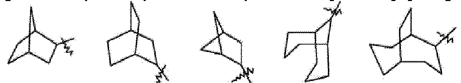

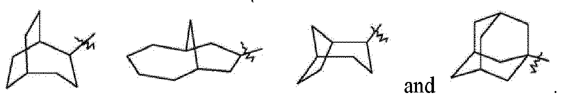

The carbon atoms in the bridged ring may optionally be replaced with a heteroatom selected from O, S, and N, i.e., a "bridged heterocycle" is also included herein.

The term "bridged heterocycle" as used herein refers to a 5- to 14-membered polycyclic heterocyclic group, wherein any two rings in the system shares a pair of non-adjacent carbon atoms, wherein the bridged heterocycle may contain one or more double bonds, but there is no ring having a completely conjugated π-electron system, and wherein one or more of the ring atoms are heteroatoms selected from N, O or S(O)ₘ (wherein m is an integer of 0 to 2), and the remaining ring atoms are carbon. The bridged heterocycle is preferably 6- to 14-membered, more preferably 7- to 10-membered. Depending on the number of ring components, the bridged heterocycle can be divided into bicyclic, tricyclic, tetracyclic or polycyclic bridged heterocycle groups, preferably a bicyclic, tricyclic or tetracyclic bridged heterocycle, more preferably a bicyclic or tricyclic bridged heterocycle. The bridged heterocycle includes but is not limited to

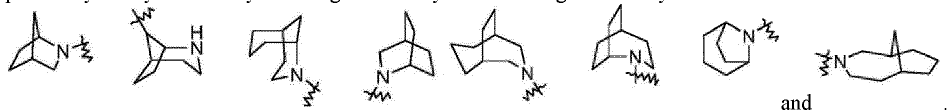

The term "spiro ring" as used herein refers to a 5- to 20-membered polycyclic group, wherein the monocyclic rings in the system shares one carbon atom (called as spiro atom). The spiro ring may contain one or more double bonds, but there is no ring having a completely conjugated π-electron system. The spiro ring is preferably 6- to 14-membered, more preferably 7- to 10-membered. Depending on the number of spiro atoms shared between the rings, the spirocycloalkyl groups are divided into monospirocycloalkyl, dispirocycloalkyl or polyspirocycloalkyl, preferably monospirocycloalkyl and dispirocycloalkyl, more preferably 4-membered/4-membered, 4-membered/5-membered, 4-membered/6-membered, 5-membered/5-membered or 5-membered/6-membered monospirocycloalkyl, including but not limited to

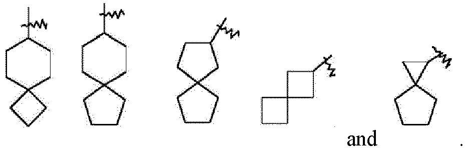

The carbon atoms in the spiro ring may be optionally replaced by a heteroatom selected from O, S, and N, i.e., a "spiro heterocycle" is also included herein.

The term "spiro heterocycle" as used herein refers to a 5- to 20-membered polycyclic heterocyclic group, wherein the monocyclic rings in the system shares one carbon atom (called as spiro atom), wherein one or more of the ring atoms are heteroatoms selected from N, O or S(O)ₘ (wherein m is an integer of 0 to 2) and the remaining ring atoms are carbon. The spiro heterocycle may contain one or more double bonds, but there is no ring having a completely conjugated π-electron system. The spiro heterocycle is preferably 6- to 14-membered, more preferably 7- to 10-membered. Depending on the number of spiro atoms shared between the rings, the spiroheterocyclyl groups are divided into monospiroheterocyclyl, dispiroheterocyclyl or polyspiroheterocyclyl, preferably monospiroheterocyclyl and dispiroheterocyclyl, more preferably 4-membered/4-membered, 4-membered/5-membered, 4-membered/6-membered, 5-membered/5-membered or 5-membered/6-membered monospiroheterocyclyl, including but not limited to

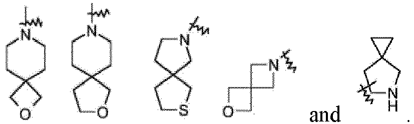

The compounds of the present invention can be prepared by a variety of synthetic methods well known to those skilled in the art, including the following specific embodiments, embodiments formed by their combination with other chemical synthetic methods, and the equivalent alternatives well known to those skilled in the art. Preferred embodiments include, but are not limited to, the examples of the present invention.

The compounds described in the present invention are named according to the chemical structural formula. Where the name of a compound is inconsistent with the chemical structural formula of the same compound, the chemical structural formula shall prevail.

In the present invention, unless defined otherwise, all scientific and technical terms used herein have the same meaning as those commonly understood by a person skilled in the art. Nevertheless, definitions of some terms are provided below for a better understanding of the present invention. Where the definitions and interpretations of the terms provided herein differ from those commonly understood by a person skilled in the art, the definitions and interpretations of the terms provided herein shall prevail.

### Detailed description

The present invention will be further described in detail below with reference to specific examples. The following examples are useful in understanding the method and the core idea of the present invention, and any possible changes or substitutions that can be made by those skilled in the art without departing from the concept of the present invention are within the scope of the present invention. The experimental methods where no specific conditions are indicated in the following examples usually adopt conventional conditions or the conditions suggested by the manufacturer; reagents without specifying sources may be commercially available conventional reagents.

### Experiment 1 - Identification and characterization of compounds

The 1H NMR spectra herein are determined using a Bruker instrument (400MHz), and chemical shifts are reported in ppm. Tetramethylsilane (0.00 ppm) was used as internal standard. 1H NMR was expressed as follows: s = singlet, d = doublet, t = triplet, m = multiplet, br = broad, dd = doublet of doublet, dt = doublet of triplet. The coupling constant, if provided, is expressed in Hz.

The mass spectra of the invention are determined by an LC/MS instrument, and ionization may be carried out by ESI or APCI.

| No. | Compound | H-NMR |
|---|---|---|
| 1 | 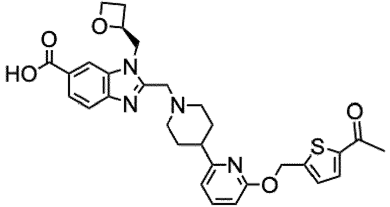 | ¹H NMR (400 MHz, DMSO-*d6*) δ 8.04 (s, 1H), 7.81 (d, *J* = 3.8 Hz, 1H), 7.75 (d, *J* = 8.1 Hz, 1H), 7.67-7.60 (m, 1H), 7.41 (s, 1H), 7.29 (d, *J* = 3.8 Hz, 1H), 6.91 (d, *J =* 7.2 Hz, 1H), 6.67 (d, *J* = 8.4 Hz, 1H), 5.58 (s, 2H), 5.09 (s, 1H), 4.69 (s, 1H), 4.59 (d, *J =* 13.7 Hz, 1H), 4.44 (s, 1H), 4.37 (d, *J* = 9.1 Hz, 1H), 3.90 *(d, J* = 13.5 Hz, 1H), 3.75 (d, *J* = 13.2 Hz, 1H), 2.99 (s, 1H), 2.87 (s, 1H), 2.67 (s, 2H), 2.48 (s, 3H), 2.34 - 2.12 (m, 3H), 1.82 (d, *J* = 12.1 Hz, 4H). |
| 2 | 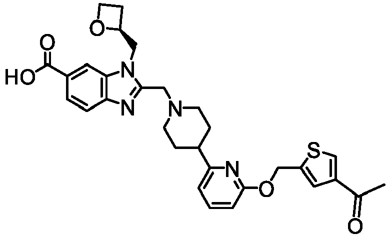 | ¹H NMR (400 MHz, DMSO-*d6*) δ 8.41 (1 H, d, *J* =1.4), 8.25 (1 H, s), 7.80 (1 H, dd, *J* =8.4, 1.5), 7.63 (2 H, dd, *J* = 12.1, 5.7), 7.56 (1 H, d, *J* =1.3), 6.90 (1 H, d, *J* = 7.2), 6.64 (1 H, d, *J* = 8.1), 5.61 - 5.49 (2 H, m), 5.11 (1 H, dt, *J* = 7.0, 4.4), 4.79 (1 H, dd, *J* = 15.2, 7.2), 4.65 (1 H, dd, *J* = 15.2, 2.7), 4.39 (2 H, ddt, *J* =11.8, 8.9, 5.9), 3.96 (1 H, d, .7=13.5), 3.78 (1 H, d*, J* =13.5), 3.03 (1 H, d, *J* = 10.5), 2.88 (1 H, d, *J* = 11.5), 2.74 - 2.61 (2 H, m), 2.44 (4 H, s), 2.31 - 2.15 (2 H, m), 1.92 -1.73 (4 H, m). |
| 3 | 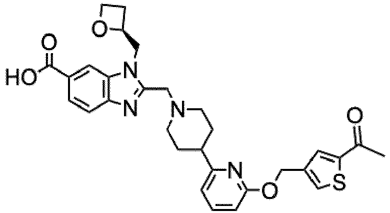 | ¹H NMR (400 MHz, DMSO-*d6*) δ 8.13 (s, 1H), 7.98 (d, *J =* 5.2 Hz, 2H), 7.80 (d, *J* = 8.3 Hz, 1H), 7.63 (t, *J* = 7.7 Hz, 1H), 7.47 (d, *J* = 8.3 Hz, 1H), 6.88 (d, *J* = 7.3 Hz, 1H), 6.66 (d, *J* = 8.2 Hz, 1H), 5.36 - 5.29 (m, 2H), 5.17 - 5.06 (m, 1H), 4.66 (ddd, *J* = 17.8, 15.2, 4.7 Hz, 2H), 4.51 - 4.32 (m, 2H), 3.92 (d, *J* = 13.4 Hz, 1H), 3.77 (d, *J* = 13.4 Hz, 1H), 2.95 (dd, *J =* 45.6, 11.0 Hz, 2H), 2.67 (dt, *J* = 26.5, 9.7 Hz, 2H), 2.48 (s, 3H), 2.47 - 2.40 (m, 1H), 2.30 - 2.12 (m, 2H), 1.90 - 1.65 (m, 4H). |
| 4 | 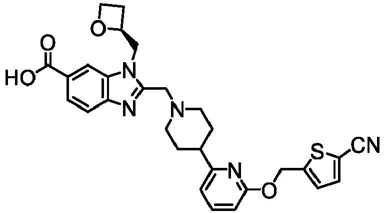 | ¹H NMR (400 MHz, DMSO-*d6*) δ 8.15 (s, 1H), 7.88 (d, *J* = 3.8 Hz, 1H), 7.80 (dd, *J* = 8.4, 1.2 Hz, 1H), 7.67 (t, *J* = 7.8 Hz, 1H), 7.51 (d, *J* = 8.4 Hz, 1H), 7.37 (d, *J =* 3.8 Hz, 1H), 6.93 (d, *J* = 7.3 Hz, 1H), 6.68 (d, *J* = 8.2 Hz, 1H), 5.62 (s, 2H), 5.11 (qd, *J* = 7.1, 3.2 Hz, 1H), 4.69 (ddd, *J* = 18.0, 15.2, 5.0 Hz, 2H), 4.50 - 4.34 (m, 2H), 3.94 (d, *J* = 13.4 Hz, 1H), 3.78 (d, *J* = 13.4 Hz, 1H), 3.02 (d, *J* = 11.4 Hz, 1H), 2.90 (d, *J* = 11.1 Hz, 1H), 2.68 (m, 2H), 2.48 - 2.39 (m, 1H), 2.30 - 2.13 (m, 2H), 1.92 - 1.69 (m, 4H). |
| 5 | 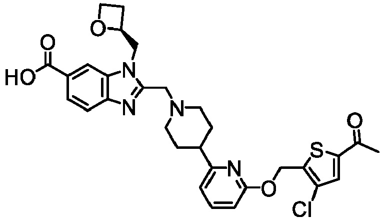 | ¹H NMR (400 MHz, DMSO-*d6*) δ 8.10 (1 H, s), 7.97 (1 H, s), 7.80 (1 H, d, *J* = 8.4), 7.66 (1 H, t, *J* = 7.7), 7.44 (1 H, d, *J* =8.3), 6.92 (1 H, d, *J* =7.3), 6.70 (1 H, d, *J* = 8.2), 5.56 (2 H, s), 5.17 - 5.03 (1 H, m), 4.73 (1 H, dd, *J* =15.2, 6.9), 4.64 - 4.56 (1 H, m), 4.47 (1 H, dd, *J* =13.9, 7.3), 4.38 (1 H, dt, *J* = 11.8, 5.9), 3.91 (1 H, d, *J* = 13.3), 3.76 (1 H, d, *J* = 13.3), 2.99 (1 H, d, *J* =10.8), 2.88 (1 H, d, *J* = 10.9), 2.66 (2 H, dd, *J* = 13.1, 5.5), 2.50 (3 H, s), 2.46 (1 H, d, *J* = 8.8), 2.27 - 2.10 (2 H, m), 1.79 (4 H, dd, *J* = 16.6, 10.3). |
| 6 | 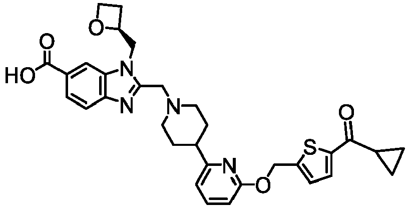 | 1H NMR (400 MHz, DMSO-d6)δ 8.13 (s, 1H), 8.00 (d, *J* = 3.8 Hz, 1H), 7.78 (d, *J* = 8.4 Hz, 1H), 7.65 (t, *J* = 7.8 Hz, 1H), 7.49 (d, *J* = 8.4 Hz, 1H), 7.32 (d, *J =* 3.8 Hz, 1H), 6.90 (d, *J* = 7.2 Hz, 1H), 6.67 (d, *J* = 8.2 Hz, 1H), 5.59 (s, 2H), 5.13-5.06 (m, 1H), 4.78-4.69 (m, 1H), 4.65-4.55 (m, 1H), 4.49-4.41 (m, 1H), 4.40-4.32(m, 1H), 3.92 (d, J = 13.4 Hz, 1H), 3.76 (d, *J =* 13.4 Hz, 1H), 3.17 (s, 1H), 3.00 (d, *J =* 11.2 Hz, 1H), 2.87 (d, *J* = 11.2 Hz, 1H), 2.80-2.73 (m, 1H), 2.69 - 2.62 (m, 2H), 2.46 - 2.40 (m, 1H), 2.21 (m, 2H), 1.78 (m, 4H), 1.00 - 0.95 (m, 4H). |
| 7 | 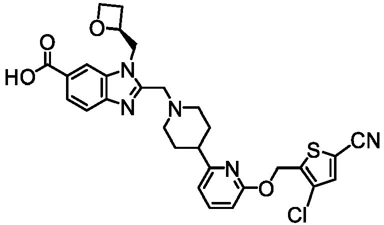 | δ H (400 MHz, DMSO-*d6*) 8.38 (1 H, s), 8.23 (1 H, s), 8.06 (1 H, s), 7.82 (1 H, d, *J* = 8.4), 7.68 (1 H, t, *J* = 7.8), 7.58 (1 H, d, *J* = 8.3), 6.94 (1 H, d, *J* = 7.3), 6.71 (1 H, d, *J* = 8.1), 5.58 (2 H, s), 5.11 (1 H, d, *J* = 4.2), 4.79 (1 H, dd, *J* = 15.0, 6.9), 4.65 (1 H, d, *J* = 13.1), 4.47 (1 H, dd, *J* = 13.5, 7.4), 4.41 - 4.32 (1 H, m), 3.94 (1 H, d, *J* = 13.5), 3.83 - 3.73 (2 H, m), 3.00 (2 H, d, *J* = 11.5), 2.88 (1 H, d, *J* = 11.8), 2.67 (2 H, d, *J* = 8.0), 2.45 (1 H, s), 2.29 - 2.12 (2 H, m), 1.78 (4 H, dd, *J* = 37.9, 13.2). |
| 8 | 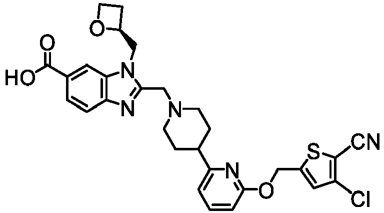 | 1H NMR (400 MHz, DMSO-d6) δ 8.06 (s, 1H), 7.77 (d, *J* = 7.6 Hz, 1H), 7.72 - 7.64 (m, 1H), 7.50 (s, 1H), 7.42 (d, *J* = 8.2 Hz, 1H), 6.94 (d, *J* = 7.3 Hz, 1H), 6.70 (d, *J =* 8.2 Hz, 1H), 5.58 (s, 2H), 5.16 - 5.05 (m, 1H), 4.72 (dd, *J* = 15.3, 7.1 Hz, 1H), 4.60 (dd, *J =* 15.6, 3.2 Hz, 1H), 4.47 (dd, *J* = 13.7, 7.7 Hz, 1H), 4.36 (dt, *J =* 8.8, 5.9 Hz, 1H), 3.91 (d, *J =* 13.3 Hz, 1H), 3.77 (d, *J* = 13.3 Hz, 1H), 3.00 (d, *J* = 10.2 Hz, 1H), 2.90 (d, *J* = 11.4 Hz, 1H), 2.67 (dt, *J* = 13.4, 7.0 Hz, 2H), 2.43 (dd, *J* = 18.9, 7.9 Hz, 1H), 2.29 - 2.13 (m, 2H), 1.90 - 1.69 (m, 4H). |
| 9 | 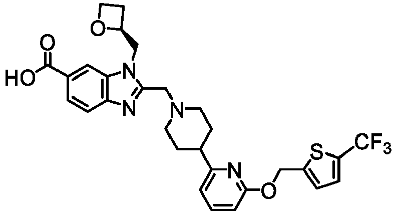 | 1H NMR (400 MHz, DMSO-d6) δ 8.06 (s, 1H), 7.76 (d, *J =* 8.2 Hz, 1H), 7.68 - 7.62 (m, 1H), 7.62 - 7.59 (m, 1H), 7.42 (d, *J =* 8.0 Hz, 1H), 7.32 (s, 1H), 6.92 (d, *J* = 7.2 Hz, 1H), 6.67 (d, *J* = 8.0 Hz, 1H), 5.60 (s, 2H), 5.10 (d, *J* = 4.2 Hz, 1H), 4.75-4.67 (m, 1H), 4.63 - 4.55 (m, 1H), 4.49-4.42 (m 1H), 4.40-4.32 (m, 1H), 3.91 (d, *J* = 13.4 Hz, 1H), 3.76 (d, *J* = 13.4 Hz, 1H), 3.01 (d, *J* = 11.0 Hz, 1H), 2.90 (d, *J* = 7.6 Hz, 1H), 2.71 - 2.61 (m, 2H), 2.47 - 2.38 (m, 1H), 2.30 - 2.12 (m, 2H), 1.80 (m, 4H). |
| 10 | 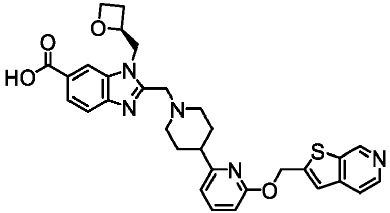 | ¹H NMR (400 MHz, DMSO) δ 9.16 (s, 1H), 8.41 (d, *J* = 5.4 Hz, 1H), 8.06 (s, 1H), 7.76 (d, *J* = 6.5 Hz, 2H), 7.69 - 7.64 (m, 1H), 7.59 (s, 1H), 7.41 (d, *J =* 8.3 Hz, 1H), 6.92 (d, *J* = 7.3 Hz, 1H), 6.71 (d, *J* = 8.2 Hz, 1H), 5.74 (s, 2H), 5.11 (m, *J* = 6.9, 3.2 Hz, 1H), 4.70 (dd, *J =* 15.2, 6.8 Hz, 1H), 4.58 (dd, *J* = 15.1, 3.1 Hz, 1H), 4.47 - 4.31 (m, 2H), 3.90 (d, *J* = 13.4 Hz, 1H), 3.75 (d, *J* = 13.4 Hz, 1H), 3.00 (d, *J* = 11.0 Hz, 1H), 2.89 (d, *J* = 11.4 Hz, 1H), 2.69 - 2.62 (m, 2H), 2.42 (dd, *J* = 11.1, 4.1 Hz, 1H), 2.27 - 2.13 (m, 2H), 1.90 - 1.72 (m, 4H). |
| 11 | 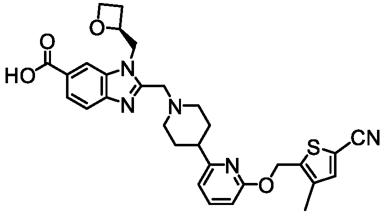 | ¹H NMR (400 MHz, DMSO-*d6*) δ 8.32 (s, 1H), 8.24 (s, 1H), 7.81 (d, *J* = 8.3 Hz, 1H), 7.74 (s, 1H), 7.67 - 7.59 (m, 2H), 6.91 (d, *J* = 7.4 Hz, 1H), 6.67 (d, *J* = 8.2 Hz, 1H), 5.55 (s, 2H), 5.11 (q, *J* = 8.5 Hz, 1H), 4.80 (dd, *J* = 15.4, 6.9 Hz, 1H), 4.66 (d, *J* = 13.3 Hz, 1H), 4.47 (dd, *J* = 13.6, 7.4 Hz, 1H), 4.37 (m, 1H), 3.95 (d, *J* = 13.5 Hz, 1H), 3.79 (d, *J* = 13.4 Hz, 1H), 3.00 (s, 1H), 2.89 (s, 1H), 2.74 - 2.60 (m, 4H), 2.46 (s, 1H), 2.29 (s, 3H), 1.84 - 1.71 (m, 4H). |
| 12 | 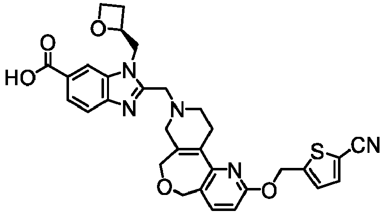 | ¹H NMR (400 MHz, MeOD) δ 8.18 (s, 1H), 7.95 (dd, *J* = 8.4, 1.5 Hz, 1H), 7.65 - 7.57 (m, 3H), 7.23 (d, *J* = 3.8 Hz, 1H), 6.70 (d, *J* = 8.2 Hz, 1H), 5.64 (s, 2H), 5.27 (d, *J* = 6.7 Hz, 1H), 4.72 (dd, *J* = 15.3, 3.0 Hz, 3H), 4.61 (dd, *J* = 13.7, 7.9 Hz, 2H), 4.46 (dd, *J* = 5.9, 3.1 Hz, 1H), 4.37 (s, 2H), 4.12 (dd, *J* = 34.0, 13.6 Hz, 2H), 3.90 (s, 2H), 2.85 (s, 4H), 2.75 (d, *J* = 6.5 Hz, 1H), 2.57 - 2.49 (m, 1H). |
| 13 | 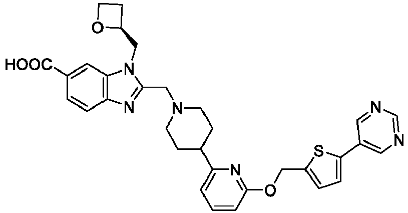 | ¹H NMR (400 MHz, DMSO) δ 9.09 (s, 1H), 9.06 (s, 2H), 8.26 (s, 1H), 7.81 (dd, *J* = 8.0Hz, 1H), 7.65 (m, 3H), 7.32 (d, *J* = 4.0 Hz, 1H), 6.91 (d, *J* = 8.0 Hz, 1H), 6.66 (d, *J* = 8.0 Hz, 1H), 5.67 - 5.54 (m, 2H), 5.11 (m,1H), 4.79 (dd, *J*₁= 8.0 Hz, *J*₂= 16.0 Hz 1H), 4.65 (dd, *J* = 12.0 Hz, 1H), 4.44 (m, 1H), 4.36 (m, 1H), 3.97 (d, *J* = 16.0 Hz, 1H), 3.82 (d, *J* = 16.0 Hz, 1H), 3.04 (d, *J* = 12.0 Hz, 1H), 2.89 (d, *J* = 12.0 Hz, 1H), 2.69 (m, 2H), 2.42 (m, 1H), 2.31 - 2.15 (m, 2H), 1.96 - 1.72 (m, 4H). |

### Experiment 2 - Biological assays

### (1) Test instruments and reagents

| Instruments/reagents | Supplier | Model |
|---|---|---|
| cAMP-GS DYNAMIC kit | CisBio | 62AM4PEC |
| DMEM | CellMax | CGN101.5 |
| FBS | Gemini | 900-108 |
| 1% Pen-3trep | Sangom biotech | E607011-0100 |
| IBMX | Meilunbio | MB5226 |
| 384 well plate | Coming | 3824 |
| Incubator | Thermo | 3111 |
| Microscope | Jiangnan | XD-202 |
| Cell counter | Counter Star | Star IC1000 |
| Plate reader | Tecan | Tecan Spark |

### (2) GLP-1R kit

GLP-1R-mediated agonist activity was determined by cell-based assays using a homogeneous time-resolved fluorescence (i.e., HTRF)-based cAMP detection kit, which measures the level of cAMP in cells. The method was a competitive immunoassay. It enabled direct pharmacological characterization of compounds acting on Gs-coupled receptors in adherent or suspending cells.

The standard curve of native cAMP or unlabeled cAMP produced by cells competed with d2-labeled cAMP red receptors to bind monoclonal anti-cAMP Eu3+ cryptate donors, and the specific signal was inversely proportional to the concentration of cAMP in standard or tested samples.

Human GLP-1R encoding sequence (NCBI reference sequence NP_002053.3) was subcloned into pEGFP-N1 (tsingke), and the cell line stably expressing the receptor was isolated. The expression density of GLP-1R was confirmed by the expression of GFP observed under a fluorescence microscope.

### (3) GLP-1R-GFP-293A cell culture

293A GFP-GLP-1R cells were incubated in DMEM growth medium, 10% heat-inactivated fetal bovine serum (GEMINI Cat # 900-108), 1% Pen-3Trep (Sangom Biotech Cat # E607011-0100)] in a moist incubator with 5% COz at 37 °C.

### (4) cAMP level test method

The tested compounds (in DMSO) at different concentrations were 1:5 diluted in distilled water in a stimulating buffer, followed by addition of 500 µm 3-isobutyl-1-methylxanthine (IBMX; Meilunbiocat # MB5226) to obtain a working solution of 2X compound, and then 5 µL of the compound was added to a white 384-well assay plate (Corning 3824) using a multi-channel pipette. The final DMSO concentration in the buffer mixture was determined to be 1 %o.

Cells were collected from a T25 tissue culture flask and centrifuged at room temperature at 1000 rpm for 5 minutes. The cell precipitates were then re-suspended in 1 mL of the stimulating buffer. 20 µL sample of cell suspension was counted on a counter STAR IC 1000 to determine the cell viability and the cell count per mL. The remaining cell suspension was then regulated with the stimulating buffer to deliver 2000 living cells per well using a multi-channel pipette. 5 µL of the cell suspension was added to each well of the plate which already contained the compound. The plate was sealed and incubated at 37 °C with 5% COz for 30 minutes.

After 30 minutes of incubation, 5 µL of d2-labeled cAMP and 5 µL of anti-cAMP cryptate (both 1: 20 diluted in the cell lysis buffer) were added to each well of the plate. The plate was then incubated at room temperature for 60 minutes, and the changes of HTRF signal were read with Tecan Spark reader: absorbance values at 340 nm (excitation)/at 615 nm and 665 nm (emission). Raw data were converted into nM cAMP by interpolation from the cAMP standard curve, and the effect in percentage was determined relative to the saturated concentration of the complete agonist GLP-17~37 (400 nM) contained in each plate. Determination of EC50 was performed based on the agonist dose-response curve, which was analyzed using a four-parameter logical dose-response equation with a curve fitting program.

This test proved that the compound of the invention activated GLP-1R signaling through the cAMP pathway, thus acting as a GLP-1R agonist. The test data presented the results in the form of a geometric mean (EC50s) based on the number of repetition times.

### Experimental results

| Compound No. | EC₅₀ (nM) |
|---|---|
| 1 | 4.67 |
| 2 | 88.78 |
| 3 | 121 |
| 4 | 1.0 |
| 5 | 0.56 |
| 6 | 0.03 |
| 7 | 0.02 |
| 8 | 0.05 |
| 9 | 0.08 |
| 10 | 0.05 |
| 11 | 0.1 |
| 12 | 0.5 |
| 13 | 0.47 |

### Experiment 3 - Test for inhibition of hERG potassium channels

| 1. Experimental materials: stable cell line HEK-hERG, strain: HEK 293, source: Academy of Military Medical Sciences; | | |
|---|---|---|
| Instrument | Model | Supplier |
| Manual patch clamp system | EPC 10 USB PatchMaster software | HEKA Elektronik |
| Rapid perfusion system | ALA-VM8 | ALA Scientific Ins. |
| Micro manipulator | MPC200 | Sutter Instrument Co. |
| Inverted microscope | TI-FL | Nikon |
| Microelectrode puller | PC-10 | NARISHIGE |
| Vibration isolation table | 637512M | TMC |
| Peristaltic pump | LEAD 15-24 | Longer pump |

### 2. Electrophysiological solution

Extracellular fluid (mM): N-2-hydroxyethylpiperazine-N'-2-ethanesulfonic acid (HEPES) 10, NaCl 145, KCl 4, CaCl₂ 2, MgCl₂ 1, Glucose 10, a pH adjusted to 7.3-7.4 with sodium hydroxide; an osmotic pressure adjusted to 290-310 mOsm; stored at 4 °C after filtration.

Pippette solution (mM): KCl 120, KOH 31.25, CaCl₂ 5.374, MgCl₂ 1.75, ethylene glycol-bis(β-aminoethyl ether)-N,N,N',N'-tetraacetic acid (EGTA) 10, HEPES 10, Na₂-ATP 4, a pH adjusted to 7.2-7.3 with potassium hydroxide; an osmotic pressure adjusted to 290-310 mOsm; packed after filtration and stored at -20 °C.

### 3. Positive control compound

Positive control: Amitriptyline hydrochloride or Terfenadine
Source: Sigma-Aldrich

### 4. Preparation of the dosed formulations

Preparation of the solvent control: a certain volume of DMSO was added to the extracellular fluid to the same content of DMSO as in the final test solution (if the test solution contained a different content of DMSO, the maximum DMSO content shall prevail), so as to eliminate the interference of DMSO on the own current of cells.

Preparation of test samples: the above 10 mM mother liquor was prepared into a DMSO stock solution with a desired concentration (generally 1000/3 times of the actual dosing concentration), which was finally diluted with the extracellular fluid to the desired dosing concentration for the experiment.

Preparation of the positive control solution: a proper amount of the positive control compound was weighed and placed in a suitable container, followed by addition of a certain volume of DMSO, and extensive stirring or shaking to dissolve all positive control compound to prepare a 10 mM stock solution, which was then proportionally prepared into a stock solution with a desired concentration. The resulting solution was finally diluted with the extracellular fluid to a desired dosing concentration for the experiment.

Before using the solution with the working concentration, whether precipitation occurred was checked. If precipitation occurred, the stock solution was diluted to raise the final concentration of DMSO in the extracellular fluid, but the final concentration of DMSO in the extracellular fluid should not exceed 0.5%. Continuous perfusion from low concentration to high concentration was adopted in the experiment. After the experiment was complete, the remaining dosing solutions of the test sample and the positive control were treated as waste liquids.

### 5. Experimental protocol

### Preparation of Cells

After the passage and culture of HEK-293-hERG cells to a proper state, the cells were washed with PBS (or DPBS), digested and separated with Tryple solution, and then resuspended in the medium and stored in a centrifuge tube. After centrifugation, the supernatant was discarded, and the cells were resuspended in the extracellular fluid for later use and stored at 2-8 °C. Before patch clamp recording, the cells were dropped into a culture dish to ensure that the cells had a certain density and were isolated from one another.

### Concentration settings:

| Tested sample/positive control sample | Concentration (µM) |
|---|---|
| The compounds of the invention | 1-10 |
| Amitriptyline hydrochloride or Terfenadine | 1 |

### Electrophysiological test

A whole-cell patch clamp technique was used to record hERG current. The cell suspension was added to a small petri dish and placed on an inverted microscope stage. After adherence, the cells were perfused with the extracellular fluid at a recommended flow rate of 1-2 mL/min. The glass microelectrode was made by two-step pulling using a microelectrode puller, and had a resistance of 2 to 5 MΩ in water after filled with the electrode interior liquid.

After the whole-cell recording mode was set up, the clamping potential was maintained at -80 mV. The depolarization voltage was applied to +60 mV for 850 ms, and then repolarized to -50 mV for 1275 ms to induce hERG tail current. Such a set of pulse programming was repeated every 15 seconds throughout the experiment.

After the current was stable, a dosing mode was applied using extracellular continuous perfusion from low to high concentrations. Starting from a low concentration, perfusion continued until the efficacy was stable, then perfusion at a next concentration was performed. In this experiment, the blocking effect of the test sample and the positive control on hERG tail current was tested (N ≥ 2); and the actual concentration could be adjusted according to the actual solubility and effect, which was not regarded as deviation from the protocol.

Stable efficacy was defined as follows: it was considered as stable that the change of the current value in the last five stimulations during the dosing at each concentration was less than 10% of the average value (when the current was greater than or equal to 200 pA) or less than 30% of the average value (when the current was less than 200 pA); if unstable, data for the concentration would not be adopted.

### 6. Data analysis

In data processing, when determining the blocking effect on hERG, the peak value and baseline of tail current were calibrated. The inhibition rate (IR) of tail current was used to represent the effects of the compounds at different concentrations. An SD ≤ 15 of the %IR for all cells at various concentrations was considered an acceptable standard (except for abnormal data).

IR = 100% × (the peak value of the tail current before dosing - the peak value of the tail current after dosing)/the peak value of the tail current before dosing.

### 7 Experimental results:

| Compound No. | hERG (IR) |
|---|---|
| 1 | 9.82%(1 µM) |
| | 53.82 (10 µM) |
| 4 | 14.24%(1 µM) |
| | 72.22 (10 µM) |

### 8. Experimental conclusion: The compound of the present invention did not exhibit hERG inhibitory activity.

### Experiment 4 - Metabolic stability in (human) liver microsomes

1. Experimental design: test concentration: 1 µM; control compound: testosterone; culture conditions: cultured at 37 °C for 0, 5, 15, 30, 45 minutes; method of determination: LC-MS/MS; calculation method: T_{1/2}= 0.693/K (K is the rate constant of the ln [concentration] vs. incubation time profile), Clᵢₙₜ = (0.693/T_{1/2}) × (1/(microsomal protein concentration (0.5 mg/mL))) × scaling factor.
The scaling factors for predicting the intrinsic clearance in human microsomes are provided in the following table:

| Species | Microsome protein | Liver weight/ kg body weight | Scaling factor | Hepatic blood flow (mL/min/kg) |
|---|---|---|---|---|
| | /g liver | | | |
| Mouse | 45 | 87.5 | 3937.5 | 90 |
| Rat | 44.8 | 40 | 1792 | 55.2 |
| Monkey | 45 | 32.5 | 1462.5 | 44 |
| Human | 48.8 | 25.7 | 1254.2 | 20.7 |
| Scaling factor = (microsome protein/g liver) × (liver weight/kg body weight) | | | | |

2. Experimental method: 1. preheating 0.1 M K-buffer, 5 nM MgCl₂, pH=7.4; 2. test solutions of test compound and reference compound, 500 µM additive solution: 5 µL of 10 mM stock solution was added to 95 µL can; 1.5 µM additive solution of microsome (0.75 Mg/mL): 1.5 µL of 500 µM additive solution and 18.75 µL of 20 Mg/mL liver microsome were added to 479.75 µL of K/Mg buffer; 3. 3 x NADPH stock solution (6 mM, 5 mg/mL) was prepared by dissolving NADPH in the buffer solution; 4. 30 µL of 1.5 µM additive solution containing 0.75 mg/mL microsomal solution was distributed to the plates designated for different time points (0, 5, 15, 30, 45 minutes); 5. at 0 min, 150 µL of ACN containing IS was added to the wells of the plate, followed by addition of 15 µL of NADPH stock solution (6 mM, step 3); 6. all other plates were pre-incubated at 37 °C for 5 minutes; 7. adding 15 µL of NADPH stock solution to the plate to start the reaction and timing; 8. 150 µL of ACN containing IS was added to the wells of the corresponding plates to stop the reaction at 5 min, 15 min, 30 min and 45 min, respectively; 9. after quenching, the plates were shaken on a shaker for 10 minutes (600 rpm/min) and then centrifuged at 6000 rpm for 15 minutes; 10. 80 µL of supernatant was transferred from each well to a 96-well sample plate containing 140 µL water for LC/MS analysis.
3. Analysis method
Detection method: LC-MS/MS-11 (8050), internal standard: tolbutamide; MS conditions: positive ion ESI for testosterone and test compound, and negative ion ESI for tolbutamide; Mobile phases: mobile phase A is 0.1% FA in water, and mobile phase B is 0.1% FA in ACN; Column and specification: ACQUITY UPLC HSS T3 1.8 um 2.1*50 mm.

### LC conditions:

| Testosterone | | Test compound | |
|---|---|---|---|
| 0.60 mL/min | | 0.60 mL/min | |
| Time | Pump B | Time | Pump B |
| 0.01 | 10 | 0.01 | 10 |
| 0.5 | 90 | 0.3 | 95 |
| 1.5 | 90 | 1 | 95 |
| 1.51 | 10 | 1.01 | 10 |
| 1.8 | Stop | 1.5 | Stop |

### 4. Experimental results (human microsomes)

| Compound No. | LMS(t_{1/2} min) |
|---|---|
| 1 | 114.9 |
| 4 | 132.3 |
| 5 | 37.33 |
| 7 | 21.25 |
| 10 | 22.54 |
| 12 | 59.84 |

### 5. Experimental conclusion: the compounds of the invention exhibited good stability in liver microsomes.

### Preparation examples

The intermediate reaction materials used in the preparation process were prepared with reference to the preparation method described in WO2018109607A1.

### Preparation methods for intermediates

### Intermediate Int-2, (S)-methyl 2-(chloromethyl)-1-(oxetan-2-ylmethyl)-1H-benzo[d]imidazole-6-carboxylate was prepared as follows:

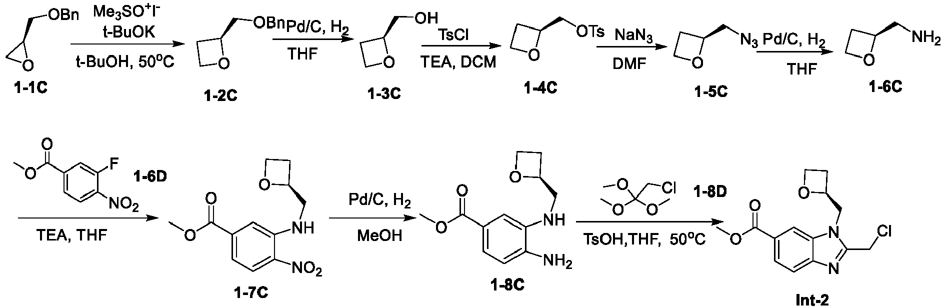

### (1) Preparation of Compound 1-2C

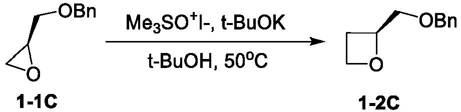

Me₃SO⁺ I⁻ (335 g, 1520 mmol, 2.5 eq) was added in batches to a stirred solution of t-BuOK (170 g, 1520 mmol, 2.5 eq) in t-BuOH (500 mL) under argon atmosphere at 60 °C, and after 30 minutes, (S)-2-((benzyloxy)methyl)ethylene oxide 1-1C, (100 g, 610 mmol, 1.00 eq) was added dropwise to the mixture. The resulting mixture was further stirred at 60 °C for 13 hours. The mixture was cooled to room temperature, then filtered, and the filter cake was washed with EtOAc (3 × 200 mL). The combined organic layers were washed with brine (200 mL), dried with Na₂SO₄ and concentrated in vacuum to obtain a residue, which was then purified by silica gel column chromatography and eluted with PE/EtOAc (10: 1) to give 1-2C, (S)-2-((benzyloxy)methyl)oxetane (50.0 g, 46% yield).

¹H NMR (400 MHz, CDCl3) δ = 7.39 - 7.26 (m, 5H), 5.04 - 4.90 (m, 1H), 4.73 - 4.50 (m, 4H), 3.64 (qd, *J* = 11.0, 4.3 Hz, 2H), 2.72 - 2.45 (m, 2H).

### (2) Preparation of Compound 1-3C

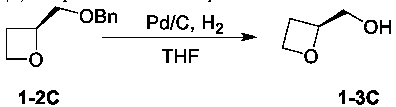

A solution of (S)-2-((benzyloxy)methyl)oxetane 1-2C (50 g, 280.9 mmol, 1.0 eq) and Pd/C (20 g, wet) in THF (200 mL) was stirred under H₂ (4 MPa) at 50 °C for 16 hours. The mixture was cooled to room temperature, then filtered, and the filter cake was washed with THF (100 mL). The filtrate was concentrated in vacuum to give 1-3C, (S)-oxetan-2-ylmethanol (28 g, crude product), which was directly used in the next step.

### (3) Preparation of Compound 1-4C

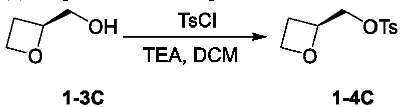

TsCl (66.6 g, 349.6 mmol, 1.1 equiv.) and TEA (48.2 g, 476.7 mmol, 1.5 equiv.) were added to a solution of (S)-oxetan-2-ylmethanol 1-3C (28 g, 317.8 mmol, 1 equiv.) in THF (200 mL) at 25 °C. The mixture was stirred at room temperature for 2 hours. The mixture was diluted with H₂O (100 mL) and extracted with DCM (100 mL × 3). The combined organic layers were dried with Na₂SO₄, filtered and concentrated to obtain a residue, which was then purified by silica gel column chromatography and eluted with PE/EtOAc (0-10%) to give 1-4C, (S)-oxetan-2-ylmethyl 4-toluenesulfonate (56 g, 72.7% yield).

¹H NMR (400 MHz, CDCl3) δ =7.85 - 7.79 (m, 2H), 7.35 (dd, *J* = 8.6, 0.6 Hz, 2H), 5.00 - 4.83 (m, 1H), 4.68 - 4.38 (m, 2H), 4.16 (d, *J* = 4.0 Hz, 2H), 2.78 - 2.64 (m, 1H), 2.58 (d, *J* = 9.0 Hz, 1H), 2.45 (s, 3H).

### (4) Preparation of Compound 1-5C

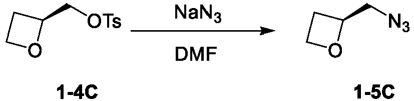

NaN₃ (22.5 g, 346.7 mmol, 1.5 equiv.) was added to a solution of (S)-oxetan-2-ylmethyl-4-toluenesulfonate 1-4C (56 g, 231 mmol, 1 equiv.) in DMF (200 mL). The mixture was stirred at 60 °C for 12 minutes. The mixture was diluted with H₂O (100 mL) and extracted with EtOAc (100 mL × 3). The combined organic layers were dried with Na₂SO₄, filtered and concentrated to give 1-5C, (S)-2-(azidomethyl)oxetane (20 g, crude product), which was directly used in the next step.

### (5) Preparation of Compound 1-6C

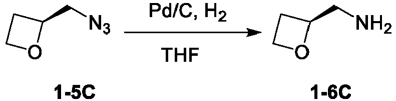

A solution of (S)-2-(azidomethyl)oxetane 1-5C (20 g, crude product) and Pd/C (8 g) in THF (100 mL) was stirred under H₂ (15 Psi) at 25°C for 16 hours. The resulting mixture was filtered and the filter cake was washed with THF (3 × 100 mL). The filtrate was directly concentrated to give 1-6C, (S)-oxetan-2-ylmethylamine (3.8 g, crude product).

¹H NMR (400 MHz, DMSO) δ = 4.60 (dq, *J* = 6.5, 5.2 Hz, 1H), 4.52 - 4.43 (m, 1H), 4.40 - 4.30 (m, 1H), 2.67 (t, *J=* 5.5 Hz, 2H), 2.57 - 2.51 (m, 1H), 2.38 (ddt, *J* = 10.8, 9.0, 7.0 Hz, 2H).

### (6) Preparation of Compound 1-7C

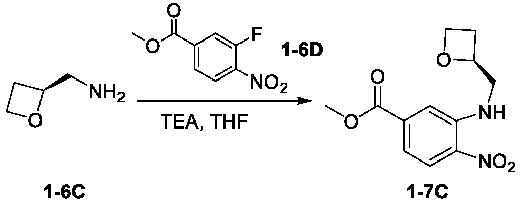

Methyl 3-fluoro-4-nitrobenzoate 1-6D (8.69 g, 43.6 mmol, 1.0 equiv.) and TEA (8.83 g, 87.2 mmol, 2 equiv.) were added to a solution of (S)-oxetan-2-ylmethylamine 1-6C (3.8 g, 43.6 mmol, 1 equiv.) in THF (80 mL) at 25 °C. The mixture was stirred at 40 °C for 6 hours. The mixture was concentrated to give a residue, which was purified by silica gel column chromatography and eluted with (EtOAc/petroleum ether = 0-80%) to give 1-7C, (S)-methyl 4-nitro-3-((oxetan-2-ylmethyl)amino)benzoate (6.2 g, 53.4% yield).

¹H NMR(400 MHz, CDCl3) δ = 8.36 (s, 1H), 8.23 (d, *J* = 8.9 Hz, 1H), 7.63 (d, *J* = 1.4 Hz, 1H), 7.26 (dd, *J* = 8.8, 1.7 Hz, 1H), 5.16 (tt, *J* = 7.4, 4.5 Hz, 1H), 4.81 - 4.55 (m, 2H), 3.94 (s, 3H), 3.71 - 3.55 (m, 2H), 2.84 - 2.72 (m, 1H), 2.70 - 2.52 (m, 1H).

### (7) Preparation of Compound 1-8C

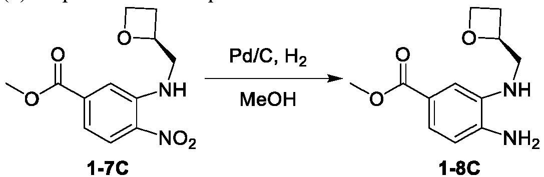

A solution of (S)-methyl 4-nitro-3-((oxetan-2-ylmethyl)amino)benzoate 1-7C (6.2 g, 23.3 mmol, 1.0 equiv.) and Pd/C (1.0 g, wet) in MeOH (100 mL) was stirred under Hz (1 atm) at 25°C for 12 hours. The resulting mixture was filtered and the filter cake was washed with MeOH (3 × 20 mL). The filtrate was directly concentrated to give 1-8C, (S)-methyl 4-amino-3-((oxetan-2-ylmethyl)amino)benzoate (5.2 g, 94.5% yield).

LCMS: r.t. = 1.201 min, [M+1]⁺= 237.1, purity: 89.7%.

### (8) Preparation of Compound Int-2

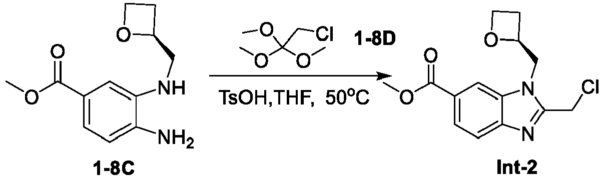

2-chloro-1,1,1-trimethoxyethane 1-8D (0.98 g, 6.35 mmol, 1.5 equiv.) and T_{S}OH·H₂O (0.08 g, 0.423 mmol, 0.1 equiv.) were added to a solution of (S)-methyl 4-amino-3-((oxetan-2-ylmethyl)amino)benzoate 1-8C (1.0 g, 4.23 mmol, 1 equiv.) in THF (20 mL). The mixture was stirred at 50°C for 8 hours. The mixture was diluted with a saturated sodium bicarbonate solution NaHCO₃ (20 mL), and extracted with EtOAc (10 mL × 3). The combined organic layers were dried with Na₂SO₄, filtered and concentrated to obtain a residue, which was then purified by silica gel column chromatography and eluted with EtOAc/PE(0-80%) to give Int-2, (S)-methyl 2-(chloromethyl)-1-(oxetan-2-ylmethyl)-1H-benzo[d]imidazole-6-carboxylate (1.1 g, 88% yield).

¹H NMR(400 MHz, CDCl3) δ 8.12 (d, *J* = 0.9 Hz, 1H), 8.01 (dd, *J* = 8.5, 1.5 Hz, 1H), 7.79 (d, *J* = 8.5 Hz, 1H), 5.21 (ddd, *J* = 9.6, 7.3, 2.7 Hz, 1H), 5.03 (s, 2H), 4.69 - 4.45 (m, 3H), 4.34 (d, *J* = 9.2 Hz, 1H), 3.96 (s, 3H), 2.76 (dtd, *J* = 11.5, 8.1, 6.0 Hz, 1H), 2.42 (ddt, *J* = 11.5, 9.2, 7.3 Hz, 1H).

### Intermediate Int-3, tert-butyl 4-(6-hydroxypyridin-2-yl)piperidine-1-carboxylate was prepared as follows.

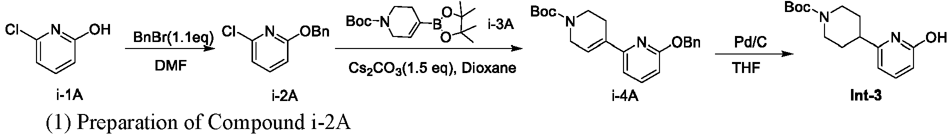

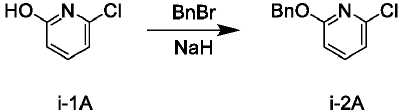

NaH was added to a mixture of 6-chloropyridine-2-ol i-1A (30.00 g, 231.58 mmol) in DMF (200 mL) at 0 °C. The mixture was stirred under Ar₂ at 0 °C for 0.5 hours. BnBr (43.57 g, 254.74 mmol) was then added to the above solution, and stirred under Ar₂ at room temperature for 1 hour. When the reaction was completed as determined by TLC, the reaction mixture was diluted with water (200 mL) and extracted with ethyl acetate (100 mL × 3). The combined organic layers were washed with saturated NaCl, dried with anhydrous Na₂SO₄ and filtered through a filter. The filtrate was concentrated to obtain a residue, which was purified on silica gel by column chromatography and eluted with PE/EA (0-20%) to give i-2A, 2-(benzyloxy)-6-chloropyridine (25 g, 47.18%).

¹H NMR (400 MHz, CDCl₃) δ 7.54 - 7.44 (m, 3H), 7.40 - 7.30 (m, 3H), 6.91 (dd, *J* = 7.5, 0.6 Hz, 1H), 6.70 (dd, *J* = 8.2, 0.6 Hz, 1H), 5.36 (s, 2H).

### (2) Preparation of Compound i-4A

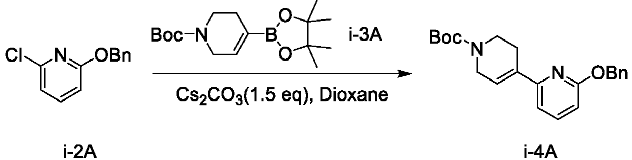

Pd(dppf)Cl₂ (8.25 g, 11.38 mmol) was added to a mixture of tert-butyl 4-(4,4,5,5-tetramethyl-1,3,2-dioxaborolan-2-yl)-3,6-dihydropyridine-1(2H)-carboxylate i-3A (35.19 g, 113.81 mmol), 6-chloropyridin-2-ol i-2A (25.00 g, 113.81 mmol) and Cs₂CO₃ (55.62 g, 170.71 mmol) in dioxane (200 mL). The reaction mixture was stirred under N₂ atmosphere at 100°C for 16 hours. When the reaction was completed as determined by LCMS, the mixture was concentrated to obtain a residue, which was purified by silica gel column chromatography and eluted with EA/PE (0-10%) to give i-4A, tert-butyl 6-(benzyloxy) -3',6'-dihydro-[2,4'-bipyridine]-1'(2'H)-carboxylate (47.00 g, 59.9%).

LCMS: r. t. = 2.368 min, [M+1]⁺ = 367, purity: 75.78 %.

### (3) Preparation of Compound Int-3

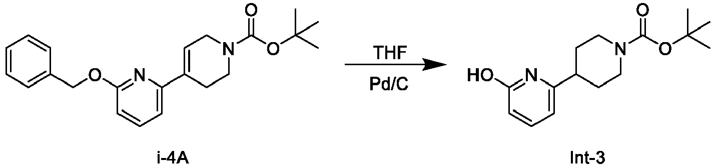

Pd/C (4 g, wet) was added to a mixture of tert-butyl 6-(benzyloxy)-3',6'-dihydro-[2,4'-bipyridine]-1'(2'H)-carboxylate I-4A (23 g, 62.76 mmol) in THF (200 mL). The mixture was stirred overnight under H₂ at room temperature. The mixture was filtered through diatomite and the filter cake was washed with EA (50 mL×3). The combined filtrate was concentrated to obtain a residue, which was purified by silica gel column chromatography and eluted with MeOH/DCM (0-5%) to give Int-3, tert-butyl 4-(6-hydroxypyridin-2-yl)piperidine-1-carboxylate (9.2 g,53%).

LCMS: r. t. = 0.94 min, [M-55]⁺ = 223, purity: 60%.

¹H NMR (400 MHz, CDCl₃) δ 11.32 (s, 1H), 7.38 (dd, *J* = 9.1, 6.9 Hz, 1H), 6.42 (d, *J* = 8.5 Hz, 1H), 6.03 (d, *J* = 6.8 Hz, 1H), 4.25 (s, 2H), 2.83 (s, 2H), 2.61 (dd, *J* = 13.8, 10.5 Hz, 1H), 1.92 (d, *J* = 12.0 Hz, 2H), 1.48 (s, 9H), 1.25 (s, 2H).

### Intermediate Int-5, 4-(((6-(1-(tert-butoxycarbonyl)piperidin-4-yl)pyridin-2-yl)oxy)methyl)-3-fluorobenzoic acid was prepared as follows:

### (1) Preparation of Compound i -2

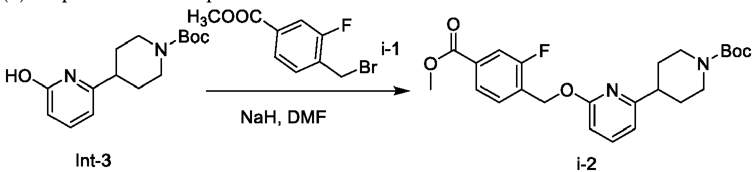

NaH (0.26 g, 6.5 mmol, 1.3 equiv.) was added to a solution of tert-butyl 4-(6-hydroxypyridin-2-yl)piperidine-1-carboxylate Int-3 (1.4 g, 5.0 mmol, 1 equiv.) in DMF (20 mL) at 0 °C. The mixture was stirred at 0 °C for 30 minutes. Then methyl 4-(bromomethyl)-3-fluorobenzoate i-1 (1.6 g, 6.5 mmol, 1.3 equiv.) was added to the above solution, and stirred at 25 °C for 2 hours. TLC showed complete consumption of the starting materials and new spots were found. The mixture was diluted with H₂O (10 mL) and extracted with EtOAc (3×10 mL). The combined organic layers were washed with brine (100 mL), dried with Na₂SO₄ and concentrated in vacuum to obtain a residue, which was then purified by silica gel column chromatography and eluted with PE/EtOAc (0-20%) to give i-2, tert-butyl 4-(6-((2-fluoro-4-(methoxycarbonyl)benzyl)oxy)pyridin-2-yl)piperidine-1-carboxylate (1.1 g, 50%).

¹H NMR (400 MHz, CDCl3) δ = 7.81 (dd, *J* = 8.0, 1.5 Hz, 1H), 7.73 (dd, *J* = 10.4, 1.5 Hz, 1H), 7.62 - 7.44 (m, 2H), 6.73 (d, *J* = 7.3 Hz, 1H), 6.65 (d, *J* = 8.1 Hz, 1H), 5.49 (s, 2H), 4.20 (s, 1H), 3.92 (s, 3H), 2.77 (d, *J=* 43.3 Hz, 3H), 2.05 (s, 1H), 1.85 (d, *J* = 12.6 Hz, 2H), 1.49 (s, 9H).

### (2) Preparation of Compound Int-5

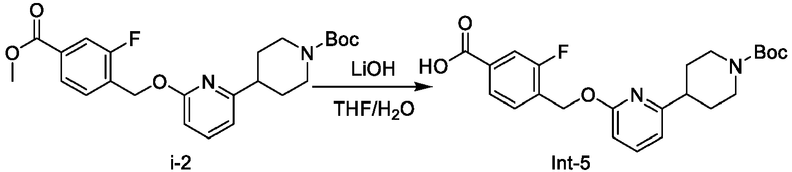

LiOH (0.378 g, 15.7 mmol, 5.0 equiv.) in H₂O (10 mL) was added to a solution of tert-butyl 4-(6-((2-fluoro-4-(methoxycarbonyl)benzyl)oxy)pyridin-2-yl)piperidine-1-carboxylate (i-2, 1.4 g, 3.15 mmol, 1 equiv.) in THF (10 mL) at 25 °C. The mixture was stirred at room temperature for 2 hours. The mixture was adjusted to a pH of 7 with HCl (1 N). The mixture was diluted with H₂O (100 mL) and extracted with EtOAc (3×50 mL). The combined organic layers were washed with brine (100 mL), dried with Na₂SO₄ and concentrated in vacuum to give Int-5, 4-(((6-(1-(tert-butoxycarbonyl)piperidin-4-yl) pyridin-2-yl)oxy)methyl)-3-fluorobenzoic acid (0.47 g, 34.7% yield).

LCMS: r. t. = 2.177 min, [M+1]⁺ = 431.2, purity: 69.7%.

### Intermediate Int-7, tert-butyl 4-(6-((2-fluoro-4-(methoxy(methyl)carbamoyl)benzyl)oxy)pyridin-2-yl)piperidine-1-carboxylate was prepared as follows.

### (1) Preparation of Compound Int-7

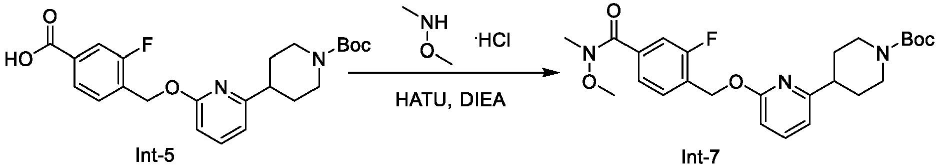

A solution of 4-(((6-(1-(tert-butoxycarbonyl)piperidin-4-yl)pyridin-2-yl)oxy)methyl)-3-fluorobenzoic acid Int-5 (0.470 g, 1.09 mmol, 1.0 equiv.), N,O-dimethylhydroxylamine hydrochloride (0.214 g, 2.18 mmol, 2.0 equiv.), DIEA (0.564 g, 4.37 mmol, 4.0 equiv.) and HATU (0.623 g, 1.64 mmol, 1.5 equiv.) in DMF (6 mL) was stirred at 25°C for 2 hours. The mixture was diluted with EtOAc (50 mL), washed with H₂O (80 mL), and extracted with EtOAc (30 mL × 3). The combined organic layers were washed with brine (100 mL), dried with Na₂SO₄ and concentrated in vacuum to obtain a residue, which was then purified by silica gel column chromatography and eluted with PE/EtOAc (3: 1) to give Int-7, tert-butyl 4-(6-((2-fluoro-4-(methoxy(methyl)carbamoyl)benzyl)oxy)pyridin-2-yl)piperidine-1-carboxylate (0.440 g, 85.4% yield).

LCMS: r. t. = 2.262 min, [M+1]⁺ = 474.3, purity: 98%.

### Intermediate Int-2A, (S)-methyl 2-(chloromethyl)-3-(oxetan-2-ylmethyl)-3H-imidazo[4,5-b]pyridine-5-carboxylate was prepared as follows.

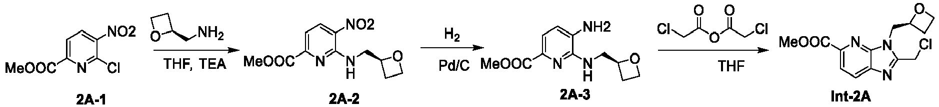

(S)-oxetan-2-ylmethylamine (900 mg, 10.34 mmol) and TEA (2.0 g, 20 mmol) were added to a solution of methyl 6-chloro-5-nitropicolinate (2A-1, 2.2 g, 10.34 mmol) in THF (20 mL). The reaction mixture was stirred under nitrogen at 40 °C for 16 hours. The reaction mixture was concentrated to obtain a crude product, which was further purified by column chromatography (MeOH/DCM = 0-3%) to give 2A-2, (S)-methyl 5-nitro-6-((oxetan-2-ylmethyl)amino)picolinate (1.2 g, 45%). Ethyl acetate and Pd/C (500 mg) were added to a solution of 2A-2 (1.2 g, 4.49 mmol) in MeOH (5 mL), and stirred under H₂ atmosphere at room temperature for 16 hours. The reaction mixture was further purified via filtration and concentrated to give a crude product of 2A-3, (S)-methyl 5-amino-6-((oxetan-2-ylmethyl)amino)picolinate (840 mg, 78.9%). 2-chloroacetic anhydride (667 mg, 3.9 mmol) was added to a solution of 2A-3 (840 mg, 3.54 mmol) in THF (10 mL), and stirred at 50 °C for 16 hours, and the reaction mixture was then quenched with saturated aqueous NaHCO₃ solution, extracted with EA (40 mL × 3), washed with brine, and concentrated to obtain a crude product, which was further purified by column chromatography (PE/EA = 10-51%) to give Int-2A, (S)-methyl 2-(chloromethyl)-3 -(oxetan-2-ylmethyl)-3H-imidazo [4,5 -b]pyridine-5 -carboxylate (422 mg, 43%).

LCMS: r. t. = 1.694 min, [M+H]⁺ = 296, purity: 98%.

### Example 1

### (S)-2-((4-(6-((5-acetylthiophen-2-yl)methoxy)pyridin-2-yl)piperidin-1-yl)methyl)-1-(oxetan-2-yl methvl)-1H-benzodimidazole-6-carboxlic acid (Compound 1)

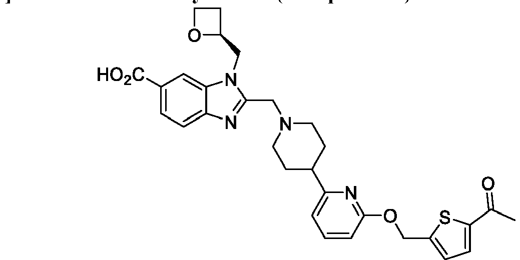

### (1) Preparation of Compound 1-2

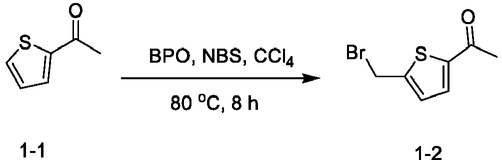

NBS (279 mg, 1.57 mmol) was added to a mixture of compound 1-1 (200 mg, 1.43 mmol) and BPO (7 mg, 0.03 mmol) in CCl₄ (5 mL), and then the reaction solution was stirred at 80°C for 8 h. The reaction mixture was then quenched with a saturated solution of Na₂S₂O₃ (5 mL). The solution was extracted with water (20 mL) and DCM (20 mL × 3). The combined organic phases were washed with brine (20 mL), dried (Na₂SO₄), filtered and concentrated. 1-(5-(bromomethyl)thiophen-2-yl)ethan-1-one was yielded as compound 1-2 after processed by flash chromatography (SiO₂, 20% EtOAc-hexane) (260 mg, 83%).

¹H NMR (400 MHz, CDCl₃) δ 7.53 (d, *J* = 3.8 Hz, 1H), 7.12 (d, *J* = 3.8 Hz, 1H), 4.67 (s, 2H), 2.54 (s, 3H).

### (2) Preparation of Compound 1-3

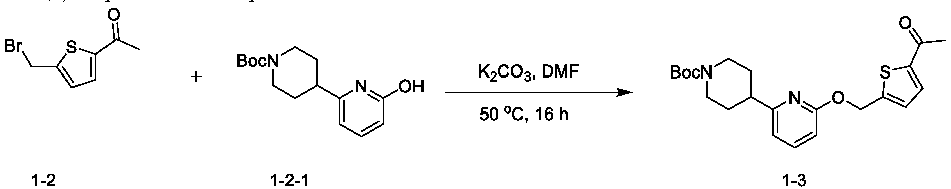

A mixture of compound 1-2 (2 g, 9.13 mmol), compound 1-2-1 (3.05 g, 10.95 mmol) and K₂CO₃ (2.52 g, 18.26 mmol) in DMF (30 mL) was stirred at 50°C for 16 h. H₂O (100 mL) was added. The reaction solution was extracted with EtOAc (3 × 100 mL). The combined organic phases were washed with brine (10 mL), dried (Na₂SO₄), filtered, and concentrated. After processed by flash chromatography (SiO₂, 25% EtOAc-hexane), tert-butyl 4-(6-((5-acetylthiophen-2-yl)methoxy)pyridin-2-yl)piperidine-1-carboxylate was yielded as compound 1-3 (2 g, 53%).

¹H NMR (400 MHz, CDCl₃) δ 7.58 (d, *J* = 3.8 Hz, 1H), 7.52 (dd, *J* = 8.1, 7.5 Hz, 1H), 7.12 (d, *J* = 3.8 Hz, 1H), 6.75 (d, *J* = 7.3 Hz, 1H), 6.62 (d, *J* = 8.1 Hz, 1H), 5.56 (s, 2H), 4.22 (s, 2H), 2.92 - 2.69 (m, 3H), 2.53 (s, 3H), 1.90 (d, *J=* 13.5 Hz, 2H), 1.74 (qd, *J* = 12.6, 3.8 Hz, 2H), 1.48 (s, 9H).

### (3) Preparation of Compound 1-4

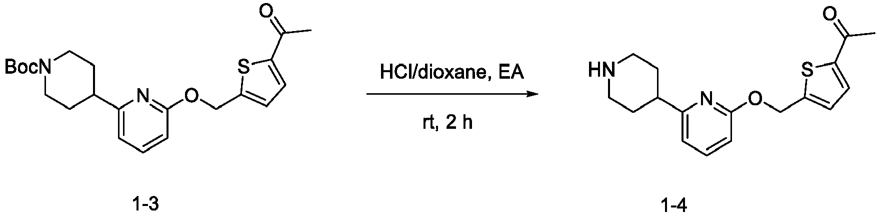

HCl/dioxane (2 mL) was added to a solution of compound 1-3 (45 mg, 0.11 mmol) in EtOAc (2 mL). The mixture was stirred at room temperature for 1 hour, and concentrated to give a solid. The solid was dissolved in H₂O (5 mL), adjusted to pH > 7 with NaHCO₃ (eq), and extracted with EtOAc (10 mL × 3). The combined organic phases were washed with brine (10 mL), dried (Na₂SO₄), filtered, and concentrated to give 1-(5-(((6-(piperidin-4-yl)pyridin-2-yl)oxy)methyl)thiophen-2-yl)ethane-1-one as compound 1-4 (20 mg, 58.8%).

¹H NMR (400 MHz, DMSO-J6) δ 7.82 (d, *J* = 3.8 Hz, 1H), 7.68 - 7.61 (m, 1H), 7.29 (d, *J* = 3.8 Hz, 1H), 6.88 (d, *J* = 7.0 Hz, 1H), 6.66 (d, *J* = 8.2 Hz, 1H), 5.58 (s, 2H), 3.01 (d, *J* = 9.4 Hz, 1H), 2.80 - 2.52 (m, 4H), 2.51 (s, 3H), 1.77 (d, *J* = 11.6 Hz, 2H), 1.69 - 1.52 (m, 2H), 1.24 (s, 1H).

LCMS (ESI) m/z: 317.0 [M+H]⁺.

### (4) Preparation of Compound 1-5

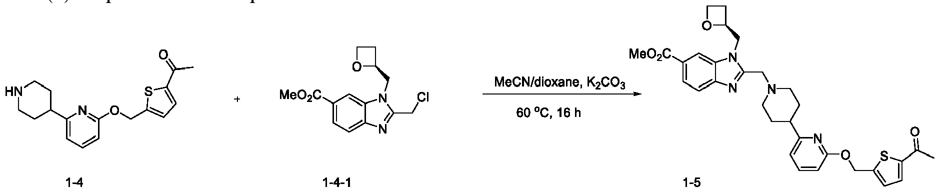

K₂CO₃ (175 mg, 1.26 mmol) was added to a mixture of compound 1-4 (100 mg, 0.32 mmol) and compound 1-4-1 (98.2 mg, 0.32 mmol) in dioxane (2 mL) and CH₃CN (1 mL). The mixture was stirred at 60°C for 16 hours. Water (10 mL) was added. The solution was extracted with DCM (10 mL × 3). The combined organic phases were washed with brine (30 mL), dried (Na₂SO₄), filtered, and concentrated. After processed by flash chromatography (silica gel, eluted with 0-10% MeOH/DCM), (S)-methyl 2-((4-(6-((5-acetylthiophen-2-yl)methoxy)pyridin-2-yl)piperidin-1-yl)methyl)-1-(oxetan-2-ylmethyl)-1H-benzo [d] imidazole-6-carboxylate was yielded as compound 1-5 (50 mg, 27.6%).

¹H NMR (400 MHz, CDCl3) δ 8.17 (s, 1H), 7.97 (d, *J* = 8.7 Hz, 1H), 7.75 (d, *J* = 8.5 Hz, 1H), 7.56 (d, *J* = 3.8 Hz, 1H), 7.51 (t, *J* = 7.8 Hz, 1H), 7.12 (d, *J* = 3.7 Hz, 1H), 6.75 (d, *J* = 7.3 Hz, 1H), 6.60 (d, *J* = 8.2 Hz, 1H), 5.56 (s, 2H), 5.27 - 5.19 (m, 1H), 4.74 (qd, *J* = 15.5, 4.2 Hz, 2H), 4.61 (dd, *J* = 14.0, 7.8 Hz, 1H), 4.40 (dt, *J* = 9.2, 6.0 Hz, 1H), 3.96 (d, *J =* 11.2 Hz, 5H), 2.97 (d, *J =* 15.4 Hz, 2H), 2.88 (s, 1H), 2.79 - 2.60 (m, 2H), 2.52 (s, 3H), 2.30 (dd, *J* = 25.1, 12.1 Hz, 2H), 1.98 - 1.79 (m, 4H).

LC-MS (ESI) m/z: 576.0 [M+H]⁺.

### (5) Preparation of Compound 1

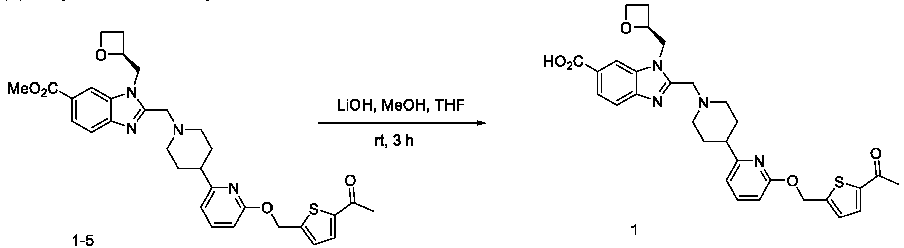

LiOH (2 mL) was added to a solution of compound 1-5 (50 mg, 0.08 mmol) in THF (0.5 mL) and MeOH (0.5 mL). The mixture was stirred at room temperature for 3 hours, and then the solvent was removed under reduced pressure to give a crude product which was purified by HPLC (gradient: 10% MeCN/90% H₂O, 0.1% NH₃·H₂O to 100% MeCN) to give (S)-2-((4-(6-((5-acetylthiophen-2-yl)methoxy)pyridin-2-yl) piperidin-1-yl)methyl)-1-(oxetan-2-ylmethyl)-1H-benzo[d]imidazole-6-carboxylic acid as compound 1 (14.93 mg, 29.2%).

¹H NMR (400 MHz, DMSO-d6) δ 8.04 (s, 1H), 7.81 (d,*J* = 3.8 Hz, 1H), 7.75 (d, *J* = 8.1 Hz, 1H), 7.67 - 7.60 (m, 1H), 7.41 (s, 1H), 7.29 (d, *J* = 3.8 Hz, 1H), 6.91 (d, *J* = 7.2 Hz, 1H), 6.67 (d, *J* = 8.4 Hz, 1H), 5.58 (s, 2H), 5.09 (s, 1H), 4.69 (s, 1H), 4.59 (d, *J* = 13.7 Hz, 1H), 4.44 (s, 1H), 4.37 (d, *J* = 9.1 Hz, 1H), 3.90 (d, *J* = 13.5 Hz, 1H), 3.75 (d, *J* = 13.2 Hz, 1H), 2.99 (s, 1H), 2.87 (s, 1H), 2.67 (s, 2H), 2.48 (s, 3H), 2.34 - 2.12 (m, 3H), 1.82 (d, *J* = 12.1 Hz, 4H).

LC-MS (ESI) m/z: 561.3 [M+H]⁺.

### Example 2

### (S)-2-((4-(6-((4-acetylthiophen-2-yl)methoxy)pyridin-2-yl)piperidin-1-yl)methyl)-1-(oxetan-2-ylm ethyl)-1H-benzo[d]imidazole-6-carboxylic acid (Compound 2)

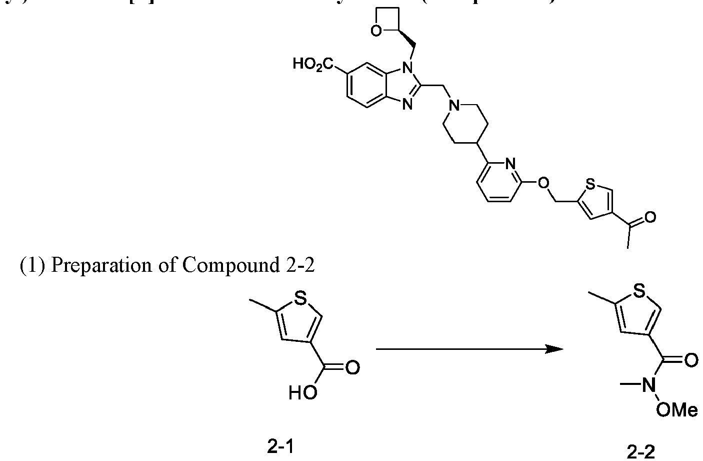

To a solution of compound 2-1 (1 g, 3.52 mmol) and N,O-dimethylhydroxylamine (750 mg, 3.87 mmol) in anhydrous DMF (20 mL) was added HATU (4 g, 5.28 mmol), DIEA (4.54 g, 17.6 mmol). The reaction mixture was stirred at room temperature for 16 hours. The reaction mixture was partitioned between water (50 mL) and EtOAc (50 mL × 3). The combined organic phases were washed with brine (50 mL), dried (Na₂SO₄), filtered, and concentrated. After processed by flash chromatography (SiO₂, 0-100% EtOAc-hexane), N-methoxy-N,5-dimethylthiophene-3-carboxamide was given as compound 2-2 (1.1 g, 84.6%).

¹H NMR (400 MHz, DMSO-d₆) δ 7.90 (1 H, d, *J* =1.4), 7.20 - 7.10 (1 H, m), 3.64 (3 H, s), 3.23 (3 H, s), 2.45 (3 H, d, *J* =1.1).

LCMS: (ESI) m/z: 186.0 [M+H]⁺.

### (2) Preparation of Compound 2-3

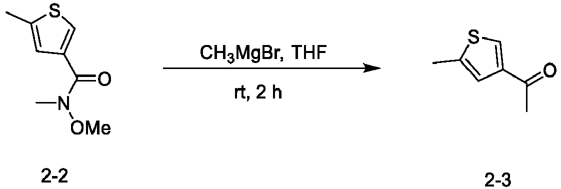

To a solution of CH₃MgBr (1.8 mL, 5.4 mmol) in THF was added dropwise a solution of compound 2-2 (500 mg, 2.7 mmol) in anhydrous THF (3 mL) at 0°C under nitrogen. After quenching the reaction mixture with NH₄Cl (eq), the reaction mixture was stirred at room temperature for 2 hours. The solution was extracted with water (10 mL) and EtOAc (10 mL × 3). The organic phase was washed with brine, and dried (Na₂SO₄). After filtration, the solvent was concentrated under reduced pressure to give 1-(5-methylthiophen-3-yl) ethan-1-one as compound 2-3 (310 mg, 82.0%).

¹H NMR (400 MHz, DMSO-d6) δ 8.24 (1 H, d, *J* =1.4), 7.17 (1 H, s), 2.45 (3 H, d, *J* =0.9), 2.44 (3 H, s).

LCMS: (ESI) m/z: 141.0 [M+H]⁺.

### (3) Preparation of Compound 2-4

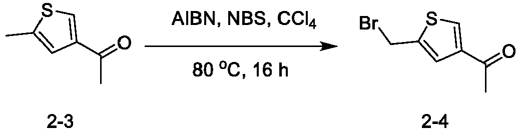

A mixture of compound 2-3 (310 mg, 2.21 mmol), AIBN (7 mg, 0.044 mmol) and NBS (434 mg, 2.43 mmol) in CCl₄ (6 mL) was stirred at 80°C for 16 hours. The reaction mixture was then quenched with Na₂S₂O₃ (eq). The solution was extracted with water (20 mL) and DCM (20 mL × 3). The combined organic phases were washed with brine (20 mL), dried (Na₂SO₄), filtered, and concentrated. After processed by flash chromatography (SiO₂, 0-100% EtOAc-hexane), 1-(5-(bromomethyl)thiophen-3-yl)ethan-1-one was given as compound 2-4 (280 mg, 57.9%).

¹H NMR (400 MHz, DMSO-J6) δ 8.44 (1 H, s), 7.50 (1 H, s), 4.95 (2 H, s), 2.41 (3 H, s).

LC-MS: (ESI) m/z: 219.0 [M+H]⁺.

### (4) Preparation of Compound 2-5

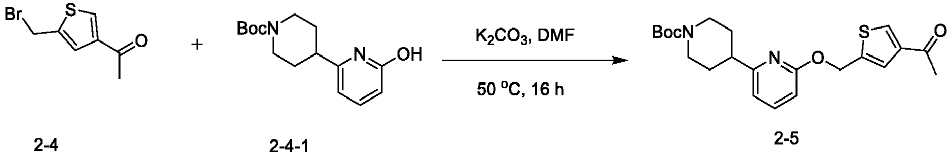

A mixture of compound 2-4 (280 mg, 1.28 mmol), compound 2-4-1 (427 mg, 1.53 mmol) and K₂CO₃ (353 mg, 2.56 mmol) in DMF (5 mL) was stirred at 50°C for 16 hours. The reaction mixture was partitioned between water (10 mL) and EtOAc (10 mL × 3). The combined organic phases were washed with brine (10 mL), dried (Na₂SO₄), filtered, and concentrated. After processed by flash chromatography (SiOz, 0-100% EtOAc-hexane), tert-butyl 4-(6-((4-acetylthiophen-2-yl)methoxy)pyridin-2-yl)piperidine-1-carboxylate was given as compound 2-5 (220 mg, 41.3%).

¹H NMR (400 MHz, DMSO) δ 8.42 (1 H, d, *J* =1.5), 7.65 (1 H, dd, *J* =8.1, 7.4), 7.56 (1 H, d, *J* =1.3), 6.90 (1 H, d, *J* =7.2), 6.66 (1 H, d, *J=* 8.0), 5.53 (2 H, s), 4.06 - 4.02 (2 H, m), 2.94 - 2.74 (3 H, m), 2.46 (3 H, s), 1.85 (2 H, d, *J* =12.8), 1.71 - 1.59 (2 H, m), 1.41 (9 H, s).

LCMS: (ESI) m/z: 417.0 [M+H]⁺.

### (5) Preparation of Compound 2-6

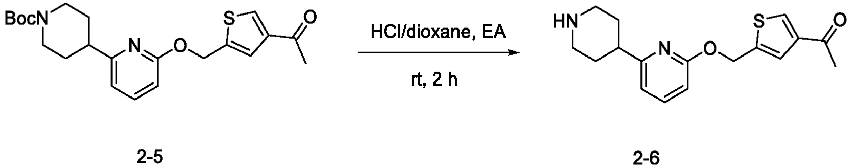

A solution of compound 2-5 (220 mg, 0.53 mmol) and HCl/dioxane (1 mL) in EtOAc (1.5 mL) was stirred at room temperature for 1 hour, then the mixture was concentrated to give a solid which was dissolved in water (5 mL). The solution was adjusted to pH > 7 with NaHCO₃ (eq), and extracted with EtOAc (5 mL × 3). The combined organic phases were washed with brine (10 mL), dried (Na₂SO₄), filtered, and concentrated to give 1-(5-(((6-(piperidin-4-yl) pyridin-2-yl)oxy)methyl)thiophen-3-yl)ethane-1-one as compound 2-6 (120 mg, 71.8%).

¹H NMR (400 MHz, DMSO-J6) δ 8.42 (1 H, d, *J* = 1.4), 7.67 - 7.61 (1 H, m), 7.57 (1 H, d, *J* =1.2), 7.21 (1 H, dd, *J*= 27.2, 7.3), 6.86 (1 H, d, *J* =7.3), 6.63 (1 H, d, *J =* 8.1), 5.54 (2 H, s), 3.02 (2 H, d, *J =* 12.0), 2.68 (1 H, ddd, J=11.9, 8.3, 3.7), 2.62 - 2.54 (2 H, m), 2.46 (3 H, s), 1.78 (2 H, d, *J =* 12.0), 1.64 (2 H, dd, *J* = 12.3, 3.8).

LCMS: (ESI) m/z: 316.0 [M+H]⁺.

### (6) Preparation of Compound 2-7

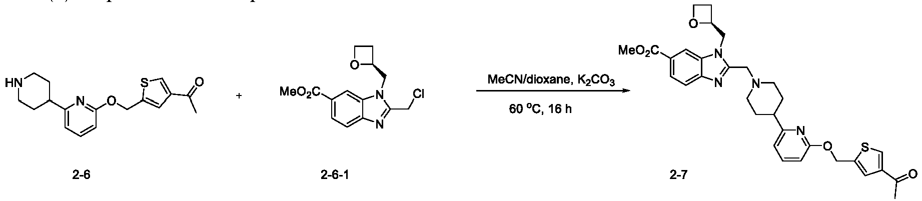

A mixture of compound 2-6 (120 mg, 0.379 mmol), compound 2-6-1 (123 mg, 0.417 mmol) and K₂CO₃ (209 mg, 1.516 mmol) in dioxane (12 mL) and CH₃CN (6 mL) was stirred at 60°C for 16 hours. The solution was extracted with water (30 mL) and DCM (30 mL × 3). The combined organic phases were washed with brine (30 mL), dried (Na₂SO₄), filtered, and concentrated. After processed by flash chromatography (SiO₂, 0-100% EtOAc-hexane), (S)-2-((4-(6-((4-acetylthiophen-2-yl)methoxy)pyridin-2-yl)piperidin-1-yl) methyl)-1-(oxetan-2-ylmethyl)-1H-benzo[d]imidazole-6-carboxylate was given as compound 2-7 (140 mg, 64.2%).

¹H NMR (400 MHz, DMSO-d6) δ 8.41 (1 H, d, *J* = 1.5), 8.29 (1 H, s), 7.82 (1 H, dd, *J* = 8.4, 1.5), 7.73 - 7.60 (2 H, m), 7.56 (1 H, d, *J* = 1.4), 6.90 (1 H, d, *J* =7.2), 6.64 (1 H, d, *J* =8.2), 5.54 (2 H, d, *J* =1.6), 5.11 (1 H, d, *J* =7.1), 4.82 (1 H, dd, *J* =15.2, 7.2), 4.67 (1 H, dd, *J* =15.1, 2.6), 4.49 - 4.32 (2 H, m), 4.01 (1 H, dd, *J* =13.2, 6.1), 3.87 (3 H, s), 3.79 (1 H, d, *J* =13.3), 3.03 (1 H, d, *J* =11.5), 2.88 (1 H, d, *J* =10.7), 2.74 - 2.61 (2 H, m), 2.44 (4 H, s), 2.23 (2 H, d, *J* =29.5), 1.84 (4 H, d, *J* =17.5).

LC-MS: (ESI) m/z: 575.4 [M+H]⁺.

### (7) Preparation of Compound 2

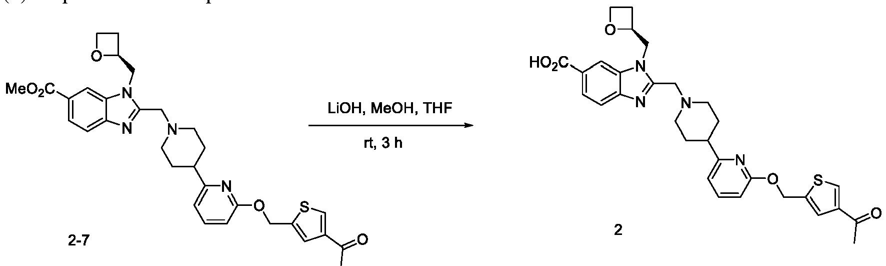

A solution of compound 2-7 (100 mg, 0.17 mmol), LiOH (2 mL) in THF (0.5 mL) and MeOH (0.5 mL) were stirred at room temperature for 3 hours. The solvent was then removed under reduced pressure to give a crude product which was purified by HPLC (gradient: 10% MeCN/90% HzO, 0.1% NH₃·H₂O to 100% MeCN) to give (S)-2-((4-(6-((4-acetylthiophen-2-yl)methoxy)pyridin-2-yl)piperidin-1-yl) methyl)-1-(oxetan-2-ylmethyl)-1H-benzo[d]imidazole-6-carboxylic acid as compound 2 (10.67 mg, 10.9%).

¹H NMR (400 MHz, DMSO-*d6*) δ 8.41 (1 H, d, *J* =1.4), 8.25 (1 H, s), 7.80 (1 H, dd, *J* =8.4, 1.5), 7.63 (2 H, dd, *J* =12.1, 5.7), 7.56 (1 H, d, J=1.3), 6.90 (1 H, d, *J* =7.2), 6.64 (1 H, d, *J* =8.1), 5.61 - 5.49 (2 H, m), 5.11 (1 H, dt, *J* =7.0, 4.4), 4.79 (1 H, dd, *J* =15.2, 7.2), 4.65 (1 H, dd, *J* =15.2, 2.7), 4.39 (2 H, ddt, *J* =11.8, 8.9, 5.9), 3.96 (1 H, d, *J* =13.5), 3.78 (1 H, d, *J* =13.5), 3.03 (1 H, d, *J* =10.5), 2.88 (1 H, d, *J* =11.5), 2.74 - 2.61 (2 H, m), 2.44 (4 H, s), 2.31 - 2.15 (2 H, m), 1.92 -1.73 (4 H, m).

LC-MS: MC21-01-042R1, (ESI) m/z: 561.1 [M+H]⁺.

### Example 3

### (S)-2-((4-(6-((5-acetylthiophen-3-yl)methoxy)pyridin-2-yl)piperidin-1-yl)methyl)-1-(oxetan-2-ylm ethyl)-1H-benzo[d]imidazole-6-carboxylic acid (Compound 3)

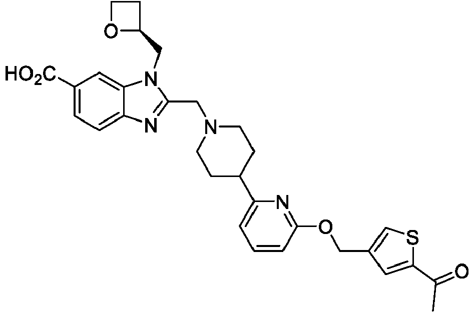

### (1) Preparation of Compound 3-2

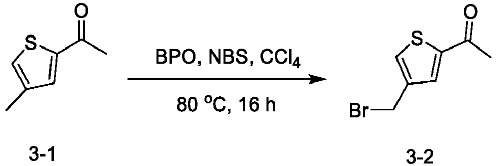

NBS (139 mg, 0.78 mmol) was added to a mixture of compound 3-1 (100 mg, 0.71 mmol) and BPO (3.5 mg, 0.01 mmol) in CCl₄ (2 mL). Then the reaction solution was stirred at 80°C for 16 hours. The reaction mixture was then quenched with a saturated solution of Na₂S₂O₃ (5 mL). The solution was extracted with water (20 mL) and DCM (20 mL × 3). The combined organic phases were washed with brine (20 mL), dried (Na₂SO₄), filtered, and concentrated. After processed by flash chromatography (SiO₂, 20% EtOAc-hexane), 1-(4-(bromomethyl)thiophen-2-yl)ethan-1-one was given as compound 3-2 (40 mg, 25.8%).

¹H NMR (400 MHz, CDCl3) δ 7.69 (d, *J* = 1.3 Hz, 1H), 7.57 (d, *J* = 0.6 Hz, 1H), 4.48 (s, 2H), 2.56 (s, 3H).

### (2) Prearation of Compound 3-3

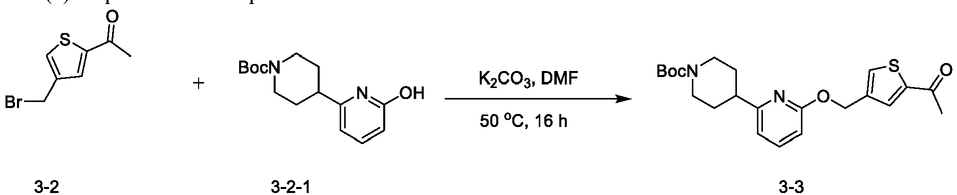

A mixture of compound 3-2 (40 mg, 0.18 mmol), compound 3-2-1 (61 mg, 0.22 mmol) and K₂CO₃ (50.5 mg, 0.37 mmol) in DMF (2 mL) was stirred at 50°C for 16 hours. Water (5 mL) was added, and the reaction solution was extracted with EtOAc (3 × 10 mL). The combined organic phases were washed with brine (10 mL), dried (Na₂SO₄), filtered, and concentrated. After processed by flash chromatography (SiO₂, 25% EtOAc-hexane), tert-butyl 4-(6-((5-acetylthiophen-3-yl)methoxy)pyridin-2-yl)piperidine-1-carboxylate was given as compound 3-3 (22 mg, 29%).

¹H NMR (400 MHz, CDCl₃) δ 7.76 (s, 1H), 7.62 (s, 1H), 7.52 (t, *J* = 7.7 Hz, 1H), 6.74 (d, *J* = 7.3 Hz, 1H), 6.61 (d, *J* = 8.2 Hz, 1H), 5.36 (s, 2H), 4.24 (s, 2H), 2.94 - 2.67 (m, 3H), 2.56 (d, *J* = 2.7 Hz, 4H), 1.89 (d, *J* = 15.0 Hz, 2H), 1.76 (t, *J* = 11.4 Hz, 2H), 1.48 (s, 9H).

### (3) Preparation of Compound 3-4

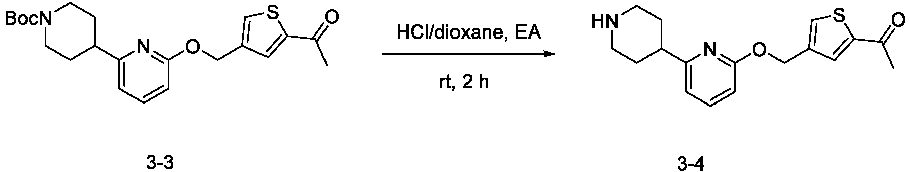

HCl/dioxane (5 mL) was added to a solution of compound 3-3 (230 mg, 0.55 mmol) in EtOAc (5 mL). The mixture was stirred at room temperature for 1 hour, and then concentrated to give a solid. The solid was dissolved in water (5 mL), adjusted to pH > 7 with NaHCO₃ (eq), and extracted with EtOAc (10 mL × 3). The combined organic phases were washed with brine (10 mL), dried (Na₂SO₄), filtered, and concentrated to give 1-(4-(((6- (piperidin-4-yl)pyridin-2-yl)oxy)methyl)thiophen-2-yl)ethane-1-one as compound 3-4 (140 mg, 80%).

¹H NMR (400 MHz, CDCl₃) δ 7.78 (d, *J=* 1.3 Hz, 1H), 7.71 - 7.64 (m, 1H), 7.55 - 7.49 (m, 1H), 6.75 (d, *J* = 7.2 Hz, 1H), 6.62 (d, *J* = 8.2 Hz, 1H), 5.38 (s, 2H), 3.32 (d, *J* = 12.4 Hz, 2H), 2.88 - 2.70 (m, 3H), 2.56 (d, *J* = 2.3 Hz, 3H), 2.02 - 1.83 (m, 4H), 1.25 (s, 1H).

LCMS: (ESI) m/z: 317.0 [M+H]⁺.

### (4) Preparation of Compound 3-5

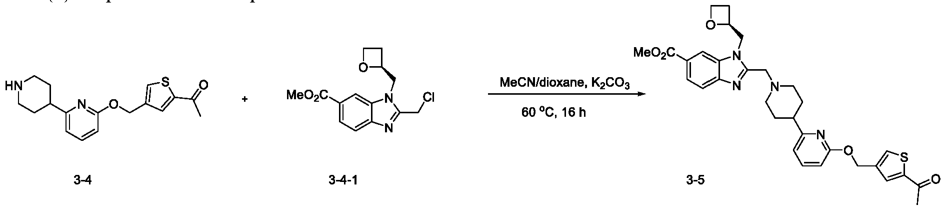

K₂CO₃ (175 mg, 1.26 mmol) was added to a mixture of compound 3-4 (100 mg, 0.32 mmol) and compound 3-4-1 (93 mg, 0.32 mmol) in dioxane (6 mL) and CH₃CN (3 mL). The mixture was stirred at 60°C for 16 hours. Water (10 mL) was added, and the solution was extracted with DCM (10 mL × 3). The combined organic phases were washed with brine (20 mL), dried (Na₂SO₄), filtered, and concentrated. After processed by flash chromatography (silica gel, eluted with 0-10% MeOH/DCM) to give (S)-methyl 2-((4-(6-((5-acetylthiophen-3-yl)methoxy)pyridin-2-yl)piperidin-1-yl)methyl)-1-(oxetan-2-ylmethyl)-1H-b enzo[d]imidazole-6-carboxylate as compound 3-5 (100 mg, 55.1%).

¹H NMR (400 MHz, CDCl₃) δ 8.16 (s, 1H), 7.97 (d, *J* = 9.5 Hz, 1H), 7.76 (s, 2H), 7.63 (s, 1H), 7.51 (dd, *J* = 9.6, 6.0 Hz, 1H), 6.75 (d, *J* = 7.3 Hz, 1H), 6.60 (d, *J* = 8.2 Hz, 1H), 5.37 (s, 2H), 5.23 (s, 1H), 4.74 (d, J = 8.8 Hz, 2H), 4.62 (dd, *J* = 14.7, 7.2 Hz, 1H), 4.40 (s, 1H), 3.96 (d, J = 10.5 Hz, 5H), 3.00 (t, *J* = 12.3 Hz, 2H), 2.73 (s, 1H), 2.64 (s, 1H), 2.54 (s, 3H), 2.47 (d, J = 5.7 Hz, 1H), 2.30 (dd, *J=* 25.5, 12.1 Hz, 2H), 1.95 - 1.80 (m, 4H).

LC-MS: (ESI) m/z: 575.3 [M+H]⁺.

### (5) Preparation of Compound 3

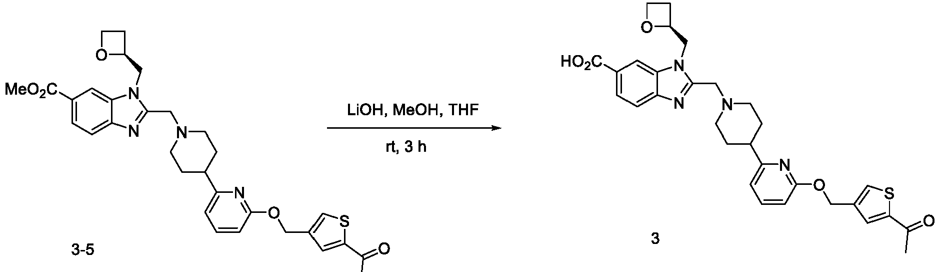

LiOH (5 mL) was added to a solution of compound 3-5 (150 mg, 0.26 mmol) in THF (2 mL) and MeOH (2 mL). The mixture was stirred at room temperature for 3 hours, and then the solvent was removed under reduced pressure to give a crude product which was purified by HPLC (gradient: 10% MeCN/90% H₂O, 0.1% NH₃·H₂O to 100% MeCN) to give (S)-2-((4-(6-((5-acetylthiophen-3-yl)methoxy)pyridin-2-yl) piperidin-1-yl)methyl)-1-(oxetan-2-ylmethyl)-1H-benzo[d]imidazole-6-carboxylic acid as compound 3 (62.17 mg, 43.6%).

¹H NMR (400 MHz, DMSO-J6) δ 8.13 (s, 1H), 7.98 (d, *J* = 5.2 Hz, 2H), 7.80 (d, *J* = 8.3 Hz, 1H), 7.63 (t, *J =* 7.7 Hz, 1H), 7.47 (d, *J* = 8.3 Hz, 1H), 6.88 (d, *J* = 7.3 Hz, 1H), 6.66 (d, *J* = 8.2 Hz, 1H), 5.36 - 5.29 (m, 2H), 5.17 - 5.06 (m, 1H), 4.66 (ddd, J = 17.8, 15.2, 4.7 Hz, 2H), 4.51 - 4.32 (m, 2H), 3.92 (d, J = 13.4 Hz, 1H), 3.77 (d, *J =* 13.4 Hz, 1H), 2.95 (dd, *J* = 45.6, 11.0 Hz, 2H), 2.67 (dt, *J* = 26.5, 9.7 Hz, 2H), 2.48 (s, 3H), 2.47 - 2.40 (m, 1H), 2.30 - 2.12 (m, 2H), 1.90 - 1.65 (m, 4H).

LC-MS: (ESI) m/z: 561.3 [M+H]⁺.

### Example 4

### (S)-2-((4-(6-((5-Cyanothiophen-2-yl)methoxy)pyridin-2-yl)piperidin-1-yl)methyl)-1-(oxetan-2-yl methyl)-1H-benzo[d]imidazole-6-carboxylic acid (Compound 4)

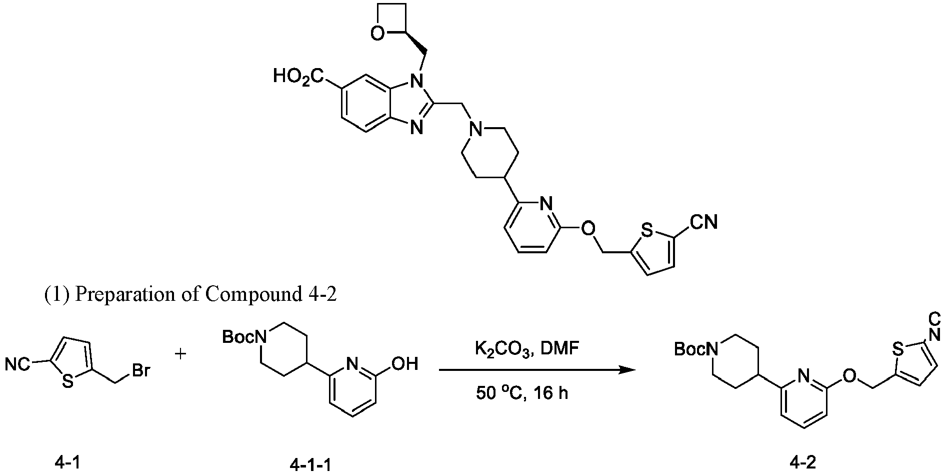

A mixture of compound 4-1 (200 mg, 9.13 mmol), compound 4-1-1 (331 mg, 1.19 mmol) and K₂CO₃ (274 g, 1.98 mmol) in DMF (5 mL) was stirred at 50°C for 16 hours. Water (20 mL) was added, and the reaction solution was extracted with EtOAc (3 × 20 mL). The combined organic phases were washed with brine (20 mL), dried (Na₂SO₄), filtered, and concentrated. After processed by flash chromatography (SiO₂, 25% EtOAc-hexane), tert-butyl 4-(6-((5-cyanothiophen-2-yl)methoxy)pyridin-2-yl) piperidine-1-carboxylate was given as compound 4-2 (270 mg, 68.4%).

¹H NMR (400 MHz, CDCl3) δ 7.60 - 7.44 (m, 2H), 7.11 (d, *J* = 3.8 Hz, 1H), 6.78 (d, *J* = 7.3 Hz, 1H), 6.62 (d, *J =* 8.1 Hz, 1H), 5.57 (s, 2H), 4.23 (s, 2H), 2.94 - 2.68 (m, 3H), 1.90 (d, *J* = 12.4 Hz, 2H), 1.82 - 1.61 (m, 2H), 1.49 (s, 9H).

LC-MS: (ESI) m/z: 344.1 [M+H]⁺.

### (2) Preparation of Compound 4-3

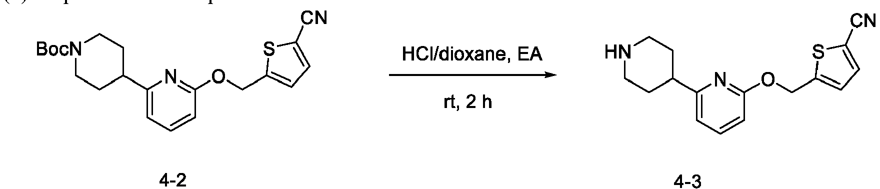

HCl/dioxane (5 mL) was added to a solution of compound 4-2 (270 mg, 0.68 mmol) in EtOAc (3 mL). The mixture was stirred at room temperature for 1 hour, and then concentrated to give a solid. The solid was dissolved in water (10 mL), adjusted to pH > 7 with NaHCO₃ (eq), and extracted with EtOAc (10 mL × 3). The combined organic phases were washed with brine (10 mL), dried (Na₂SO₄), filtered, and concentrated to give (S)-methyl 2-((4-(6-((5-cyanothiophen-2-yl)methoxy)pyridin-2-yl)piperidin-1-yl)methyl)-1-(oxetan-2-ylmethyl)-1H-benzo[d]imidazole-6-carboxylate as compound 4-3 (190 mg, 94%).

¹H NMR (400 MHz, DMSO-*d6*) δ 7.88 (d, *J* = 3.8 Hz, 1H), 7.74 - 7.62 (m, 1H), 7.39 (d, *J* = 3.8 Hz, 1H), 6.91 (d, *J* = 7.3 Hz, 1H), 6.69 (d, *J* = 8.2 Hz, 1H), 5.62 (s, 2H), 3.11 (dd, *J* = 8.8, 6.1 Hz, 2H), 2.83 - 2.63 (m, 3H), 1.84 (d, *J* = 11.9 Hz, 2H), 1.70 (m, *J* = 12.3, 4.1 Hz, 2H).

### (3) Preparation of Compound 4-4

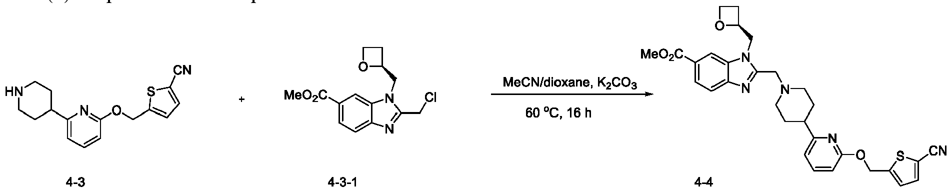

K₂CO₃ (369 mg, 2.67 mmol) was added to a mixture of compound 4-3 (190 mg, 0.67 mmol) and compound 4-3-1 (200 mg, 0.67 mmol) in dioxane (6 mL) and CH₃CN (3 mL). The mixture was stirred at 60°C for 16 hours. Water (20 mL) was added, and the solution was extracted with DCM (10 mL × 3). The combined organic phases were washed with brine (30 mL), dried (Na₂SO₄), filtered, and concentrated. After processed by flash chromatography (silica gel, eluted with 0-10% MeOH/DCM), (S)-methyl 2-((4-(6-((5-cyanothiophen-2-yl)methoxy)pyridin-2-yl)piperidin-1-yl)methyl)-1-(oxetan-2-ylmethyl)-1H-b enzo[d]imidazole-6-carboxylate was given as compound 4-4 (240 mg, 64.4%).

¹H NMR (400 MHz, CDCl₃) δ 8.16 (s, 1H), 7.97 (d, *J* = 9.0 Hz, 1H), 7.75 (d, *J* = 8.5 Hz, 1H), 7.56 - 7.49 (m, 2H), 7.11 (d, *J* = 3.1 Hz, 1H), 6.79 (d, *J* = 7.4 Hz, 1H), 6.60 (d, *J* = 8.4 Hz, 1H), 5.57 (s, 2H), 5.23 (s, 1H), 4.74 (d, *J* = 8.5 Hz, 2H), 4.61 (td, *J* = 8.1, 6.2 Hz, 1H), 4.47 - 4.35 (m, 1H), 3.97 (d, *J* = 14.9 Hz, 5H), 3.01 (t, *J* = 12.3 Hz, 2H), 2.79 - 2.61 (m, 2H), 2.40 (m, 3H), 1.89 (dd, *J* = 28.0, 11.6 Hz, 4H).

LC-MS: (ESI) m/z: 558.3 [M+H]⁺.

### (4) Preparation of Compound 4

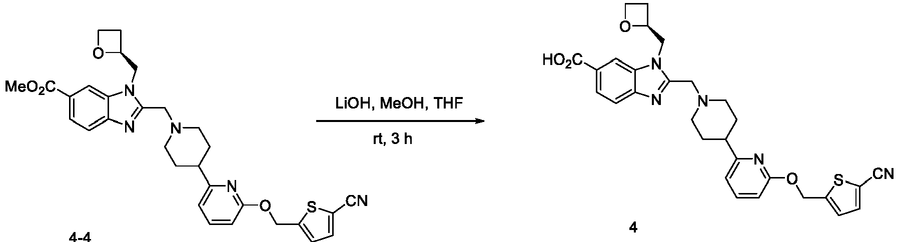

LiOH (2 mL) was added to a solution of compound 4-4 (50 mg, 0.09 mmol) in THF (1 mL) and MeOH (1 mL). The mixture was stirred at room temperature for 2 hours, and then the solvent was removed under reduced pressure to give a crude product which was purified by HPLC (gradient: 10% MeCN/90% H₂O, 0.1%NH₃·H₂O to 100% MeCN) to give (S)-2-((4-(6-((5-cyanothiophen-2-yl)methoxy)pyridin-2-yl) piperidin-1-yl)methyl)-1-(oxetan-2-ylmethyl)-1H-benzo[d]imidazole-6-carboxylic acid as compound 4 (10.73 mg, 22.0%).

¹H NMR (400 MHz, DMSO-J6) δ 8.15 (s, 1H), 7.88 (d, *J* = 3.8 Hz, 1H), 7.80 (dd, *J* = 8.4, 1.2 Hz, 1H), 7.67 (t, *J* = 7.8 Hz, 1H), 7.51 (d, *J=* 8.4 Hz, 1H), 7.37 (d, *J* = 3.8 Hz, 1H), 6.93 (d, *J* = 7.3 Hz, 1H), 6.68 (d, *J* = 8.2 Hz, 1H), 5.62 (s, 2H), 5.11 (qd, *J* = 7.1, 3.2 Hz, 1H), 4.69 (ddd, *J* = 18.0, 15.2, 5.0 Hz, 2H), 4.50 - 4.34 (m, 2H), 3.94 (d, *J* = 13.4 Hz, 1H), 3.78 (d, *J* = 13.4 Hz, 1H), 3.02 (d, *J* = 11.4 Hz, 1H), 2.90 (d, *J* = 11.1 Hz, 1H), 2.68 (m, 2H), 2.48 - 2.39 (m, 1H), 2.30 - 2.13 (m, 2H), 1.92 -1.69 (m, 4H).

LC-MS: (ESI) m/z: 544.3 [M+H]⁺.

### Example 5

### (S)-2-((4-(6-((5-acetyl-3-chlorothien-2-yl)methoxy)pyridin-2-yl)piperidin-1-yl)methyl)-1-(oxetan-2-yl methyl)-1H-benzo[d]imidazole-6-carboxylic acid (Compound 5)

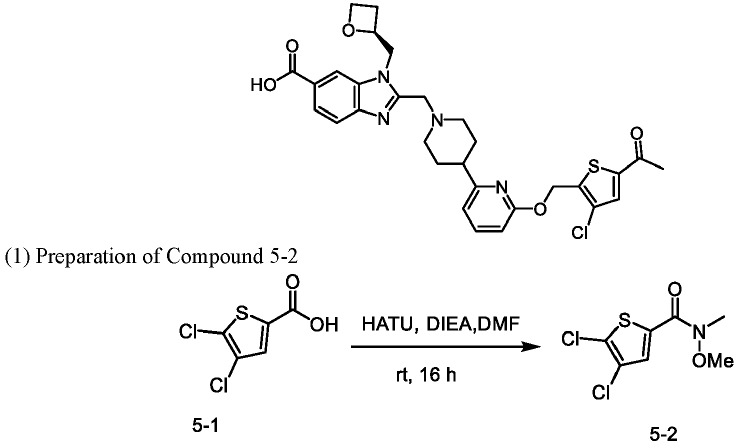

To a solution of compound 5-1 (1 g, 5.08 mmol) and N,O-dimethylhydroxylamine (541 mg, 5.58 mmol) in anhydrous DMF (20 mL) was added HATU (2.89 G, 7.62 mmol), DIEA (3.28 G, 25.40 mmol). The reaction mixture was stirred at room temperature for 16 hours. The reaction mixture was mixed between water (50 mL) and EtOAc (50 mL × 3). The combined organic phases were washed with brine (50 mL), dried (Na₂SO₄), filtered, and concentrated. After processed by flash chromatography (SiO₂, 0-100% EtOAc-hexane), 4,5-dichloro-N-methoxy-N-methylthiophene-2-carboxamide was given as compound 5-2 (900 mg, 75.0%).

¹H NMR (400 MHz, DMSO-*d₆*) δ 7.76 (1 H, s), 3.80 (3 H, s), 3.29 (3 H, s).

LCMS: (ESI) m/z: 240.0 [M+H]⁺.

### (2) Preparation of Compound 5-3

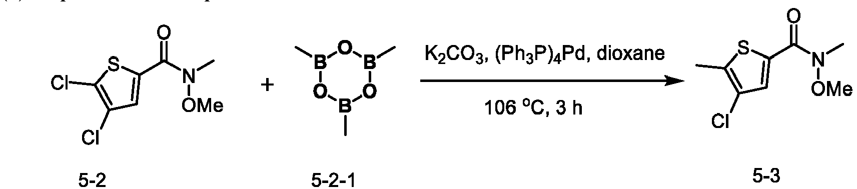

A mixture of compound 5-2 (900 mg, 3.75 mmol), compound 5-2-1 (3.96 mL, 13.87 mmol), K₂CO₃ (1.6 g, 11.62 mmol) and (Ph₃P)₄Pd (433 mg, 0.37 mmol) in dioxane (10 mL) was stirred at 106°C for 3 hours, and then cooled to room temperature. The mixture was filtered through Celite, and the filtrate was concentrated under reduced pressure. The residue was purified by silica gel chromatography (SiO₂, 40% EtOAc-hexane) to give 4-chloro-N-methoxy-N,5-dimethylthiophene-2-carboxamide as compound 5-3 (700 mg, 85.3%).

¹H NMR (400 MHz, DMSO-J6) δ7.62 (1 H, s), 3.76 (3 H, s), 3.27 (3 H, s), 2.41 (3 H, s).

LCMS: MF12-MC21-01-066 R1, (ESI) m/z: 220.0 [M+H]⁺.

### (3) Preparation of Compound 5-4

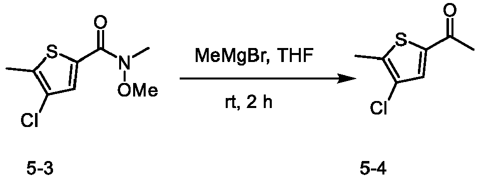

To a solution of CH₃MgBr (2.1 mL, 6.4 mmol) in THF was added dropwise a solution of compound 5-3 (700 mg, 3.2 mmol) in anhydrous THF (5 mL) at 0°C under nitrogen. After quenching the reaction mixture with NH₄Cl (eq), the reaction mixture was stirred at room temperature for 2 hours. The solution was extracted with water (15 mL) and EtOAc (15 mL × 3). The organic phase was washed with brine, and dried (Na₂SO₄). After filtration, the solvent was concentrated under reduced pressure to give 1-(4-chloro-5-methylthiophen-2-yl)ethan-1-one as compound 5-4 (450 mg, 80.9%).

¹H NMR (400 MHz, DMSO-*d6*) δ 7.93 (1 H, s), 2.50 (3 H, s), 2.44 (3 H, s).

LCMS: (ESI) m/z: 175.1 [M+H]⁺.

### (4) Preparation of Compound 5-5

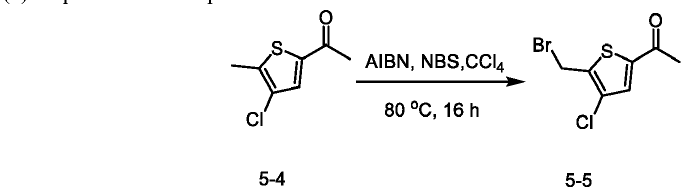

A mixture of compound 5-4 (450 mg, 2.58 mmol), AIBN (8 mg, 0.052 mmol) and NBS (505 mg, 2.84 mmol) in CCl₄ (10 mL) was stirred at 80°C for 16 hours. The reaction mixture was then quenched with Na₂S₂O₃. The solution was extracted with water (20 mL) and DCM (20 mL × 3). The combined organic phases were washed with brine (20 mL), dried (Na₂SO₄), filtered, and concentrated. After processed by flash chromatography (SiO₂, 0-100% EtOAc-hexane), 1-(5-(bromomethyl)-4-chlorothiophen-2-yl)ethan-1-one was given as compound 5-5 (550 mg, 84.3%).

¹H NMR (400 MHz, DMSO-*d6*) δ 8.00 (1 H, s), 4.94 (2 H, s), 2.55 (3 H, s).

LC-MS: (ESI) m/z: 253.0 [M+H]⁺.

### (5) Preparation of Compound 5-6

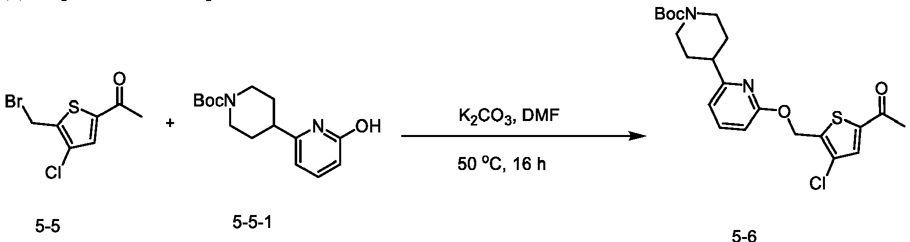

A mixture of compound 5-5 (530 mg, 1.98 mmol), compound 5-5-1 (702 mg, 2.38 mmol) and K₂CO₃ (580 mg, 3.97 mmol) in DMF (10 mL) was stirred at 50°C for 16 hours. The reaction mixture was mixed between water (20 mL) and EtOAc (20 mL × 3). The combined organic phases were washed with brine (20 mL), dried (Na₂SO₄), filtered, and concentrated. After processed by flash chromatography (SiOz, 0-100% EtOAc-hexane), tert-butyl 4-(6-((5-acetyl-3-chlorothiophen-2-yl)methoxy)pyridin-2-yl)piperidine-1-carboxylate was given as compound 5-6 (350 mg, 36.9%).

¹H NMR (400 MHz, DMSO-76) δ 7.96 (1 H, s), 7.70 - 7.60 (1 H, m), 6.92 (1 H, d, *J* =7.3), 6.71 (1 H, d, *J* =8.1), 5.56 (2 H, s), 4.09 - 4.02 (2 H, m), 2.96 - 2.75 (3 H, m), 2.52 (3 H, s), 1.80 (2 H, d, *J* =11.0), 1.61 (2 H, dd, *J* =12.3, 3.8), 1.41 (9 H, s).

LCMS: (ESI) m/z: 451.1 [M+H]⁺.

### (6) Preparation of Compound 5-7

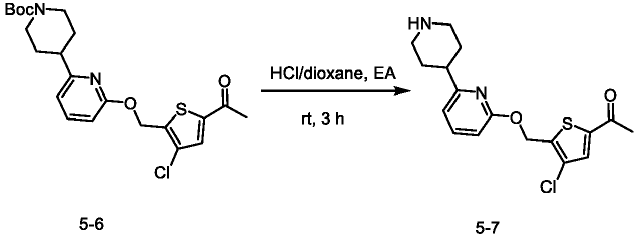

A solution of compound 5-6 (340 mg, 0.76 mmol) and HCl/dioxane (7 mL) in EtOAc (3 mL) was stirred at room temperature for 3 hours, and then concentrated to give a solid. The solid was dissolved in water (10 mL). The solution was adjusted to pH > 7 with NaHCO₃ (eq), and extracted with EtOAc (10 mL × 3). The combined organic phases were washed with brine (10 mL), dried (Na₂SO₄), filtered, and concentrated to give 1-(4-chloro-5-(((6-(piperidin-4-yl)pyridin-2-yl)oxy)methyl)thiophen-2-yl)ethane-1-one as compound 5-7 (200 mg, 75.7%).

¹H NMR (400 MHz, DMSO-J6) δ 7.98 (1 H, d, *J* =6.8), 7.67 (1 H, t, *J* =7.8), 6.89 (1 H, d, *J* =7.3), 6.70 (1 H, d, *J* =8.2), 5.57 (2 H, s), 3.04 (2 H, d, *J* =12.1), 2.68 (1 H, dd, *J* =9.5, 5.8), 2.61 (2 H, dd, *J* =12.1, 10.1), 2.52 (3 H, s), 1.78 (2 H, d, *J* =11.2), 1.62 (2 H, qd, *J* =12.3, 3.9).

LCMS: (ESI) m/z: 351.1 [M+H]⁺.

### (7) Preparation of Compound 5-8

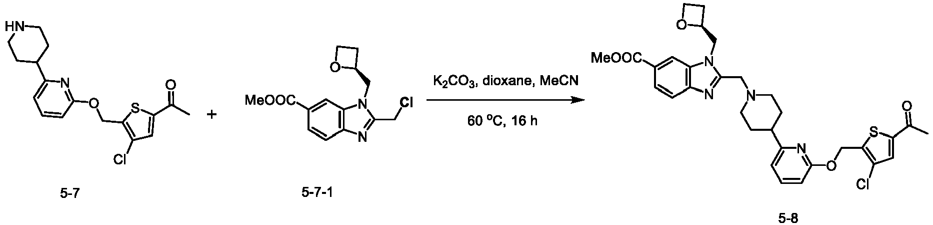

A mixture of compound 5-7 (200 mg, 0.57 mmol), compound 5-7-1 (185 mg, 0.63 mmol) and K₂CO₃ (315 mg, 2.28 mmol) in dioxane (10 mL) and CH₃CN (5 mL) was stirred at 60°C for 16 hours. The solution was extracted with water (30 mL) and DCM (30 mL × 3). The combined organic phases were washed with brine (30 mL), dried (Na₂SO₄), filtered, and concentrated. After processed by flash chromatography (SiO₂, 0-100% EtOAc-hexane), (S)-methyl 2-((4-(6-((5-acetyl-3-chlorothien-2-yl)methoxy)pyridin-2-yl)piperidin-1-yl)methyl)-1-(oxetan-2-ylmethyl)-1H-benzo[d]imidazole-6-carboxylate was given as compound 5-8 (200 mg, 57.8%).

¹H NMR (400 MHz, DMSO-J6) δ 8.30 (1 H, d, *J* =1.2), 7.97 (1 H, s), 7.83 - 7.77 (1 H, m), 7.71 - 7.61 (2 H, m), 6.92 (1 H, d, *J* =7.3), 6.70 (1 H, d, *J* =8.1), 5.56 (2 H, s), 5.11 (1 H, dd, *J* =7.2, 2.3), 4.83 (1 H, dd, *J* =15.2, 7.2), 4.68 (1 H, dd, *J* =15.2, 2.6), 4.46 (1 H, dd, *J* =10.6, 4.8), 4.36 (1 H, dt, *J* =9.0, 5.9), 4.07 - 3.91 (1 H, m), 3.87 (3 H, s), 3.79 (1 H, d, *J* =13.5), 3.01 (1 H, d, *J* =11.5), 2.86 (1 H, d, *J* =11.4), 2.75 - 2.58 (2 H, m), 2.50 (3 H, s), 2.44 (1 H, s), 2.22 (2 H, dd, *J* =27.9, 2.8), 1.89 - 1.68 (4 H, m).

LC-MS: (ESI) m/z: 609.2 [M+H]⁺.

### (8) Preparation of Compound 5

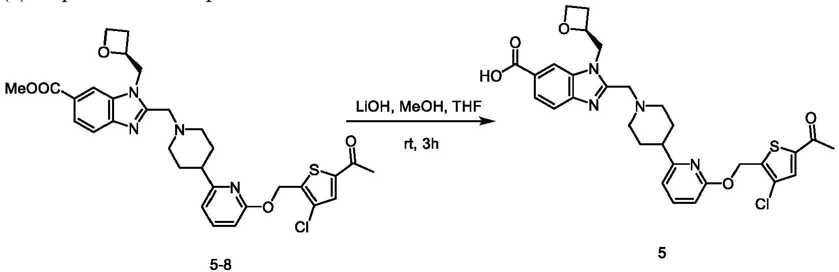

A solution of compound 5-8 (177 mg, 0.29 mmol) and LiOH (4 mL) in THF (2.5 mL) and MeOH (1.5 mL) were stirred at room temperature for 3 hours. The solvent was then removed under reduced pressure to give a crude product which was purified by HPLC (gradient: 10% MeCN/90% H₂O, 0.1%NH₃·H₂O to 100% MeCN) to give (S)-2-((4-(6-((5-acetyl-3-chlorothiophen-2-yl)methoxy)pyridin-2-yl)piperidin-1-yl)methyl) -1-(oxetan-2-ylmethyl)-1H-benzo[d]imidazole-6-carboxylic acid as compound 5 (79.71 mg, 46.2%).

¹H NMR (400 MHz, DMSO-J6) δ 8.10 (1 H, s), 7.97 (1 H, s), 7.80 (1 H, d, *J* =8.4), 7.66 (1 H, t, *J* =7.7), 7.44 (1 H, d, *J* =8.3), 6.92 (1 H, d, *J* =7.3), 6.70 (1 H, d, *J* =8.2), 5.56 (2 H, s), 5.17 - 5.03 (1 H, m), 4.73 (1 H, dd, *J* =15.2, 6.9), 4.64 - 4.56 (1 H, m), 4.47 (1 H, dd, *J* =13.9, 7.3), 4.38 (1 H, dt, *J* =11.8, 5.9), 3.91 (1 H, d, *J* =13.3), 3.76 (1 H, d, *J* =13.3), 2.99 (1 H, d, *J* =10.8), 2.88 (1 H, d, *J* =10.9), 2.66 (2 H, dd, *J* =13.1, 5.5), 2.50 (3 H, s), 2.46 (1 H, d, *J* =8.8), 2.27 - 2.10 (2 H, m), 1.79 (4 H, dd, *J* =16.6, 10.3).

LC-MS: (ESI) m/z: 595.2 [M+H]⁺.

### Example 6

### (S)-2-((4-(6-((5-(cyclopropanecarbonyl)thiophen-2-yl)methoxy)pyridin-2-yl)piperidin-1-yl)methyl)-1-(oxetan-2-ylmethyl)-1H-benzo[d]imidazole-6-carboxylic acid (Compound 6)

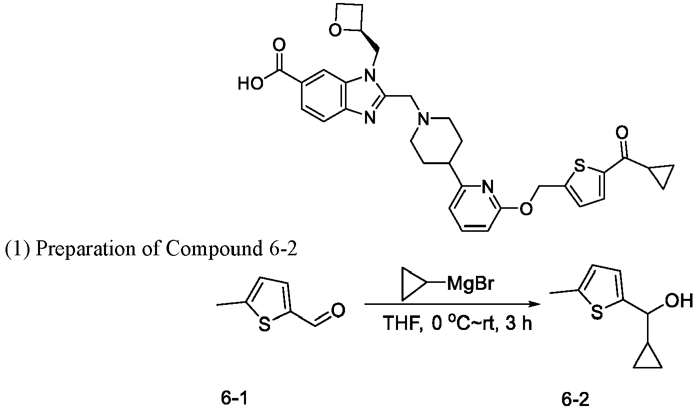

To a solution of 6-1 (5.0 g, 39.6 mmol) in anhydrous THF (50 mL) was added dropwise cyclopropylmagnesium bromide (1.0 M in THF, 47.6 mL, 47.6 mmol) at 0°C under N₂. The mixture was stirred at this temperature for 0.5 hour, and then stirred at room temperature under N₂ for 2.5 hours. The reaction solution was quenched with H₂O (100 mL), and extracted with EtOAc (3 × 100 mL). The combined organic phases were washed with brine (100 mL), dried (Na₂SO₄), filtered, and concentrated. After processed by flash chromatography (SiO₂, EtOAc-hexane), 6-2 was given (7 g, 99.9%).

¹H NMR (400 MHz, DMSO-J6) δ 6.72 (d, *J* = 3.3 Hz, 1H), 6.63 - 6.55 (m, 1H), 5.37 (d, *J* = 4.9 Hz, 1H), 4.08 (dd, *J* = 7.3, 5.0 Hz, 1H), 2.38 (d, *J* = 0.6 Hz, 3H), 1.06 (qt, *J* = 7.9, 5.0 Hz, 1H), 0.47 - 0.42 (m, 2H), 0.41 - 0.36 (m, 1H), 0.28 (ddd, J = 9.3, 4.2, 1.4 Hz, 1H).

### (2) Preparation of Compound 6-3

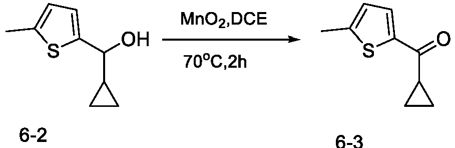

6-2 (8.6 g, 51.12 mmol) was added to a solution of manganese oxide (44.44 g, 511.1 mmol) in DCE (172 mL). The mixture was stirred at 70°C for 3 hours. The mixture was filter off, and the filtrate was concentrated. After processed by flash chromatography (SiO₂, 16% EtOAc-hexane), 6-3 was given (7 g, 83.8%).

¹H NMR (400 MHz, CDCl₃)δ 7.63 (d, *J* = 4.0 Hz, 1H), 6.81-2-6.79 (m, 1H), 2.53 (d, *J* = 0.6 Hz, 3H), 2.50-2.42 (m, 1H), 1.23 - 1.17 (m, 2H), 1.01-0.94 (m, 2H).

### (3) Preparation of Compound 6-4

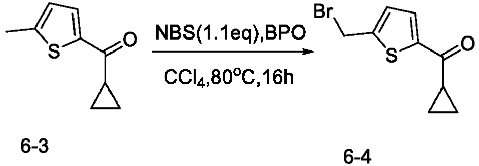

6-3 (700 mg, 4.211 mmol) was added to a solution of BPO (20.4 mg, 0.0842 mmol) and NBS (824.4 mg, 4.632 mmol) in CCl₄ (14 mL). The mixture was stirred at 80°C for 16 hours. After dilution with EA, the mixture was extracted with EA (200 mL × 3) and HzO. The organic layer was concentrated in vacuo. After processed by flash chromatography (SiO₂, 16% EtOAc-hexane), 6-4 was given (700 mg, yield: 67.9%).

¹H NMR (400 MHz, CDCl₃) δ 7.67 - 7.63 (m, 1H), 7.14 (d, *J* = 3.8 Hz, 1H), 4.68 (s, 2H), 2.53-2.45 (m, 1H), 1.29 - 1.21 (m, 2H), 1.08 - 1.00 (m, 2H).

### (4) Preparation of Compound 6-5

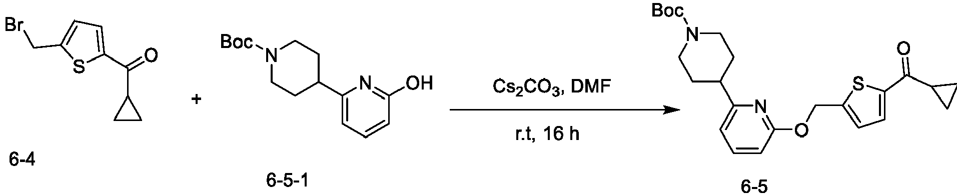

To a solution of 6-4 (200 mg, 0.816 mmol) and 6-5-1 (340.6 mg, 1.224 mmol) in DMF (2 ml) was added Cs₂CO₃ (798 mg, 2.448 mmol). The mixture was stirred under N₂ at room temperature for 12 hours, and extracted with H₂O (5 mL) and EA (10 mL * 3). The organic phase was washed with H₂O (10 mL * 3) and brine (10 mL), and dried over Na₂SO₄, and concentrated. The residue was purified by flash column chromatography to give compound 6-5 (168 mg, yield: 74.4%).

¹H NMR (400 MHz, DMSO-*d*₆)δ 7.66-7.54 (m, 2H), 7.28 (d, *J* = 9.4 Hz, 2H), 6.86 (d, J = 7.2 Hz, 1H), 6.69 (d, *J* = 8.0 Hz, 1H), 5.39 (s, 2H), 4.74 (q, *J* = 7.2 Hz, 4H), 4.06 - 4.00 (m, 2H), 3.03 (s, 3H), 2.90 - 2.70 (m, 3H), 1.74 *(d, J=* 12.0 Hz, 2H), 1.57 (d, *J* = 12.1 Hz, 2H), 1.42 (s, 9H).

### (5) Preparation of Compound 6-6

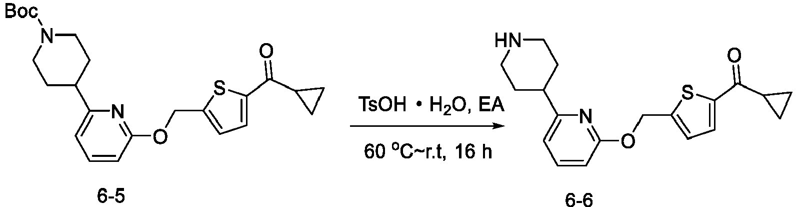

6-5 (168 mg, 0.380 mmol) was dissolved in a solution of TsOH · H₂O (216.8 mg, 1.140 mmol) in EA (10 mL). The mixture was stirred at 60°C under N₂ atmosphere for 1 hour. The mixture was stirred at room temperature under N₂ atmosphere for 16 hours. The mixture was filtered, and the filter cake was washed with EA (5 mL * 3), and dried to give 6-6 (130 mg).

LC-MS: (ESI) m/z: 343.1 [M+H]⁺.

### (6) Preparation of Compound 6-7

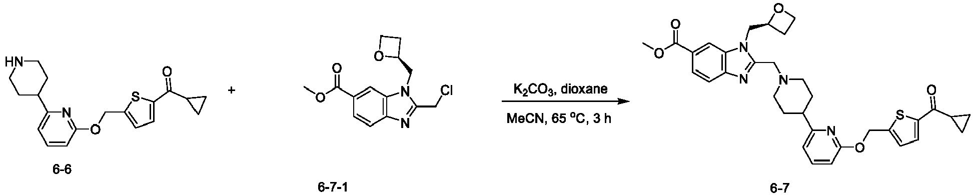

A mixture of 6-6 (130 mg, 0.380 mmol) and 6-7-1 (112 mg, 0.380 mmol) was added to dioxane (9 ml) and MeCN (6 ml) as solvents followed by addition of K₂CO₃ (419 mg). The mixture was stirred at 65°C for 16 hours, and extracted with EA (100 ml * 3) and H₂O (100 ml). The organic phase was washed with brine (100 ml), and dried over Na₂SO₄. The residue was purified by column chromatography (PE: EA = 5:1) to give 6-7 (112 mg, yield: 49.1%).

LC-MS: (ESI) m/z: 601.3 [M+H]⁺.

### (7) Preparation of Compound 6

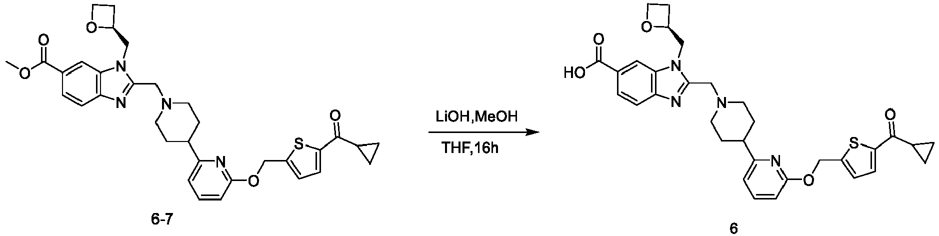

7-7 (40 mg, 0.0666 mmol) and 1 M LiOH (0.5 mL) were added to MeOH (0.5 mL) and THF (0.5 ml). The mixture was stirred at room temperature for 16 hours while removing the solvent under reduced pressure to obtain a crude product, which was purified by HPLC to give 5.78 mg of compound 6.

¹H NMR (400 MHz, DMSO-*d*₆)δ 8.13 (s, 1H), 8.00 (d, J = 3.8 Hz, 1H), 7.78 (d, J = 8.4 Hz, 1H), 7.65 (t, J = 7.8 Hz, 1H), 7.49 (d, J = 8.4 Hz, 1H), 7.32 (d, J = 3.8 Hz, 1H), 6.90 (d, J = 7.2 Hz, 1H), 6.67 (d, J = 8.2 Hz, 1H), 5.59 (s, 2H), 5.13-5.06 (m, 1H), 4.78-4.69 (m, 1H), 4.65-4.55 (m, 1H), 4.49-4.41 (m, 1H), 4.40-4.32(m, 1H), 3.92 (d, J = 13.4 Hz, 1H), 3.76 (d, J = 13.4 Hz, 1H), 3.17 (s, 1H), 3.00 (d, J = 11.2 Hz, 1H), 2.87 (d, J = 11.2 Hz, 1H), 2.80-2.73 (m, 1H), 2.69 - 2.62 (m, 2H), 2.46 - 2.40 (m, 1H), 2.21 (m, 2H), 1.78 (m, 4H), 1.00 - 0.95 (m, 4H).

### Example 7

### (S)-2-((4-(6-((3-chloro-5-cyanothiophen-2-yl)methoxy)pyridin-2-yl)piperidin-1-yl)methyl)-1-(oxetan-2-ylmethyl)-1H-benzo[d]imidazole-6-carboxylic acid (Compound 7)

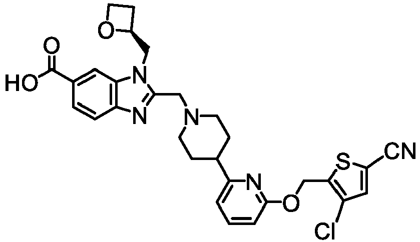

### (1) Preparation of Compound 7-2

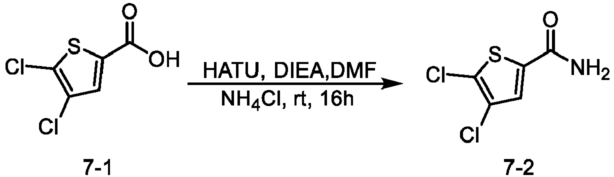

To a solution of 7-1 (3.4 g, 17.26 mmol) in DMF (50 mL) was added DIEA (11.15 g, 86.3 mmol). Then HATU (9.86 g, 25.89 mmol) was added. The mixture was stirred at room temperature for 0.5 hour. NHaCl (1 G, 18.98 mmol) was then added. The mixture was stirred at room temperature for 16 hours. H₂O (100 mL) was added, and the reaction solution was extracted with EtOAc (3 × 100 mL). The combined organic phases were washed with H₂O (3 × 100 mL) and brine (100 mL), dried (Na₂SO₄), filtered, and concentrated. After processed by flash chromatography (SiO₂, hexane), 2.6 g of compound 7-2 was given with a yield of 76.9%.

¹H NMR (400 MHz, DMSO-d6) δ 8.14 (s, 1H), 7.80 (s, 1H), 7.75 (s, 1H).

### (2) Preparation of Compound 7-3

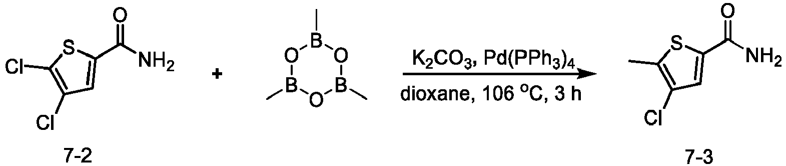

A mixture of 7-2 (1.0 g, 5.10 mmol) and 2,4,6-trimethyl-1,3,5,2,4,6-trioxatriborinane (3.5 M in THF, 5.4 mL, 18.87 mmol) was added to the solvent dioxane (10 mL). Then K₂CO₃ (2.2 g, 15.81 mmol) and Pd(PPh₃)₄ (589 mg, 0.51 mmol) were added. The reaction solution was stirred at 106°C for 3 hours. H₂O (10 mL) was added, and the reaction solution was extracted with EtOAc (3 × 10 mL). The combined organic phases were washed with brine (10 mL), dried (Na₂SO₄), filtered, and concentrated. After purification by flash chromatography (SiOz, EtOAc-hexane) 300 mg of compound 7-3 was given with a yield of 33.4%.

δ H (400 MHz, DMSO-d6) 7.96 (1 H, s), 7.66 (1 H, s), 7.49 (1 H, s), 2.38 (3 H, s).

### (3) Preparation of Compound 7-4

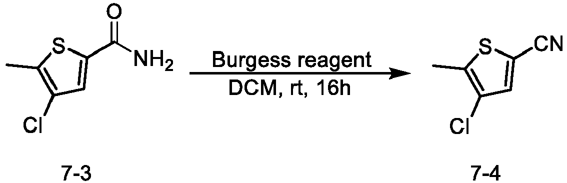

To a solution of 7-3 (530 mg, 3.03 mmol) in DCM (30 mL) was added Burgess reagent (2.2 g, 9.08 mmol). The mixture was stirred at room temperature under N₂ atmosphere for 16 hours. H₂O (30 mL) was added, and the reaction solution was extracted with DCM (3 × 30 mL). The combined organic phases were washed with brine (30 mL), dried (Na₂SO₄), filtered, and concentrated. After processed by flash chromatography (SiO₂, EtOAc-hexane), 390 mg of compound 7-4 was given with a yield of 81.9%.

¹H NMR (400 MHz, DMSO-76) δ 8.00 (s, 1H), 2.46 (s, 3H).

### (4) Preparation of Compound 7-5

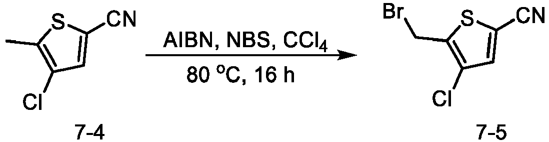

To a solution of 7-4 (390 mg, 2.48 mmol) in CCl₄ (8 mL) was added NBS (483 g, 2.73 mmol) followed by addition of AIBN (6 mg, 0.05 mmol). The mixture was stirred at 80°C under N₂ atmosphere for 16 hours. H₂O (10 mL) was added, and the reaction solution was extracted with DCM (3 × 10 mL). The combined organic phases were washed with brine (10 mL), dried (Na₂SO₄), filtered, and concentrated. After purification by flash chromatography (SiO₂, EtOAc-hexane), 416 mg of compound 7-5 was given with a yield of 71.2%.

δ H (400 MHz, DMSO-76) 8.08 (1 H, s), 4.98 (2 H, s).

### (5) Preparation of Compound 7-6

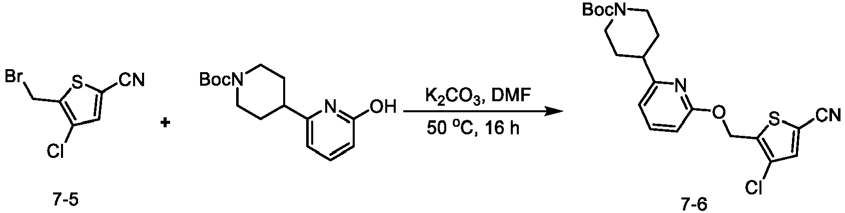

A mixture of 7-5 (416 mg, 1.76 mmol) and tert-butyl 4-(6-hydroxypyridin-2-yl) piperidine-1-carboxylate (590 mg, 2.11 mmol) were added to the solvent DMF (8 mL). Then K₂CO₃ (482 mg, 3.52 mmol) was added. The reaction solution was stirred at 50°C for 16 hours. H₂O (10 mL) was added, and the reaction solution was extracted with EtOAc (3 × 10 mL). The combined organic phases were washed with brine (10 mL), dried (Na₂SO₄), filtered, and concentrated. After purification by flash chromatography (SiO₂, EtOAc-hexane), 300 mg of compound 7-6 was given with a yield of 39.1%.

δ H (400 MHz, DMSO-76) 8.12 (1 H, s), 7.75 (1 H, dd, *J* =8.1, 7.4), 7.00 (1 H, d, *J* =7.2), 6.78 (1 H, d, *J* =8.1), 5.63 (2 H, s), 4.17 - 4.04 (2 H, m), 2.88 (3 H, ddd, *J* =11.7, 8.2, 3.6), 1.87 (2 H, d, *J* =10.9), 1.68 (2 H, td, *J* =12.5, 4.1), 1.47 (10 H, s).

### (6) Preparation of Compound 7-7

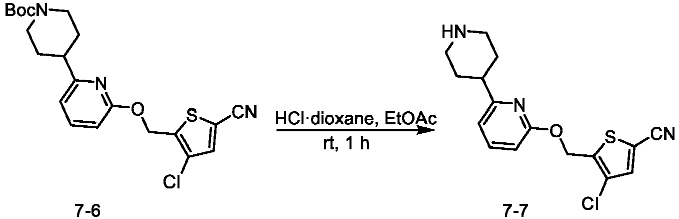

To a solution of 7-6 (300 mg, 0.692 mmol) in EtOAc (2 mL) was added HCl · dioxane (4 mL). The mixture was stirred at room temperature for 1 hour. The reaction solution was concentrated to obtain 160 mg of compound 7-7, yield: 69.2%

δ H (400 MHz, DMSO-d6) 8.07 (1 H, s), 7.68 (1 H, t, *J* =7.8), 6.91 (1 H, d, *J* =7.3), 6.70 (1 H, d, *J* =8.2), 5.58 (2 H, s), 3.03 (2 H, d, *J* =11.7), 2.70 (1 H, t, *J* =11.8), 2.59 (2 H, t, *J* =11.6), 1.78 (2 H, d, *J* =1.2), 1.62 (2 H, td, *J* =12.2, 3.8).

### (7) Preparation of Compound 7-8

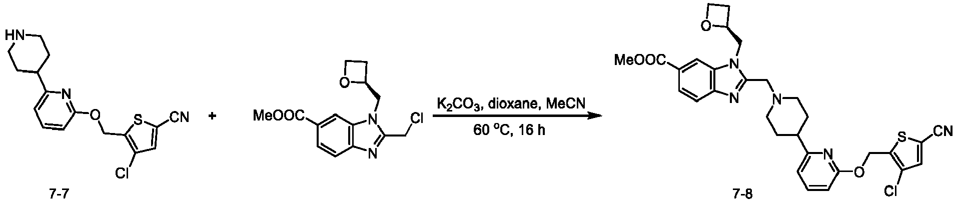

A mixture of 7-7 (160 mg, 0.479 mmol) and (S)-methyl 2-(chloromethyl)-1-(oxetan-2-ylmethyl)-1H-benzo[d]imidazole-6-carboxylate (155 mg, 0.527 mmol) was added to the solvents dioxane (6 mL) and MeCN (3 mL) followed by addition of K₂CO₃ (265 mg, 1.917 mmol). The reaction solution was stirred at 60°C for 16 hours. H₂O (10 mL) was added, and the reaction solution was extracted with EtOAc (3 × 10 mL). The combined organic phases were washed with brine (10 mL), dried (Na₂SO₄), filtered, and concentrated. After purification by flash chromatography (SiO₂, EtOAc-hexane), 190 mg of compound 7-8 was given with a yield of 66.9%.

δ H (400 MHz, DMSO-d6) 8.29 (1 H, s), 8.07 (1 H, s), 7.82 (1 H, dd, *J* =8.5, 1.4), 7.73 - 7.64 (2 H, m), 6.94 (1 H, d, *J* =7.3), 6.71 (1 H, d, *J* =8.1), 5.58 (2 H, s), 5.11 (1 H, d, *J* =4.9), 4.83 (1 H, dd, *J* =15.2, 7.2), 4.68 (1 H, dd, *J* =15.2, 2.6), 4.47 (1 H, d, *J* =5.8), 4.42 - 4.33 (1 H, m), 4.01 - 3.93 (1 H, m), 3.87 (3 H, s), 3.80 (1 H, d, *J* =13.6), 3.01 (1 H, d, *J* =11.4), 2.87 (1 H, d, *J* =11.0), 2.79 - 2.59 (2 H, m), 2.43 (1 H, dd, *J* =18.9, 8.2), 2.31 - 2.16 (2 H, m), 1.88 - 1.66 (4 H, m).

### (8) Preparation of Compound 7

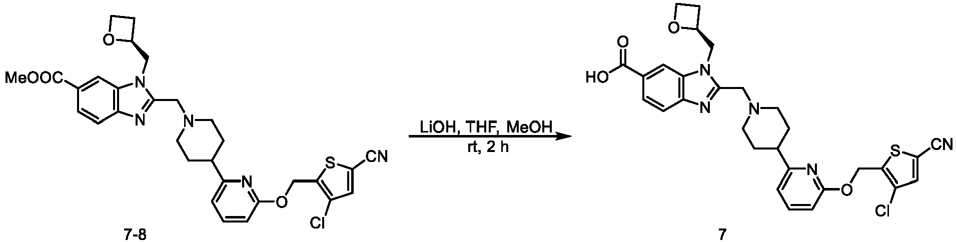

To a solution of 7-8 (90 mg, 0.152 mmol) in MeOH (2 mL) and THF (1 mL) was added 1 M LiOH (3 mL). The reaction solution was stirred at room temperature for 2 hours, concentrated, and purified by preparative HPLC to give 3.26 mg of compound 7, yield: 18.6%.

δ H (400 MHz, DMSO-*d6*) 8.38 (1 H, s), 8.23 (1 H, s), 8.06 (1 H, s), 7.82 (1 H, d, *J* =8.4), 7.68 (1 H, t, *J* =7.8), 7.58 (1 H, d, *J* =8.3), 6.94 (1 H, d, *J* =7.3), 6.71 (1 H, d, *J* =8.1), 5.58 (2 H, s), 5.11 (1 H, d, *J* =4.2), 4.79 (1 H, dd, *J* =15.0, 6.9), 4.65 (1 H, d, *J* =13.1), 4.47 (1 H, dd, *J* =13.5, 7.4), 4.41 - 4.32 (1 H, m), 3.94 (1 H, d, *J* =13.5), 3.83 - 3.73 (2 H, m), 3.00 (2 H, d, *J* =11.5), 2.88 (1 H, d, *J* =11.8), 2.67 (2 H, d, *J* =8.0), 2.45 (1 H, s), 2.29 - 2.12 (2 H, m), 1.78 (4 H, dd, *J* =37.9, 13.2).

LC-MS: (ESI) m/z: 578.2 [M+H]⁺.

### Example 8

### (S)-2-((4-(6-((4-chloro-5-cyanothiophen-2-yl)methoxy)pyridin-2-yl)piperidin-1-yl)methyl)-1-(oxetan-2-ylmethyl)-1H-benzoldlimidazole-6-carboxylic acid (Compound 8)

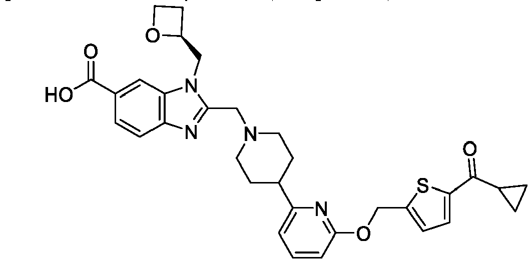

### (9) Preparation of Compound 8-2

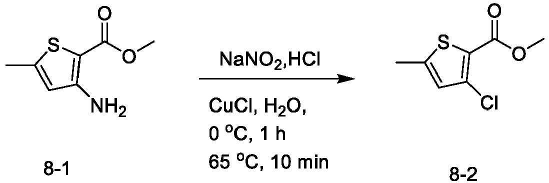

A mixture of 8-1 (1.0 g, 5.84 mmol) in 6 N HCl (5 ml) was cooled to 0°C, a solution of sodium nitrite (403 mg, 5.84 mmol) in water (1 ml) was added dropwise, and the mixture was stirred at 0°C for 60 minutes. The mixture was then added to a concentrated solution of copper chloride (578 mg, 5.84 mmol). HCl (5 ml) was added at 0°C. The reaction mixture was stirred at room temperature, and then warmed to 65°C until gas emitting ceased. The reaction mixture was diluted with water, extracted with EtOAc, washed with water and brine, dried over sodium sulfate, filtered, and evaporated. The residue was purified by flash column chromatography (12% EtOAc/heptane) to give 780 mg of 8-2, yield: 70.9%.

¹H NMR (400 MHz, CDCl3) δ 6.72 (d, *J* = 0.8 Hz, 1H), 3.86 (s, 3H), 2.47 (s, 3H).

LC-MS: MC21-306-011-P (ESI) m/z: 191.0 [M+H]⁺.

### (10) Preparation of Compound 8-3

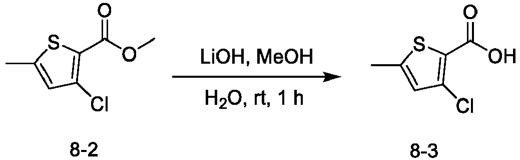

A solution of LiOH (196 mg, 8.18 mmol) in 1 mL H₂O was added to a solution of 8-2 (780 mg, 4.09 mmol) in MeOH (4 mL). The mixture was stirred at room temperature for 2 hours, then the solvent was removed under reduced pressure to obtain a crude product. The crude product was dissolved in water, and adjusted to PH = 2 with 1 mol/L hydrochloric acid. The mixture was filtered to give 8-3 (470 mg, crude).

¹H NMR (400 MHz, DMSO-*d6*) δ 6.96 (s, 1H), 2.46 (s, 3H).

### (11) Preparation of Compound 8-4

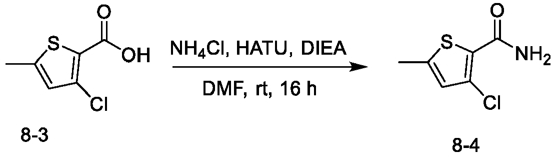

NH₄Cl (100 mg, 1.87 mmol) was added to a solution of 8-3 (300 mg, 1.70 mmol) in DMF (5 ml), and then HATU (969 mg, 2.55 mmol) and DIEA (1.10 G, 8.49 mmol) were separately added to that mixture. The mixture was stirred at room temperature under N₂ atmosphere for 16 hours. Water (20 mL) was added, the solution was extracted with ethyl acetate (10 mL × 3). The combined organic phases were washed with brine (30 mL), dried over Na₂SO₄, filtered, and concentrated. The residue was purified by flash column chromatography (28% EtOAc in heptane) to give 120 mg of 8-4, yield: 40.2%.

¹H NMR (400 MHz, DMSO-*d6*) δ 7.78 (s, 1H), 7.37 (s, 1H), 6.94 (s, 1H), 2.49 (s, 3H).

LC-MS: MC21-306-018-P, (ESI) m/z: 176.0 [M+H]⁺.

### (12) Preparation of Compound 8-5

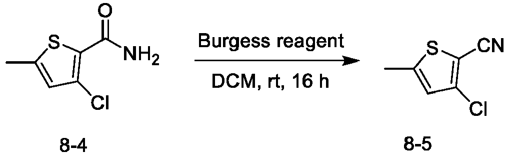

Burgess reagent (651 mg, 2.73 mmol) was added to a solution of 8-4 (160 mg, 0.91 mmol) in DCM (5 mL). The mixture was stirred at room temperature for 16 hours. Water (10 mL) was added, and the solution was extracted with DCM (10 mL × 3). The combined organic phases were washed with brine (30 mL), dried over Na₂SO₄, filtered, and concentrated. The residue was purified by flash column chromatography (25% EtOAc in heptane) to give 100 mg of 8-5, yield: 69.9%.

¹H NMR (400 MHz, DMSO-*d6*) δ 7.16 (*d*, *J* = 1.0 Hz, 1H), 2.53 (*d*, *J* = 0.9 Hz, 3H).

### (13) Preparation of Compound 8-6

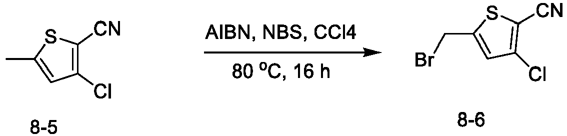

NBS (124 mg, 0.20 mmol) was added to a mixture of 8-5 (100 mg, 0.63 mmol) and AIBN (2 mg, 0.01 mmol) in CCl₄ (10 mL). Then the reaction solution was stirred at 80°C for 16 hours. The reaction mixture was then quenched with a saturated solution of Na₂S₂O₃ (5 mL). The solution was extracted with water (20 mL) and DCM (20 mL × 3). The combined organic phases were washed with brine (20 mL), dried over Na₂SO₄, filtered, and concentrated. After processed by flash chromatography (SiO₂, 0-50% EtOAc/hexane), 8-6 (50 mg, 33.6% yield) was given.

¹H NMR (400 MHz, DMSO-*d6*) δ 7.51 (s, 1H), 5.02 (s, 2H).

### (14) Preparation of Compound 8-7

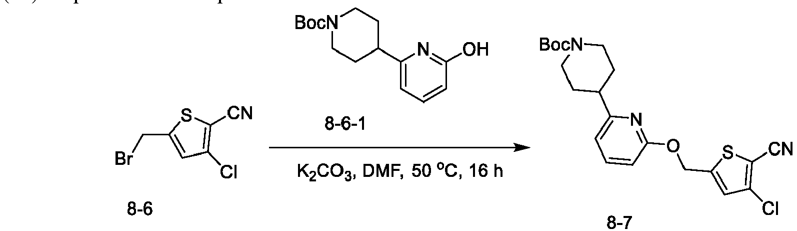

A mixture of 8-6 (50 mg, 0.21 mmol), 8-6-1 (71 mg, 0.25 mmol) and K₂CO₃ (59 mg, 0.42 mmol) in DMF (3 mL) was stirred at 50°C for 16 hours. Upon completion, H₂O (10 mL) was added, and the reaction solution was extracted with EtOAc (3×10 mL ). The combined organic phases were washed with brine (10 mL), dried (Na₂SO₄), filtered, and concentrated. After processed by flash chromatography (SiO₂, 25% EtOAc-hexane), 8-7 (40 mg, 44% yield) was given.

¹H NMR (400 MHz, DMSO-*d6*) δ 7.75 - 7.65 (m, 1H), 7.50 (s, 1H), 6.95 (d, *J* = 7.3 Hz, 1H), 6.72 (d, *J* = 8.1 Hz, 1H), 5.58 (s, 2H), 4.14 - 3.98 (m, 2H), 2.83 (t, *J* = 11.7 Hz, 3H), 1.84 (d, *J* = 12.7 Hz, 2H), 1.60 (qd, *J* = 12.6, 4.2 Hz, 2H), 1.41 (s, 9H).

### (15) Preparation of Compound 8-8

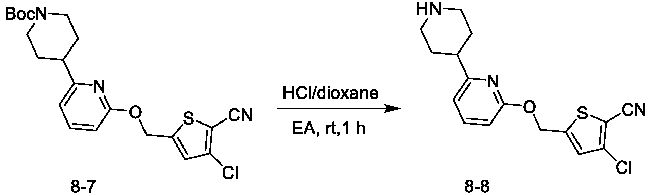

HCl/dioxane (2 mL) was added to a solution of 8-7 (40 mg, 0.09 mmol) in EtOAc (2 mL). The mixture was stirred at room temperature for 1 hour, and then concentrated to give a solid. The solid was dissolved in H₂O (3 mL), adjusted to pH > 7 with NaHCO₃ (eq), and extracted with EtOAc (5 mL × 3). The combined organic phases were washed with brine (10 mL), dried (Na₂SO₄), filtered, and concentrated to give 8-8 (35 mg, crude).

¹H NMR (400 MHz, DMSO-*d6*) δ 7.73 - 7.63 (m, 1H), 7.52 (s, 1H), 6.91 (d, *J* = 7.3 Hz, 1H), 6.70 (d, *J* = 8.1 Hz, 1H), 5.59 (s, 2H), 3.03 (d, *J* = 12.0 Hz, 2H), 2.74 - 2.54 (m, 3H), 1.78 (d, *J* = 13.0 Hz, 2H), 1.60 (qd, *J* = 12.3, 3.9 Hz, 2H).

### (16) Preparation of Compound 8-9

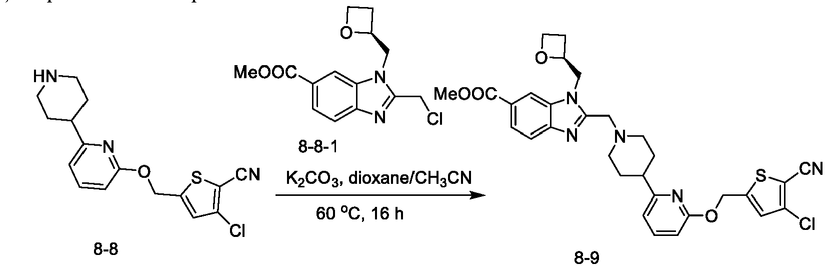

K₂CO₃ (58 mg, 0.42 mmol) was added to a mixture of 8-8 (35 mg, 0.10 mmol) and 8-8-1 (31 mg, 0.10 mmol) in dioxane (2 mL) and CH₃CN (1 mL). The mixture was stirred at 60°C for 16 hours. Water (5 mL) was added, and the solution was extracted with DCM (5 mL × 3). The combined organic phases were washed with brine (10 mL), dried (Na₂SO₄), filtered, and concentrated. After processed by flash chromatography (silica gel, eluted with 0-10% MeOH/DCM), 8-9 (34 mg, 54.8% yield) was given.

LC-MS: MC21-306-030-P, (ESI) m/z: 592.2 [M+H]⁺.

### (17) Preparation of Compound 8

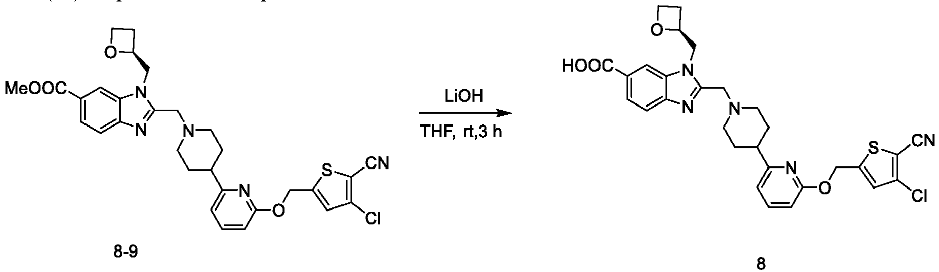

LiOH (2 mL) was added to a solution of 8-9 (34 mg, 0.06 mmol) in THF (2 mL). The mixture was stirred at room temperature for 3 hours, and then the solvent was removed under reduced pressure to give a crude product which was purified by HPLC (gradient: 10% MeCN/90% H₂O, 0.1% NH₃ H₂O to 100% MeCN) to give compound 8 (3.11 mg, 9.3% yield).

¹H NMR (400 MHz, DMSO-*d6*) δ 8.06 (s, 1H), 7.77 (*d*, *J* = 7.6 Hz, 1H), 7.72 - 7.64 (m, 1H), 7.50 (s, 1H), 7.42 (d, *J* = 8.2 Hz, 1H), 6.94 (*d*, *J* = 7.3 Hz, 1H), 6.70 (*d*, *J* = 8.2 Hz, 1H), 5.58 (s, 2H), 5.16 - 5.05 (m, 1H), 4.72 (dd, *J* = 15.3, 7.1 Hz, 1H), 4.60 (dd, *J* = 15.6, 3.2 Hz, 1H), 4.47 (dd, *J* = 13.7, 7.7 Hz, 1H), 4.36 (dt, *J* = 8.8, 5.9 Hz, 1H), 3.91 (d, *J* = 13.3 Hz, 1H), 3.77 (d, *J* = 13.3 Hz, 1H), 3.00 (d, *J* = 10.2 Hz, 1H), 2.90 (d, *J =* 11.4 Hz, 1H), 2.67 (dt, *J* = 13.4, 7.0 Hz, 2H), 2.43 (dd, *J* = 18.9, 7.9 Hz, 1H), 2.29 - 2.13 (m, 2H), 1.90 - 1.69 (m, 4H).

LC-MS: MC21-306-037-P (ESI) m/z: 578.0 [M+H]⁺.

### Example 9

### (S)-1-(oxetan-2-ylmethyl)-2-((4-(6-((5-(trifluoromethyl)thiophen-2-yl)methoxy)pyridin-2-yl)piperidin -1-yl)methyl)-1H-benzo[d]imidazole-6-carboxylic acid (Compound 9)

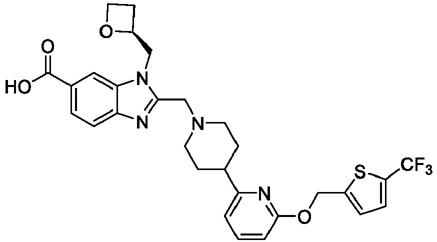

### (1) Preparation of Compound 9-2

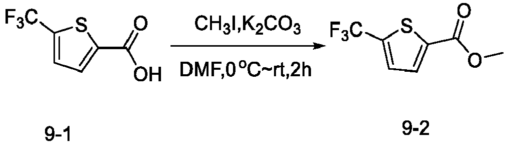

To a solution of 9-1 (2 g, 10.2 mmol) and K₂CO₃ (1.41 g, 10.2 mmol) in DMF (80 mL) was added dropwise CH₃I (2.895 g, 20.4 mmol) at 0°C under N₂. The reactants were stirred at room temperature for 2 hours and extracted with EA and H₂O (100 mL × 3). The combined organic layers were washed with brine, dried under anhydrous Na₂SO₄, and filtered. The residue was purified by column chromatography to give 9-2 (2 g, 95.2%).

¹H NMR (400 MHz, DMSO-*d*₆) δ 7.89-7.85 (m, 1H), 7.82 (d, J = 4.0 Hz, 1H), 3.90 (s, 3H).

### (2) Preparation of Compound 9-3

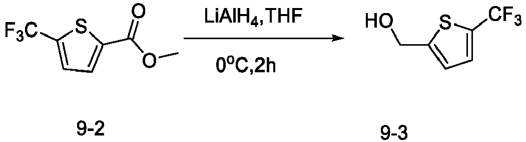

To a solution of 9-2 (2 g, 9.5 mmol) in THF (60 mL) was added dropwise LiAlH₄ (9.5 mL, 9.5 mmol) at 0°C under N₂. The reactants were stirred at room temperature for 2 hours. Afterwards, the reaction was quenched with sodium sulfate decahydrate. The solid was removed by filtration. The filtrate was concentrated to dryness to obtain a crude product. The residue was purified by column chromatography to give 9-3 (1.4 g, 82.4%).

¹H NMR (400 MHz, DMSO-*d₆*) δ 7.55-7.50 (m, 1H), 7.05-7.00 (m, 1H), 5.84-5.78 (m, 1H), 4.70 (d, J = 5.6 Hz, 2H).

### (3) Preparation of Compound 9-4

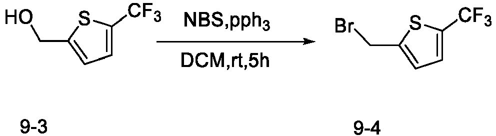

9-3 (1.4 g, 7.686 mmol) and a solution of pph3 (2.416 g, 9.22 mmol) and NBS (1.641 g, 9.22 mmol) in DCM (35 mL) were mixed. The mixture was stirred at room temperature for 5 hours. The mixture was diluted with DCM, and then extracted with DCM (20 mL × 3) and HzO. The crude residue was purified by SGC to give product 9-4 (1.3 g, 71.5%).

¹H NMR (400 MHz, DMSO-*d₆*) δ 7.58 (s, 1H), 7.37 - 7.30 (m, 1H), 5.06 (s, 2H).

### (4) Preparation of Compound 9-5

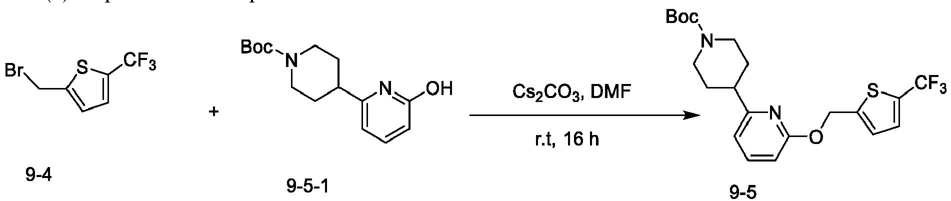

A mixture of 9-4 (220 mg, 0.898 mmol) and 9-5-1 (375 mg, 1.347 mmol) was added to the solvent DMF (10 ml) followed by addition of Cs₂CO₃ (878 mg, 2.693 mmol). The mixture was stirred in N₂ at room temperature for 12 hours. The mixture was extracted with H₂O (50 mL) and EA (100 mL × 3). The organic phase was washed with H₂O (100 mL × 3) and brine (100 mL), dried over Na₂SO₄, and concentrated. The residue was purified by column chromatography to give 9-5 (200 mg, 65.1%).

¹H NMR (400 MHz, DMSO-*d*₆) δ 7.70 - 7.63 (m, 1H), 7.60-7.57 (m, 1H), 7.30 (d, J = 2.6 Hz, 1H), 6.92 (d, J = 7.2 Hz, 1H), 6.68 (d, J = 8.0 Hz, 1H), 5.59 (s, 2H), 4.15 - 3.97 (m, 2H), 2.95-2.70 (m, 3H), 1.84 (s, 2H), 1.73 - 1.54 (m, 2H), 1.40 (s, 9H). 1HNMR (400 MHz, DMSO)

### (5) Preparation of Compound 9-6

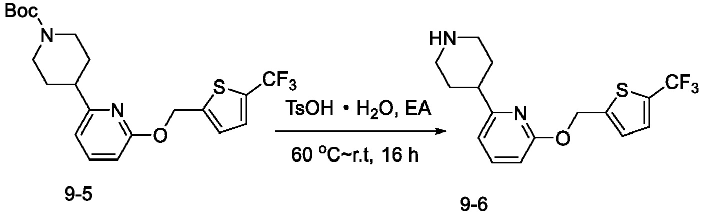

A mixture of 9-5 (200 mg, 0.0678 mmol) and TsOH · H₂O (255 mg, 0.204 mmol) was added to the solvent EA (4 ml). The mixture was stirred in N₂ at 60°C for 1 hour. The mixture was stirred in N₂ at room temperature for 16 hours. The mixture was filtered. The filter caked was washed with EA (5 mL * 3), and dried to give product 9-6 (132.2 mg).

LC-MS: (ESI) m/z: 343.0 [M+H]⁺.

### (6) Preparation of Compound 9-7

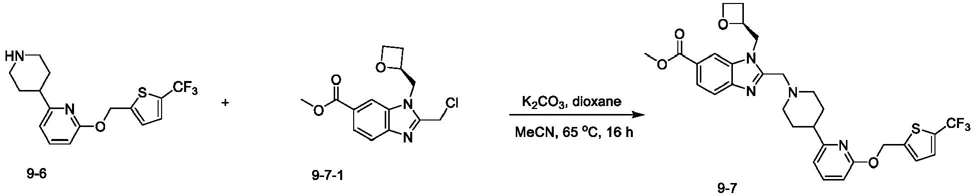

A mixture of 9-6 (132 mg, 0.380 mmol) and 9-7-1 (113.2 mg, 0.380 mmol) was added to the solvents dioxane (3 ml) and MeCN (2 ml) followed by addition of K₂CO₃ (425.3 mg). The mixture was stirred at 65°C for 12 hours. The reaction solution was extracted with EA (100 ml × 3) and H₂O (100 ml). The organic phase were washed with brine (100 ml), and dried over Na₂SO₄. The residue was purified by column chromatography to give 9-7 (140 mg, 65.1%).

LC-MS: (ESI) m/z: 601.1 [M+H]⁺.

### (7) Preparation of Compound 9

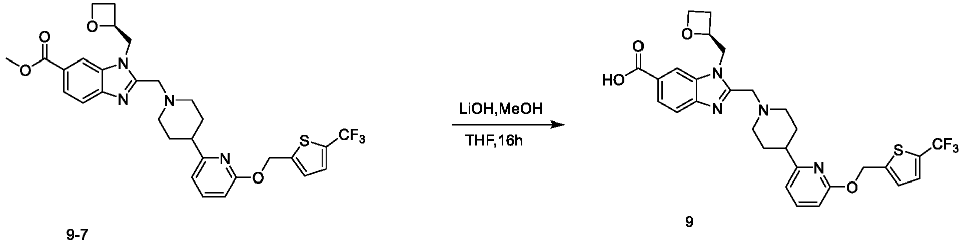

9-7 (140 mg, 0.233 mmol) and 1 M LiOH (1 mL) were added to MeOH (1 mL) and THF (1 ml). The mixture was stirred at room temperature for 16 hours. The crude product was purified by HPLC to give the title product, (S)-1-(oxetan-2-ylmethyl)-2-((4-(6-((5-(trifluoromethyl) thiophen-2-yl) methoxy)pyridin-2-yl) piperidin-1-yl)methyl)-1H-benzo[d]imidazole-6-carboxylic acid (Compound 9), 64.94 mg.

¹H NMR (400 MHz, DMSO-*d*₆) δ 8.06 (s, 1H), 7.76 (d, J = 8.2 Hz, 1H), 7.68 - 7.62 (m, 1H), 7.62 - 7.59 (m, 1H), 7.42 (d, J = 8.0 Hz, 1H), 7.32 (s, 1H), 6.92 (d, J = 7.2 Hz, 1H), 6.67 (d, J = 8.0 Hz, 1H), 5.60 (s, 2H), 5.10 (d, J = 4.2 Hz, 1H), 4.75-4.67 (m, 1H), 4.63 - 4.55 (m, 1H), 4.49-4.42 (m 1H), 4.40-4.32 (m, 1H), 3.91 (d, J = 13.4 Hz, 1H), 3.76 (d, J = 13.4 Hz, 1H), 3.01 (d, J = 11.0 Hz, 1H), 2.90 (d, J = 7.6 Hz, 1H), 2.71 - 2.61 (m, 2H), 2.47 - 2.38 (m, 1H), 2.30 - 2.12 (m, 2H), 1.80 (m, 4H).

LC-MS: MC21-238-081. (ESI) m/z: 587.3 [M+H]⁺.

### Example 10

### 1 -(oxetan-2-ylmethyl)-2-((4-(6-(thieno [2,3 -c]pyridin-2-ylmethoxy)pyridin-2-yl)piperidin-1 -yl)methyl) -1H-benzodimidazole-6-carboxlic acid (Compound 10)

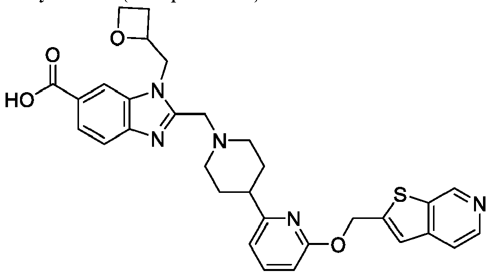

### (1) Preparation of Compound 10-2

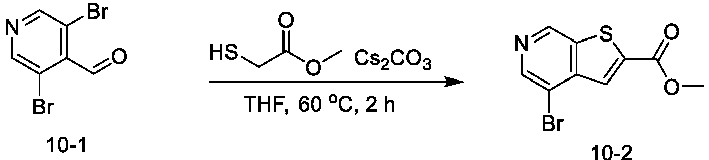

To a solution of 10-1 (1.0 g, 3.78 mmol) and Cs₂CO₃ (1.4 g, 4.16 mmol) in anhydrous THF (20 mL) was added methyl 2-mercaptoacetate (401 mg, 3.78 mmol) under nitrogen. The mixture was stirred at 60°C for 2 hours. After completion of the reaction, the mixture was filtered, concentrated, and extracted three times with EA. The combined organic layers were washed with water and brine, dried over anhydrous Na₂SO₄, filtered, and concentrated to give 10-2 (1.0 g, crude).

¹H NMR (400 MHz, CDCl₃) δ 9.08 (s, 1H), 8.65 (s, 1H), 8.16 (s, 1H), 4.01 (s, 3H).

LC-MS: (ESI) m/z: 273.9 [M+H]⁺.

### (2) Preparation of Compound 10-3

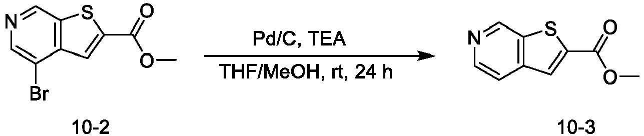

To a solution of 10-2 (1.0 g, 3.67 mmol) in anhydrous THF/MeOH (3:1, 20 mL) was added TEA (1.1 g, 11.01 mmol) and Pd/C (300 mg, 10% Pd). The mixture was stirred at 50°C under H₂ atmosphere for 24 hours. After completion of the reaction, the mixture was filtered, and concentrated to give 10-3 (825 mg, crude).

¹H NMR (400 MHz, CDCl₃) δ 9.21 (s, 1H), 8.57 (d, J = 5.5 Hz, 1H), 8.06 (s, 1H), 7.76 (d, J = 5.5 Hz, 1H), 3.98 (s, 3H).

LC-MS: (ESI) m/z: 194.2 [M+H]⁺.

### (3) Preparation of Compound 10-4

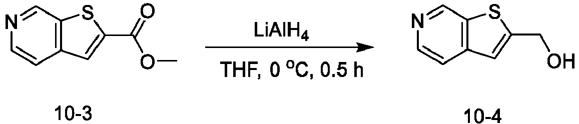

To a solution of 10-3 (2.5 g, 12.9 mmol) in anhydrous THF (25 mL) was added dropwise LiAlH₄ (13 mL, 1.0 M in THF) with stirring at 0°C. The mixture was stirred at the same temperature for 0.5 hour. After completion of the reaction, Na₂SO₄.10H₂O was added slowly to the reaction mixture, then filtered and concentrated. The resulting residue was purified by SGC (0-10% MeOH in DCM) to give 10-4 (410 mg, 19% yield).

¹H NMR (400 MHz, DMSO) δ 9.15 (s, 1H), 8.42 (d, J = 5.4 Hz, 1H), 7.74 (dd, J = 5.4, 0.9 Hz, 1H), 7.35 (d, J = 0.8 Hz, 1H), 5.85 (t, J = 5.8 Hz, 1H), 4.82 (dd, J = 5.8, 1.1 Hz, 2H).

LC-MS: (ESI) m/z: 166.2 [M+H]⁺.

### (4) Preparation of Compound 10-5

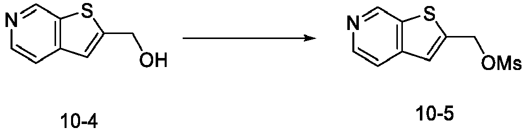

To a solution of 10-4 (100 mg, 0.6 mmol) in anhydrous DCM (5 mL) was added MsCl (83 mg, 0.72 mmol) and TEA (0.84 mL, 6.05 mmol). The reaction mixture was stirred at 0°C for 30 minutes, and then quenched with water. After extraction with DCM, the organic layer was washed with brine, dried over anhydrous Na₂SO₄, and concentrated in vacuo to give 147 mg of crude 10-5.

### (5) Preparation of Compound 10-6

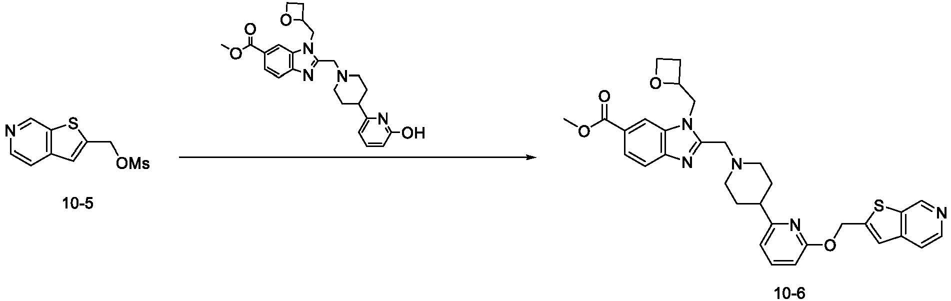

A mixture of 10-5 (147 mg, 0.6 mmol), methyl 2-((4-(6-hydroxypyridin-2-yl)piperidin-1-yl) methyl)-1-(oxetan-2-ylmethyl)-1H-benzo[d]imidazole-6-carboxylate (50 mg, 0.12 mmol), and Cs₂CO₃ (217 mg, 0.66 mmol) in DMF (10 ml) was stirred overnight at 50°C. The reaction was quenched with H₂O (10 ml). After extraction with DCM (10 ml×3), the organic layer was washed with brine, dried over anhydrous Na₂SO₄, and concentrated in vacuo to obtain a residue. The crude product was purified by HPLC (gradient: 10% MeCN/90% H₂O, H₂O to 100% MeCN) to give 10- 6 (30 mg).

LC-MS: MC21-231-109-P, (ESI) m/z: 584.2 [M+H]⁺.

### (6) Preparation of Compound 10

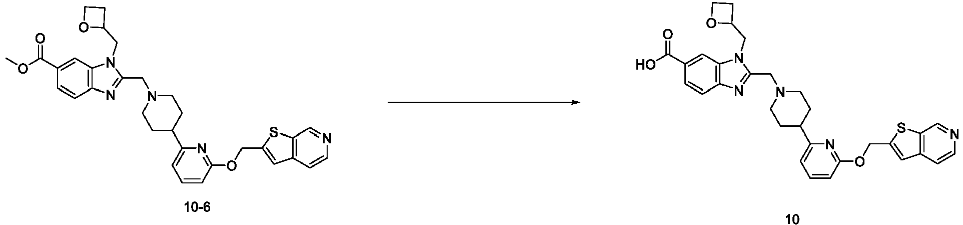

A solution of 10-6 (30 mg, 0.05 mmol) and LiOH (0.5 mL) in THF (0.5 mL) was stirred at room temperature for 2 hours. The solvent was then removed under reduced pressure to give a crude product which was purified by HPLC (gradient: 10% MeCN/90% H₂O, 0.1% NH_{3·}H₂O to 100% MeCN) to give 1.62 mg of compound 10.

¹H NMR (400 MHz, DMSO) δ 9.16 (s, 1H), 8.41 (d, *J* = 5.4 Hz, 1H), 8.06 (s, 1H), 7.76 (d, *J* = 6.5 Hz, 2H), 7.69 - 7.64 (m, 1H), 7.59 (s, 1H), 7.41 (d, *J* = 8.3 Hz, 1H), 6.92 (d, *J* = 7.3 Hz, 1H), 6.71 (d, *J* = 8.2 Hz, 1H), 5.74 (s, 2H), 5.11 (m, *J* = 6.9, 3.2 Hz, 1H), 4.70 (dd, *J* = 15.2, 6.8 Hz, 1H), 4.58 (dd, *J* = 15.1, 3.1 Hz, 1H), 4.47 - 4.31 (m, 2H), 3.90 (d, *J* = 13.4 Hz, 1H), 3.75 (d, *J* = 13.4 Hz, 1H), 3.00 (d, *J* = 11.0 Hz, 1H), 2.89 (d, *J* = 11.4 Hz, 1H), 2.69 - 2.62 (m, 2H), 2.42 (dd, *J* = 11.1, 4.1 Hz, 1H), 2.27 - 2.13 (m, 2H), 1.90 - 1.72 (m, 4H).

LC-MS: MC21-231-114-P, (ESI) m/z: 570.2 [M+H]⁺.

### Example 11

### (S)-2-((4-(6-((5-cyano-3-methylthiophen-2-yl)methoxy)pyridin-2-yl)piperidin-1-yl)methyl)-1-(oxetan-2-ylmethyl)-1H-benzo[d]imidazole-6-carboxylic acid (Compound 11)

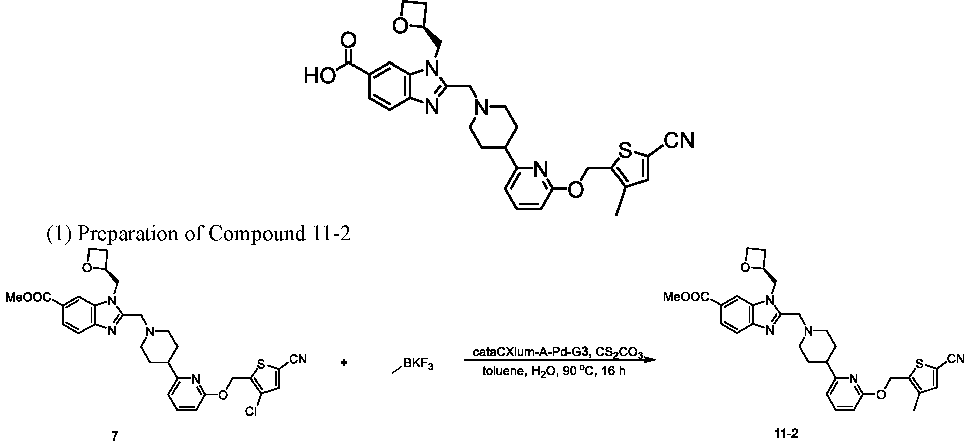

Compound 7 (60 mg, 0.101 mmol) and potassium trifluoro(methyl)borate (124 mg, 1.015 mmol) were added to the solvents toluene (20 mL) and H₂O (2 mL), followed by addition of cataCXium-A-Pd-G3 (4 mg, 0.005 mmol) and Cs₂CO₃ (100 mg, 0.304 mmol). The reaction solution was stirred at 90°C for 16 hours. H₂O (20 mL) was added, and the reaction solution was extracted with EtOAc (3 × 20 mL). The combined organic phases were washed with brine (20 mL), dried (Na₂SO₄), filtered, and concentrated. After purification by flash chromatography (SiO₂, EtOAc-hexane), 30 mg of compound 11-2 was given with a yield of 51.7%.

¹H NMR (400 MHz, CDCl₃) δ 8.10 (d, *J* = 1.1 Hz, 1H), 7.90 (dd, *J* = 8.5, 1.5 Hz, 1H), 7.68 (d, *J* = 8.5 Hz, 1H), 7.45 (dd, *J* = 8.1, 7.5 Hz, 1H), 7.27 (s, 1H), 6.70 (d, *J* = 7.3 Hz, 1H), 6.51 (d, *J* = 8.0 Hz, 1H), 5.42 (s, 2H), 5.23 (s, 1H), 5.17 (m, 1H), 4.67 (m, 2H), 4.55 (td, *J* = 7.9, 6.1 Hz, 1H), 4.33 (dt, *J* = 9.1, 5.9 Hz, 1H), 3.92 (s, 2H), 3.88 (s, 3H), 3.41 (s, 1H), 2.94 (t, *J* = 13.0 Hz, 2H), 2.73 - 2.54 (m, 2H), 2.43 - 2.36 (m, 1H), 2.25 (s, 3H), 1.95 (d, *J* = 4.0 Hz, 4H).

### (2) Preparation of Compound 11

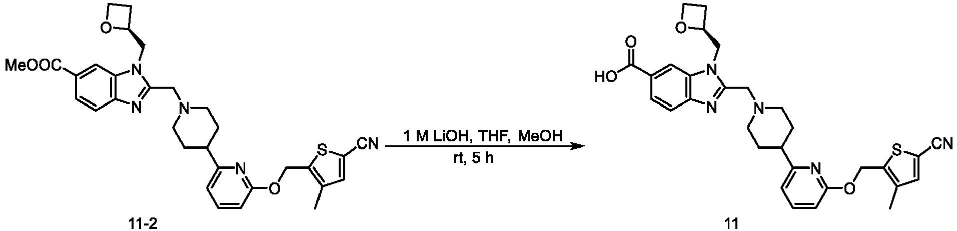

To a solution of 11-2 (30 mg, 0.05 mmol) in THF (5 mL) was added 1 M LiOH (0.5 mL). The reaction solution was stirred at room temperature for 3 hours, concentrated, and purified by preparative HPLC to give 1 mg of compound 11, yield: 3.4%.

¹H NMR (400 MHz, DMSO-J6) δ 8.32 (s, 1H), 8.24 (s, 1H), 7.81 (d, *J* = 8.3 Hz, 1H), 7.74 (s, 1H), 7.67 - 7.59 (m, 2H), 6.91 (d, *J* = 7.4 Hz, 1H), 6.67 (d, *J* = 8.2 Hz, 1H), 5.55 (s, 2H), 5.11 (q, *J* = 8.5 Hz, 1H), 4.80 (dd, *J* = 15.4, 6.9 Hz, 1H), 4.66 (d, *J* = 13.3 Hz, 1H), 4.47 (dd, *J* = 13.6, 7.4 Hz, 1H), 4.37 (m, 1H), 3.95 (d, *J* = 13.5 Hz, 1H), 3.79 (d, *J* = 13.4 Hz, 1H), 3.00 (s, 1H), 2.89 (s, 1H), 2.74 - 2.60 (m, 4H), 2.46 (s, 1H), 2.29 (s, 3H), 1.84 - 1.71 (m, 4H).

LC-MS: (ESI) m/z: 558.2 [M+H]⁺.

### Example 12

### (S)-2-((2-((5-cyanothiophen-2-yl)methoxy)-5, 8,10,11-tetrahydro-oxepino [4,3-b:6,5-c'] dipyridin-9(7H) -yl)methyl)-1-(oxetan-2-ylmethyl)-1H-benzo[d]imidazole-6-carboxyfic acid (Compound 12)

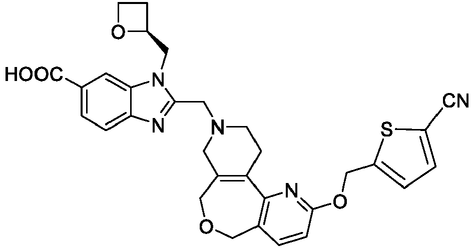

The preparation scheme for the intermediate 2-hydroxy-5,8,10,11-tetrahydro-oxepino[4,3-b:6,5-c']dipyridine-9(7H)-formate (12-i) is as follows:

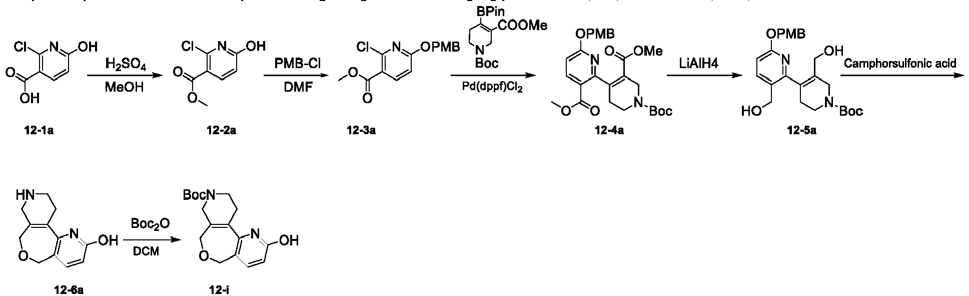

To a mixture of 2-chloro-6-hydroxynicotinic acid 12-1a (5.0 g, 29 mmol) in MeOH (40 mL) was added sulfuric acid (10 mL). The mixture was stirred at 80°C for 16 hours. The reaction mixture was quenched with ice water, and extracted with EA. The combined organic layers were washed with brine (50 mL), dried over Na₂SO₄, and concentrated under reduced pressure to give methyl 2-chloro-6-hydroxynicotinate, 12-2a (4.5 g, 83% yield).

A reaction mixture of methyl 2-chloro-6-hydroxynicotinate 12-2a (3.4 g, 18.2 mmol), PMB-Cl (3.4 g, 21.2 mmol) and K₂CO₃ (3.76 g, 27.3 mmol) in DMF (50 mL) was added. The mixture was stirred with Ar₂ at 80°C for 2 hours. The reaction of the reaction mixture was complete as detected by LC-MS. The reaction mixture was quenched by addition of water. The aqueous phase was extracted with EtOAc (100 mL × 3), and washed with brine. The combined organic layers were dried over Na₂SO₄, concentrated, and purified by elution (PE/EA = 0-20%) to give methyl 2-chloro-6-((4-methoxybenzyl)oxy)nicotinate 12-3a (2.9 g, 52% yield).

A reaction mixture of methyl 2-chloro-6-((4-methoxybenzyl)oxy)nicotinate 12-3a (2.9 g, 9.4 mmol), 4-(4,4,5,5-tetramethyl-1,3,2-dioxaborolan-2-yl)-5,6-dihydropyridine-1,3(2H)-dicarboxylate 1-4 methyl ester (4.16 g, 11.3 mmol), Cs₂CO₃ (6.16 g, 18.8 mmol), and Pd(dppf)Cl₂ (0.69 g, 0.94 mmol) in 1,4-dioxane (80 mL) was added. The mixture was stirred with Ar₂ at 110°C for 16 hours. The reaction of the reaction mixture was complete as detected by LC-MS. The reaction mixture was concentrated, and purified by elution (PE/EA = 0-20%) to give 1'-(t-butyl) 3,3'-dimethyl-6-((4-methoxybenzyl)oxy)-5',6'-dihydro-[2,4'-bipyridine]-1',3,3'(2'H)-tricarboxylate 12-4a (2.5 g, 53.2% yield).

12-4a (1.5 g, 2.9 mmol) was dissolved in anhydrous THF (15 mL), and then LiAlH₄ (0.222 g, 5.8 mmol) was added in portions at 0°C. After 10 minutes, the reaction of the reaction mixture was complete as detected by LC-MS. The reaction mixture was quenched with ice water (0.5 mL). The mixture was filtered, and concentrated to give tert-butyl 3,5'-bis(hydroxymethyl)-6-((4-methoxybenzyl)oxy)-3',6'-dihydro-[2,4' -bipyridyl]-1'(2'H)-carboxylate 12-5a (1.2 g, 92% yield).

A reaction mixture of 12-5a (1.1 g, 2.4 mmol) and camphorsulfonic acid (3.24 g, 9.6 mmol) in TOL (20 mL) was added. The mixture was heated at 110°C for 1 hour. The reaction of the reaction mixture was complete as detected by LC-MS. The reaction mixture was concentrated, and purified by elution (MeOH/DCM = 0-20%) to give 5,7,8,9,10,11-hexahydroxy-oxepino[4,3-b:6,5-c']bipyridin-2-ol 12-6a (0.4 g, 76% yield).

To a solution of 12-6a (0.35 g, 1.6 mmol) in DCM (15 mL) was added (Boc)₂O (0.42 g, 1.9 mmol) and TEA (0.2 mL). The mixture was stirred at room temperature for 1 hour. The reaction of the reaction mixture was complete as detected by LC-MS. The reaction mixture was concentrated, and purified by elution (MeOH/DCM = 0-10%) to give 2-hydroxy-5,8,10,11-tetrahydro-oxepino[4,3-b:6,5-c']dipyridine-9(7H)-formate 12-i (350 mg, 70% yield). ¹H NMR (400 MHz, CDCl₃) δ 12.63 (s, 1H), 7.43 (d, *J=* 9.2 Hz, 1H), 6.49 (d, *J* = 9.1 Hz, 1H), 4.17 (s, 4H), 3.80 (s, 2H), 3.67 (t, *J* = 5.5 Hz, 2H), 2.72 (s, 2H), 1.51 (s, 9H).

### (1) Preparation of Compound 12-1

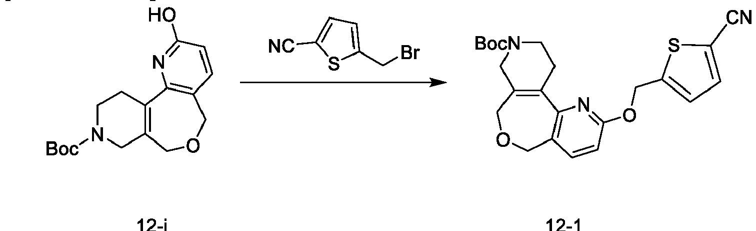

NaH (32.7 mg, 0.85 mmol) was added in portions to 12-i (200 mg, 0.65 mmol). After stirring the reaction mixture for 5 minutes, 5-(bromomethyl)thiophene-2-carbonitrile (131.5 mg, 0.65 mmol) was added, and stirred at room temperature for 10 minutes. The reaction mixture was quenched by the addition of H₂O. The aqueous phase was extracted with EtOAc (30 mL × 3), and washed with brine (30 mL × 2). The combined organic layers were dried over Na₂SO4, and concentrated. The residue was purified by elution to give 12-1 (210 mg, 71.7% yield).

LCMS: r.t. = 2.189 min, [M+H] + =440.

### (2) Preparation of Compound 12-2

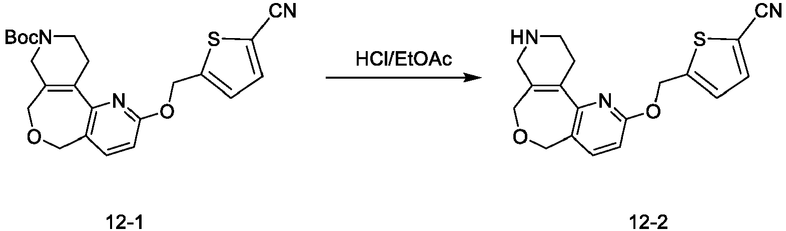

A solution of 12-1 (200 mg, 0.47 mmol) in EtOAc/HCl (10 mL) was mixed. The mixture was stirred at room temperature for 2 hours. The reaction of the reaction mixture was complete as detected by LC-MS. The reaction solution was concentrated to give 12-2 (200 mg, crude).

LCMS: r.t. = 1.391 min, [M+H] + = 340.

### (3) Preparation of Compound 12-3

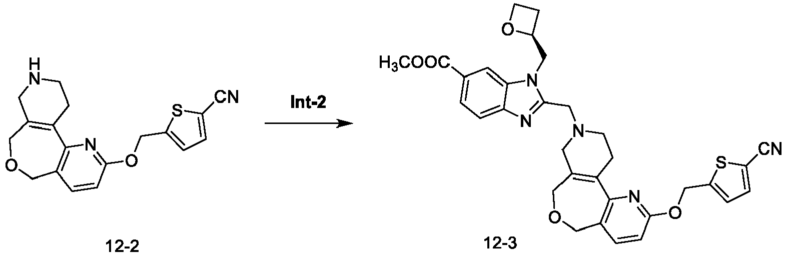

A reaction mixture of 12-2 (200 mg, 0.589 mmol) and DIEA(117.23 mg, 0.531 mmol) in 10 mL CH₃CN was stirred at room temperature for 10 minutes. Int-2 (111.5 mg, 0.358 mmol) was then added. The reaction mixture was concentrated by heating at 60°C for 12 hours, and purified by elution (MeOH/DCM = 0-5%) to give 12-3 (160 mg, 45% yield).

LCMS: r.t=1.388 min, [M+H] + =598.

### (4) Preparation of Compound 12

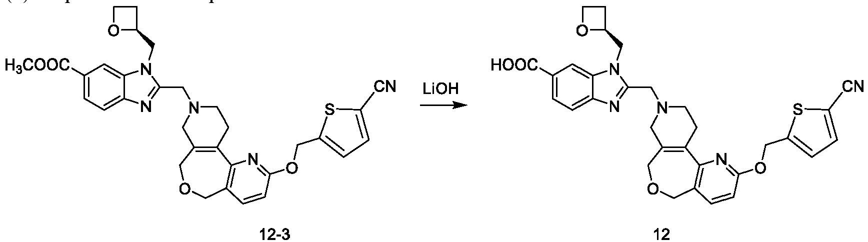

To a mixture of 12-3 (160 mg, 0.27 mmol) in THF (5 mL) was added lithium hydroxide solution (1 N, 5 mL). The mixture was stirred at room temperature for 2 hours. The mixture was concentrated, purified by pre-HPLC, and lyophilized to give compound 12 (19.65 mg, 12.58% yield).

LCMS: r.t=1.224 min, [M+H]+ =584.

¹H NMR (400 MHz, MeOD) δ 8.18 (s, 1H), 7.95 (dd, *J* = 8.4, 1.5 Hz, 1H), 7.65 - 7.57 (m, 3H), 7.23 (d, *J* = 3.8 Hz, 1H), 6.70 (d, *J* = 8.2 Hz, 1H), 5.64 (s, 2H), 5.27 (d, *J* = 6.7 Hz, 1H), 4.72 (dd, *J* = 15.3, 3.0 Hz, 3H), 4.61 (dd, *J* = 13.7, 7.9 Hz, 2H), 4.46 (dd, *J* = 5.9, 3.1 Hz, 1H), 4.37 (s, 2H), 4.12 (dd, *J* = 34.0, 13.6 Hz, 2H), 3.90 (s, 2H), 2.85 (s, 4H), 2.75 (d, *J* = 6.5 Hz, 1H), 2.57 - 2.49 (m, 1H).

### Example 13

### (S)-1-(oxetan-2-ylmethyl)-2-(4-(6-((5-(pyrimidin-5-yl)thiophen-2-yl)methoxy)pyridin-2-yl)piperidin-1-yl)methyl)-1H-benzo[d]imidazole-6-carboxylic acid (Compound 13)

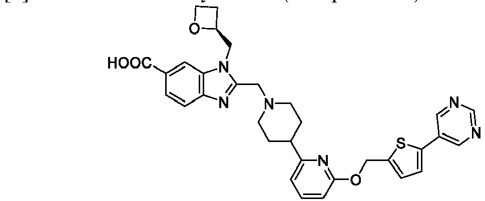

### (1) Preparation of Compound 13-2

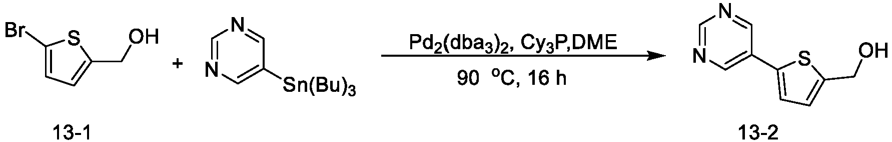

A mixture of 13-1 (871 mg, 4.52 mmol), 5-(tributylstannyl)pyrimidine (2.2 mL, 6.77 mmol), Pd₂(dbas)₂ (828 mg, 0.90 mmol) and Cy₃P (253 mg, 0.90 mmol) in dry DME (20 mL) was stirred at 90°C for 16 hours. The reaction solution was extracted with DCM/MeOH (3 × 20 mL). The combined organic phases were washed with brine (20 mL), dried (Na₂SO₄), filtered, and concentrated. After processed by flash chromatography (SiO₂, EtOAc-hexane), 360 mg of compound 13-2 was given.

¹H NMR (400 MHz, CDCl₃) 9.09 (1 H, s), 9.08 (2 H, s), 7.61 (1 H, d, *J* =3.6), 7.06 (1 H, d, *J* =3.7), 5.64 (1 H, t, *J* =5.7), 4.69 (2 H, d, *J* =5.7).

LC-MS: (ESI) m/z: 193 [M+H]⁺.

### (2) Preparation of Compound 13-3

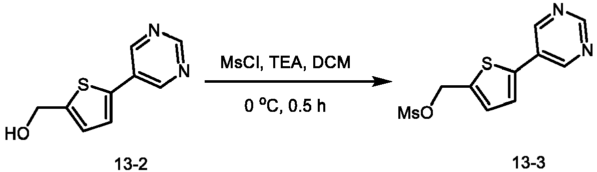

To a solution of 13-2 (360 mg, 1.87 mmol) in anhydrous DCM (5 mL) was added MsCl (257 mg, 2.24 mmol) and TEA (2.6 mL, 18.7 mmol). The reaction mixture was stirred at 0°C for 30 minutes, and then quenched with water. After extraction with DCM, the organic layer was washed with brine, dried over anhydrous Na₂SO₄, and concentrated in vacuo to give 13-3 (500 mg, crude).

### (3) Preparation of Compound 13-4

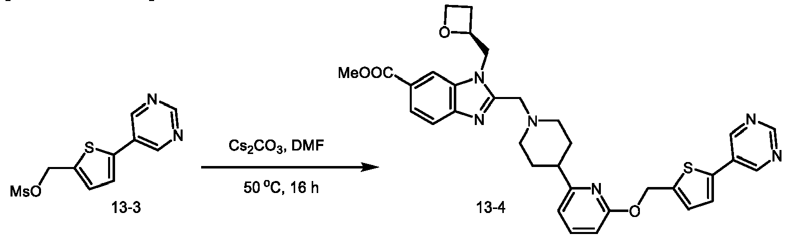

A mixture of 13-3 (500 mg, 1.84 mmol) and Cs₂CO₃ (663 mg, 2.03 mmol) in DMF (10 ml) was stirred overnight at 50°C. The reaction was quenched with H₂O (10 ml). After extraction with DCM (10 ml × 3), the organic layer was washed with brine, dried over anhydrous Na₂SO₄, and concentrated in vacuo to give a residue. The crude product was purified by HPLC (gradient: 10% MeCN/90% H₂O, H₂O to 100% MeCN) to give 194 mg of compound 13-4.

LC-MS: MC21-663-034-P, (ESI) m/z: 611.4 [M+H]⁺.

### (4) Preparation of Compound 13

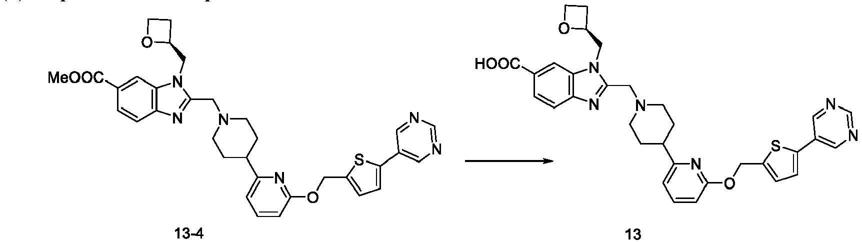

0.5 mL of a solution of 13-4 (197 mg, 0.32 mmol) and LiOH in THF (0.5 mL) was stirred at room temperature for 2 hours. The solvent was then removed under reduced pressure to give a crude product which was purified by HPLC (gradient: 10% MeCN/90% H₂O, 0.1% NH₃·H₂O to 100% MeCN) to give 64.79 mg of compound 13.

LC-MS: MC21-663-037-P, (ESI) m/z: 597.3 [M+H]⁺.

¹H NMR (400 MHz, DMSO) δ 9.09 (s, 1H), 9.06 (s, 2H), 8.26 (s, 1H), 7.81 (dd, *J* = 8.0Hz, 1H), 7.65 (m, 3H), 7.32 (d, *J =* 4.0 Hz, 1H), 6.91 (d, *J* = 8.0 Hz, 1H), 6.66 (d, *J* = 8.0 Hz, 1H), 5.67 - 5.54 (m, 2H), 5.11 (m,1H), 4.79 (dd, *J* ₁= 8.0 Hz, *J* ₂= 16.0 Hz 1H), 4.65 (dd, *J* = 12.0 Hz, 1H), 4.44 (m, 1H), 4.36 (m, 1H), 3.97 (d, *J* = 16.0 Hz, 1H), 3.82 (d, *J* = 16.0 Hz, 1H), 3.04 (d, *J* = 12.0 Hz, 1H), 2.89 (d, *J* = 12.0 Hz, 1H), 2.69 (m, 2H), 2.42 (m, 1H), 2.31 - 2.15 (m, 2H), 1.96 - 1.72 (m, 4H).

The GLP-1 receptor agonist provided by the invention and the use thereof are described in detail above. Specific examples are used herein to illustrate the principles and embodiments of the invention, and the foregoing description of the examples is made only for the purpose of facilitating an understanding of the method of the invention and its underlying concepts. It should be noted that those of ordinary skill in the art may make numerous changes and modifications to the invention without departing from the principles of the invention, and such changes and modifications are also under protection by the following claims.

## Claims

1. A compound of Formula I 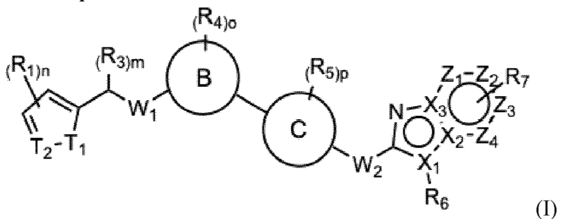
and a pharmaceutically acceptable salt thereof,
wherein
T₁ and T₂ are each independently selected from the group consisting of CH₂, NH, O, and S;
W₁ is selected from the group consisting of O, S, CH₂, and NH;
W₂ is selected from the group consisting of O, NH, CH₂, and CR_{y};
Z₁, Z₂, Z₃, and Z₄ are each independently selected from the group consisting of CH, N, or C;
X₁, X₂, and X₃ are each independently selected from the group consisting of CH, N, or C, and at most two of X₁, X₂, and X₃ are N;
ring B is selected from the group consisting of benzene ring or 5- to 7-membered heteroaromatic ring;
ring C is selected from the group consisting of benzene ring, 4 to 8-membered heterocyclic ring, 5 to 10-membered spiro ring, 5 to 10-membered bridged ring, and 5- to 7-membered heteroaromatic ring;
R₁ is independently selected from the group consisting of R₂, -carbonyl-R₂, -carbonyl-amino-R₂, -sulphonyl-R₂, -amido-R₂, -phosphoroso-Rz, -amino-R₂, and -O-R₂, wherein the R₂, amino, amido, sulphonyl, and phosphoroso in R₁ may be optionally substituted 1 to 3 times by asubstituent(s) independently selected from Rₓ;
R₂ is independently selected from the group consisting of hydrogen, oxo, halogen, -C₁₋₆ alkyl, -C₂₋₆ alkenyl, -C₂₋₆ alkynyl, -C₁₋₆ alkoxy, -C₁₋₆ cycloalkoxy, cyano, 3- to 8-membered cycloalkyl, 3- to 8-membered heterocyclyl, phenyl, and 5- to 8-membered heteroaryl, wherein the halogen, alkyl, alkenyl, alkynyl, amino, alkoxy, cycloalkoxy, cycloalkyl, heterocyclyl, phenyl, and heteroaryl in R₂ may be optionally substituted 1 to 3 times by a substituent(s) independently selected from Rₓ;
R₃ is independently selected from the group consisting of hydrogen, oxo, halogen, -CN, -C₁₋₆ alkyl, -C₂₋₆ alkenyl, -C₂₋₆ alkynyl, -C₁₋₆ alkoxy, amino, amido, sulfonyl, sulfonamido, -OH, -C₃₋₈ cycloalkyl, 3- to 8 membered heterocyclyl, 6- to 10 membered aryl, and 5- to 8 membered heteroaryl, wherein R₃ may be optionally substituted 1 to 3 times by a substituent(s) independently selected from R_{y}, where valency permits;
R₄ is independently selected from the group consisting of hydrogen, halogen, -C₁₋₃ alkyl, -C₁₋₃ haloalkyl, -C₁₋₃ alkoxy, cyano, hydroxyl, amino, amido, sulfonyl, and sulfonamido;
R₅ is independently selected from the group consisting of hydrogen, halogen, hydroxy, -CN, -C₁₋₃ alkyl, -C₁₋₃ alkoxy, and -C₁₋₃ cycloalkyl, wherein the alkyl, alkoxy, and cycloalkyl in R₅ may be optionally substituted 1 to 3 times by halogen, hydroxyl, -NR_{z}, -CN, -C₁₋₃ alkyl, -C₁₋₃ alkoxy, -C₁₋₃ cycloalkyl, where valency permits;
R₆ is selected from the group consisting of -R_{z}, -O-R_{z}, -S-R_{z}, -C₁₋₃ alkyl, -C₁₋₃ alkylene-R_{z}, -C₀₋₃ alkylene-amino-R_{z}, -C₀₋₃ alkylene-carbonyl-R_{z}, -C₀₋₃ alkylene-amido-R_{z}, -C₀₋₃ alkylene-sulfonyl-R_{z}, -C₀₋₃ alkylene-phosphoryl-R_{z}, and -C₀₋₃ alkylene-sulfonamido-R_{z}, wherein the alkyl, amino, amido, sulfonyl, sulfonamido, and phosphoryl in R₆ may be optionally substituted 1 to 3 times by halogen or one time by R_{w}, where valency permits;
R₇ is selected from the group consisting of -COOH, -C(R_{y})ₙ₀-COOH, -N(R_{z})ₙ₀-COOH, -SOz-COOH, and -SO₂-NH-COOH, wherein the R_{y} in -C(R_{y})ₙ₀- may be attached to C in the form of a backbone and/or a branched chain, the R_{z} in -N(R_{z})ₙ₀- may be attached to N in the form of a backbone and/or a side chain, wherein n₀ is an integer selected from 0, 1 or 2; when n₀ is 2, two R_{y} or R_{z} may be further cyclized to form a 3- to 8-membered carbocyclic or heterocyclic ring;
n is an integer selected from 0, 1, 2 or 3;
m is an integer selected from 0, 1 or 2;
o is an integer selected from 0, 1, 2, 3 or 4;
p is an integer selected from 0, 1, 2, 3 or 4;
when m is 2, two R₃ may be further cyclized into a 3- to 8-membered carbocyclic ring or heterocyclic ring;
when m is 1 or 2, R₁ and R₃ may be further cyclized into a 3- to 8-membered carbocyclic ring or heterocyclic ring;
when n is more than or equal to 2, any two R₁ may be further cyclized into a 3- to 8-membered carbocyclic ring, aromatic ring, heterocyclic ring or heteroaromatic ring, wherein the formed carbocyclic ring and heterocyclic ring may be optionally substituted 1 to 3 times by C₁₋₃ alkyl, C₁₋₃ haloalkyl, halogen, cyano, C₁₋₃ alkoxy, where valency permits;
when p is greater than or equal to 2, any two R₅ may be further cyclized with the ring C to form a 6- to 10-membered spiro ring or bridged ring, wherein the formed spiro ring and bridged ring may be optionally substituted 1 to 3 times by C₁₋₃ alkyl, C₁₋₃ haloalkyl, halogen, cyano, or C₁₋₃ alkoxy where valency permits;
when o is not 0 and p is not 0, any R₄ and R₅ may be further cyclized to form a 5- to 8-membered ring, wherein the formed ring may be optionally substituted 1 to 3 times by C₁₋₃ alkyl, C₁₋₃ haloalkyl, halogen, cyano, oxo, C₁₋₃ alkoxy, where valence permits;
R_{w} is independently selected from the group consisting of -CN, -CH₂CN, -C₁₋₃ alkyl, -OH, -C₁₋₃ alkoxy, amido, sulfonyl, sulfonamido, -NH₂, and -NH-C₁₋₃ alkyl, wherein the alkyl in R_{w} may be optionally substituted 1 to 3 times by C₁₋₃ alkyl, C₁₋₃ haloalkyl, halogen, cyano, oxo, C₁₋₃ alkoxy, where valence permits;
Rₓ is independently selected from the group consisting of hydrogen, halogen, oxo, C₁₋₆ alkoxy, cyano, hydroxyl, carboxyl, amino, amido, sulfonyl, sulfonamido, -C₁₋₆ alkyl, -C₂₋₆ alkenyl, -C₂₋₆ alkynyl, -C₃₋₆ cycloalkyl, 3- to 6-membered heterocyclyl, 6- to 8-membered aryl, and 5- to 8-membered heteroaryl, wherein the alkyl, alkoxy, cycloalkyl, heterocyclyl, aryl, and heteroaryl in Rₓ may be optionally substituted 1 to 3 times by C₁₋₃ alkyl, C₁₋₃ haloalkyl, halogen, cyano, oxo, C₁₋₃ alkoxy, or one time by hydroxy, where valency permits;
R_{y} is independently selected from the group consisting of hydrogen, halogen, oxo, -C₁₋₃ alkoxy, cyano, hydroxyl, amino, carboxyl, amido, sulfonyl, sulfonamido, -C₁₋₆ alkyl, -C₂₋₆ alkenyl, -C₂₋₆ alkynyl, C₃₋₆ cycloalkyl, 3- to 6-membered heterocyclyl, and 5- to 6-membered heteroaryl, wherein the alkyl, alkenyl, alkynyl, amido, amido, alkoxy, cycloalkyl, heterocyclyl and heteroaryl in R_{y} may be optionally substituted 1 to 3 times by C₁₋₃ alkyl, C₁₋₃ haloalkyl, halogen, cyano, oxo, C₁₋₃ alkoxy, where valence permits; and
R_{z} is independently selected from the group consisting of hydrogen, C₁₋₃ alkyl, C₁₋₃ alkoxy, C₃₋₆ cycloalkyl, 3- to 6-membered heterocyclyl, aryl, and 5- to 6-membered heteroaryl, wherein R_{z} may be optionally substituted 1 to 3 times by C₁₋₃ alkyl, C₁₋₃ haloalkyl, halogen, cyano, oxo, C₁₋₃ alkoxy, 3- to 6-member heterocyclyl, where valence permits.

2. The compound of Formula I according to claim 1, wherein the ring B is selected from the group consisting of 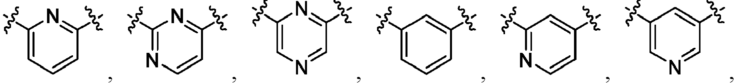 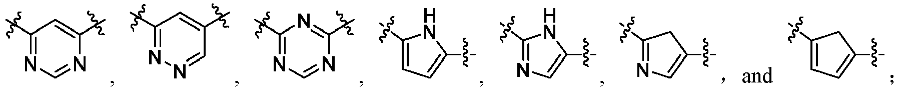
wherein the ring B is preferably and/or, 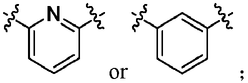
the ring C is selected from the group consisting of 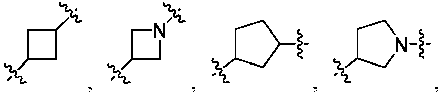 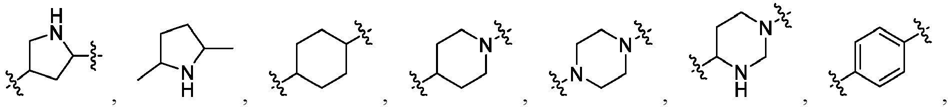 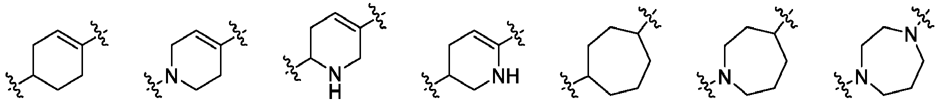 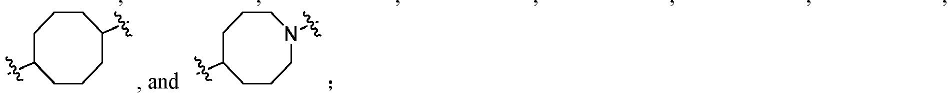 wherein the ring C is preferably 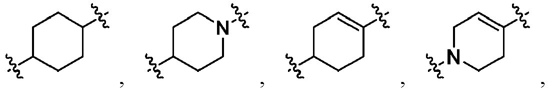 or

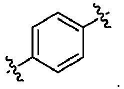

3. The compound of Formula I according to claim 1 which is a compound of Formula I-2 or Formula 1-2': 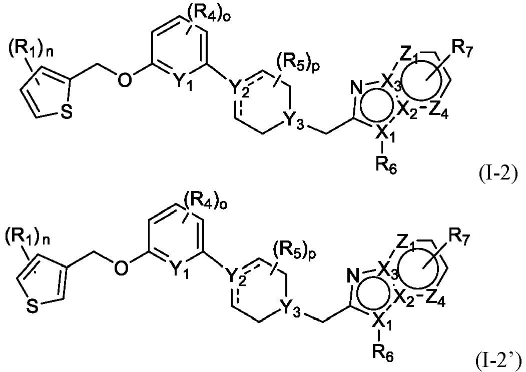
and a pharmaceutically acceptable salt thereof,
wherein, further,
------ represents the presence or absence of a chemical bond;
Z₁ and Z₄ are each independently selected from the group consisting of CH and N;
X₁, X₂ and X₃ are each independently selected from the group consisting of CH, N or C, and at most two of X₁, X₂ and X₃ are N;
Y₁ is selected from the group consisting of CH or N;
Y₂ is selected from the group consisting of CH, N or C;
Y₃ is selected from the group consisting of CH or N;
R₁ is independently selected from the group consisting of R₂, -carbonyl-R₂, -carbonyl-amino-R₂, -sulphonyl-R₂, -amido-R₂, -phosphoroso-R₂, -amino-R₂, and -O-R₂, wherein the R₂, amino, amido, sulphonyl, and phosphoroso in R₁ may be optionally substituted 1 to 3 times by a substituent(s) independently selected from Rₓ;
R₂ is independently selected from the group consisting of hydrogen, oxo, halogen, -C₁₋₆ alkyl, -C₂₋₆ alkenyl, -C₂₋₆ alkynyl, -C₁₋₆ alkoxy, -C₁₋₆ cycloalkoxy, cyano, 3- to 8-membered cycloalkyl, 3- to 8-membered heterocyclyl, phenyl, and 5- to 8-membered heteroaryl, wherein the halogen, alkyl, alkenyl, alkynyl, amino, alkoxy, cycloalkoxy, cycloalkyl, heterocyclyl, phenyl, and heteroaryl in R_{z} may be optionally substituted 1 to 3 times by a substituent(s) independently selected from Rₓ;
R₃ is independently selected from the group consisting of hydrogen, oxo, halogen, -CN, -C₁₋₆ alkyl, -C₂₋₆ alkenyl, -C₂₋₆ alkynyl, -C₁₋₆ alkoxy, amino, amido, sulfonyl, sulfonamido, -OH, -C₃₋₈ cycloalkyl, 3- to 8-membered heterocyclyl, 6- to 10-membered aryl, and 5- to 8-membered heteroaryl, wherein R₃ may be optionally substituted 1 to 3 times by a substituent(s) independently selected from R_{y}, where valency permits;
R₄ is independently selected from the group consisting of hydrogen, halogen, -C₁₋₃ alkyl, -C₁₋₃ haloalkyl, -C₁₋₃ alkoxy, cyano, hydroxyl, amino, amido, sulfonyl, and sulfonamido;
R₅ is independently selected from the group consisting of hydrogen, halogen, hydroxy, -CN, -C₁₋₃ alkyl, -C₁₋₃ alkoxy, and -C₁₋₃ cycloalkyl, wherein the alkyl, alkoxy, and cycloalkyl in R₅ may be optionally substituted 1 to 3 times by halogen, hydroxyl, -NR_{z}, -CN, -C₁₋₃ alkyl, -C₁₋₃ alkoxy, -C₁₋₃ cycloalkyl, where valency permits;
R₆ is selected from the group consisting of -R_{z}, -O-R_{z}, -S-R_{z}, -C₁₋₃ alkyl, -C₁₋₃ alkylene-R_{z}, -C₀₋₃ alkylene-amino-R_{z}, -C₀₋₃ alkylene-carbonyl-R_{z}, -C₀₋₃ alkylene-amido-R_{z}, -Co-₃ alkylene-sulfonyl-R_{z}, -C₀₋₃ alkylene-phosphoryl-R_{z}, and -C₀₋₃ alkylene-sulfonamido-R_{z}, wherein the alkyl, amino, amido, sulfonyl, sulfonamido, and phosphoryl in R₆ may be optionally substituted 1 to 3 times by halogen or one time by R_{w}, where valency permits;
R₇ is selected from the group consisting of -COOH, -C(R_{y})ₙ₀-COOH, -N(R_{z})ₙ₀-COOH, -SOz-COOH, and -SO₂-NH-COOH, wherein the R_{y} in -C(R_{y})ₙ₀- may be attached to C in the form of a backbone and/or a side chain, the R_{z} in -N(R_{z})ₙ₀- may be attached to N in the form of a backbone and/or a side chain, wherein n₀ is an integer selected from 0, 1 or 2; when n₀ is 2, two R_{y} or R_{z} may be further cyclized into a 3- to 8-membered carbocyclic ring or heterocyclic ring;
n is an integer selected from 0, 1, 2 or 3;
o is an integer selected from 0, 1, 2, 3 or 4;
p is an integer selected from 0, 1, 2, 3 or 4;
when n is more than or equal to 2, any two R₁ may be further cyclized to form a 3- to 8-membered carbocyclic ring, aromatic ring, heterocyclic ring or heteroaromatic ring, wherein the formed carbocyclic ring and heterocyclic ring may be optionally substituted 1 to 3 times by C₁₋₃ alkyl, C₁₋₃ haloalkyl, halogen, cyano, C₁₋₃ alkoxy, where valence permits;
when p is more than or equal to 2, any two R₅ may be further cyclized with the ring C to form a 6- to 10-membered spiro ring or bridged ring, wherein the formed spiro ring and bridged ring may be optionally substituted 1 to 3 times by C₁₋₃ alkyl, C₁₋₃ haloalkyl, halogen, cyano, C₁₋₃ alkoxy, where valence permits;
when o is not 0 and p is not 0, any R₄ and R₅ may be further cyclized to form a 5- to 8-membered ring, wherein the formed ring may be optionally substituted 1 to 3 times by C₁₋₃ alkyl, C₁₋₃ haloalkyl, halogen, cyano, oxo, C₁₋₃ alkoxy, where valence permits;
R_{w} is independently selected from the group consisting of -CN, -CH₂CN, -C₁₋₃ alkyl, -OH, -C₁₋₃ alkoxy, amido, sulfonyl, sulfonamido, -NH₂, and -NH-C₁₋₃ alkyl, wherein the alkyl in R_{w} may be optionally substituted 1 to 3 times by C₁₋₃ alkyl, C₁₋₃ haloalkyl, halogen, cyano, C₁₋₃ alkoxy, where valence permits;
Rₓ is independently selected from the group consisting of hydrogen, halogen, oxo, C₁₋₆ alkoxy, cyano, hydroxyl, carboxyl, amino, amido, sulfonyl, sulfonamido, -C₁₋₆ alkyl, -C₂₋₆ alkenyl, -C₂₋₆ alkynyl, -C₃₋₆ cycloalkyl, 3- to 6-membered heterocyclyl, 6- to 8-membered aryl, and 5- to 8-membered heteroaryl, wherein the alkyl, alkoxy, cycloalkyl, heterocyclyl, aryl, and heteroaryl in Rₓ may be optionally substituted 1 to 3 times by halogen or optionally substituted 0 to 1 time by hydroxyl, where valence permits;
R_{y} is independently selected from the group consisting of hydrogen, halogen, oxo, -C₁₋₃ alkoxy, cyano, hydroxyl, amino, carboxyl, amido, sulfonyl, sulfonamido, -C₁₋₆ alkyl, -C₂₋₆ alkenyl, -C₂₋₆ alkynyl, C₃₋₆ cycloalkyl, and 3- to 6-membered heterocyclyl, wherein the alkyl, alkoxy, cycloalkyl, and heterocyclyl in R_{y} may be optionally substitute 1 to 3 times by halogen, where valence permits; and
R_{z} is independently selected from the group consisting of hydrogen, C₁₋₃ alkyl, C₁₋₃ alkoxy, C₃₋₆ cycloalkyl, 4- to 6-membered heterocyclyl, 5- to 6-membered aryl, or 5- to 6-membered heteroaryl, wherein R_{z} may be optionally substituted 1 to 3 times by halogen, cyano, C₁₋₃ alkyl, C₁₋₃ alkoxy, C₃₋₆ cycloalkyl, 3- to 6-membered heterocyclyl, where valence permits.

4. The compound of Formula I according to claim 1, wherein, when o is not 0 and p is not 0, any adjacent R₄ and R₅ may be further cyclized to form a 5- to 8-membered ring, wherein the 5- to 8-membered ring comprises C₅₋₆ carbocyclic ring, 5- to 8-membered heterocyclic ring, benzene ring, and 5- to 8-member heteroaromatic ring, and the formed ring can be optionally substituted 1 to 3 times by alkyl, haloalkyl, halogen, cyano, alkoxy, wherein valence permits;
wherein, when o is not 0 and p is not 0, any adjacent R₄ and R₅ may be further cyclized to form a 5- to 8-membered ring which may be selected from the group consisting of 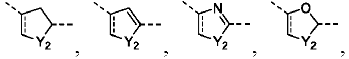 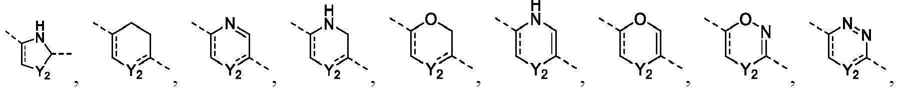 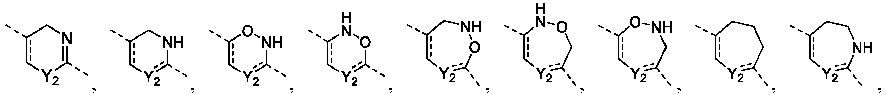 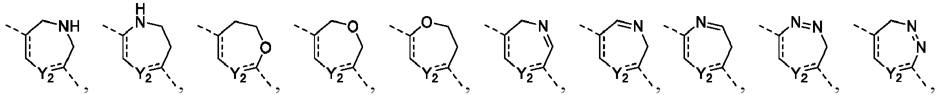 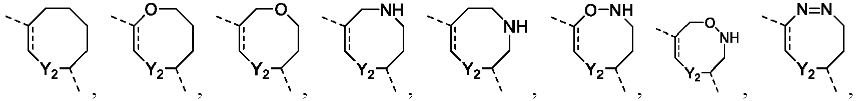 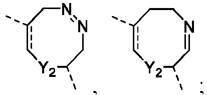 wherein the formed 5- to 8-membered ring may be optionally substituted 1 to 3 times by C₁₋₃ alkyl, C₁₋₃ haloalkyl, halogen, cyano, oxo, C₁₋₃ alkoxy, where valence permits;
and/or, when o is not 0 and p is not 0, any adjacent R₄ and R₅ may be further cyclized to form a 5- to 8-membered ring which is preferably 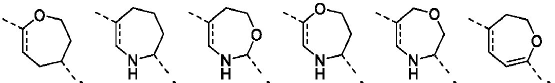 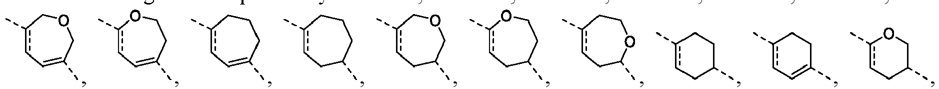 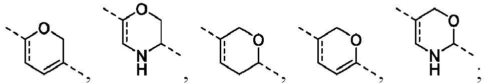 wherein the formed 5- to 8-membered ring may be optionally substituted 1 to 3 times by C₁₋₃ alkyl, C₁₋₃ haloalkyl, halogen, cyano, oxo, C₁₋₃ alkoxy, where valence permits;
and/or, when ο is not 0 and p is not 0, any adiacent R₄ and R₅ may be further cyclized to form a 5- to 8-membered ring which may be selected from the group consisting of 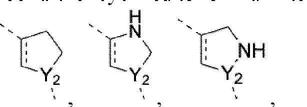 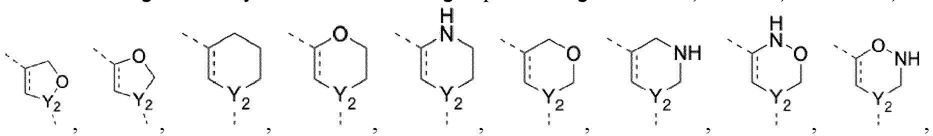 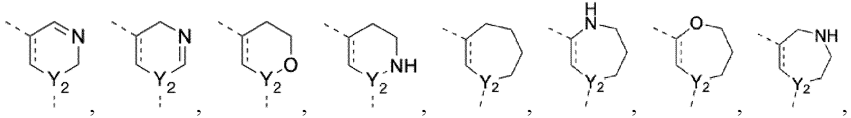 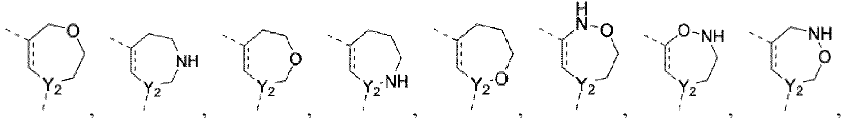 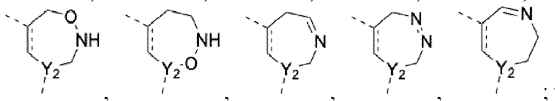 wherein the formed 5- to 8-membered ring may be optionally substituted 1 to 3 times by C₁₋₃ alkyl, C₁₋₃ haloalkyl, halogen, cyano, oxo, C₁₋₃ alkoxy, where valence permits.

5. The compound of Formula I according to claim 1, wherein the structural unit 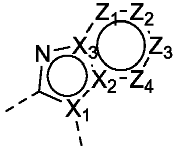 may be further selected from the group consisting of

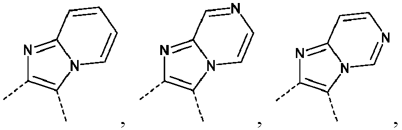

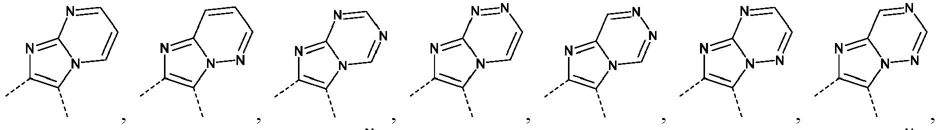

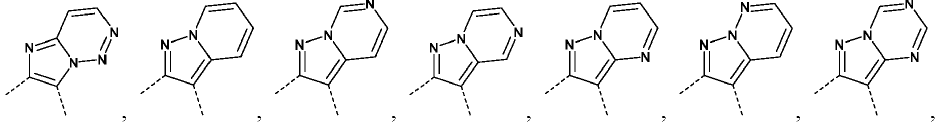

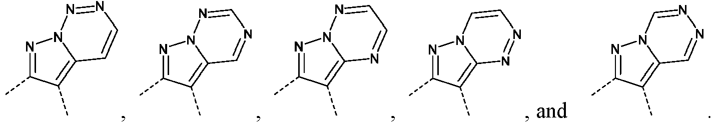

6. The compound of formula I according to claim 1 which may have the following subformula, 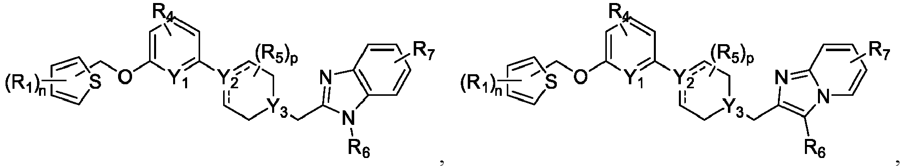 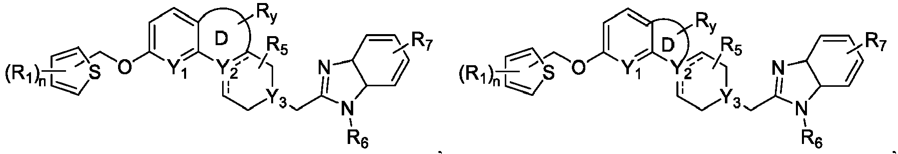 or

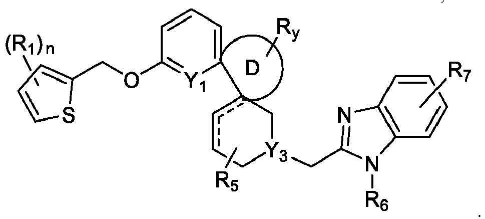

7. The compound of Formula I according to claim 1, wherein said n is selected from 1, 2 or 3; and/or said p is selected from 0, 1 or 2.

8. The compound of Formula I according to claim 1, wherein said R₁ may be further independently selected from the group consisting of -F, -Cl, -CN, -OCH₃, -OCH₂CH₃, -O-cyclopropyl, -CH₃, -CH₂CH₃, -CH₂CH₂CH₃, -(CH)₂CH₃, -COCH₃, -CONH₂, -CF₃, -CHF₂, -CH₂F, -CH₂CH₂F, -CO-cyclopropyl, 5- to 6-membered heterocyclyl, and 5- to 6-membered heteroaryl;
and/or, said R₃ may be further selected from the group consisting of -F, -Cl, -CH₃, -OCH₃, -NH₂, -OH, -CH₂CH₃, -CH₂OH, -NHCH₃, -COCH₃, -SO₂CH₃, -OCH₂CH₃, -CF₃, -CHF₂, -CH₂F, isopropyl, cyclopropyl, and fluorocyclopropyl;
and/or, said R₂ may be further independently selected from the group consisting of -H, -CH₃, -CHF₂, -CH₂F, -CF₃, -CH₂CH₃, -CH₂CH₂F, -NH₂, cyclopropyl, 5- to 6-membered heterocyclyl, and 5- to 6-membered heteroaryl;
and/or, said R₄ may be further selected from the group consisting of -CN, -CH₃, -OH, -CH₂OH, -CH₂OCH₃, -OCH₃, -NH₂, -NHCH₃, -COCH₃, and -OCH₂CH₃;
and/or, said R₅ is selected from the group consisting of -F, -Cl, -CN, -CH₃, -CH₂CH₃, -CF₃, -CHF₂, -CH₂F, -CH₂OH, -OH, -CH₂OCH₃, -OCH₃, -CH₂CH₂OH, -CH₂CH₂OCH₃, isopropyl or cyclopropyl;
and/or, said R₆ is selected from the group consisting of -R_{z}, -O-R_{z}, -S-R_{z}, -C₁₋₃ alkylene-R_{z}, -C₀₋₃ alkylene-amino-R_{z}, and -C₀₋₃ alkylene-carbonyl-R_{z}, wherein the alkyl, amino, amido, sulfonyl, sulfonamido and phosphoryl in R₆ may be optionally substituted 1 to 3 times by halogen or one time by R_{w}, where valence permits;
and/or, R₇ is selected from the group consisting of -COOH, -CH₂COOH, -CH₂CH₂COOH, and -CH(CH₃)COOH, wherein said R₇ may be optionally substituted 1 to 3 times by halogen, where valence permits.

9. The compound of Formula I according to claim 1 wherein said R_{z} may be further selected from the group consisting of methyl, ethyl, isopropyl, cyclopropyl, cyclobutyl, methoxy, ethoxy. 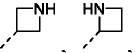 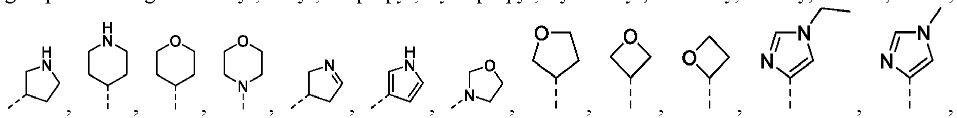 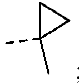 R_{z} may be optionally substituted 1 to 3 times by halogen, cyano, C₁₋₃ alkyl, C₁₋₃ alkoxy, C₃₋₆ cycloalkyl, 3- to 6-membered heterocyclyl, where valency permits;
and/or, said R_{y} may be further selected from the group consisting of -F, -Cl, methyl, ethyl, trifluoromethyl, difluoromethyl, fluoromethyl, fluoroethyl, methoxy, amino, hydroxy, propyl, isopropyl, cyclopropyl, and cyclobutyl.

10. The compound of Formula I according to claim 1 which is 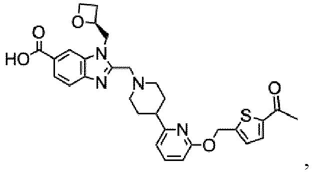 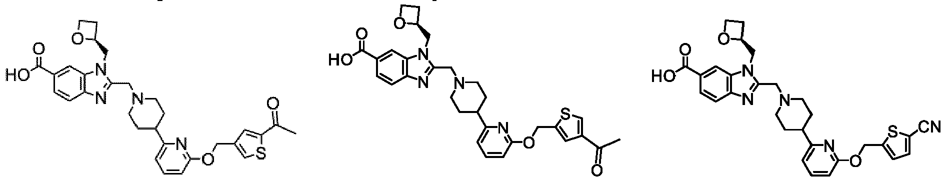 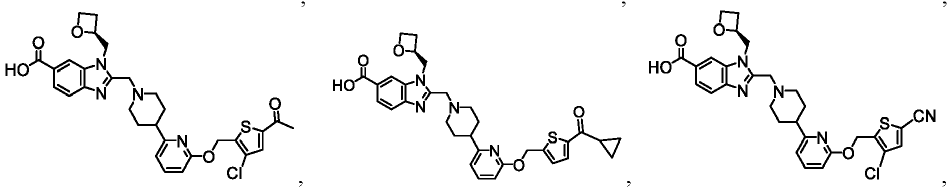 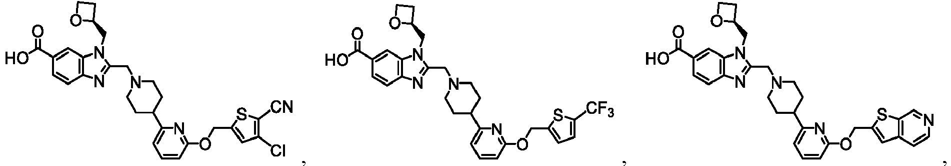 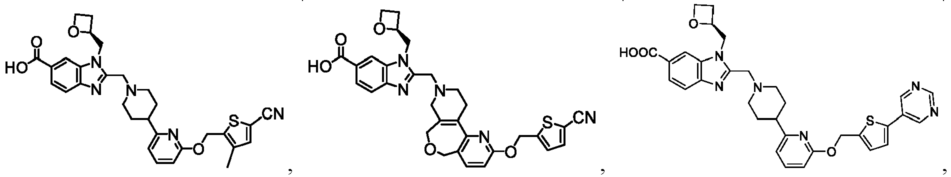 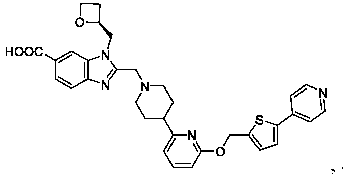 and a pharmaceutically acceptable salt thereof.

11. The compound of formula I according to claim 1 which is 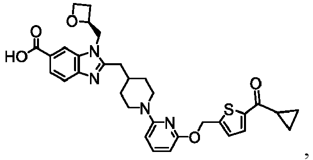 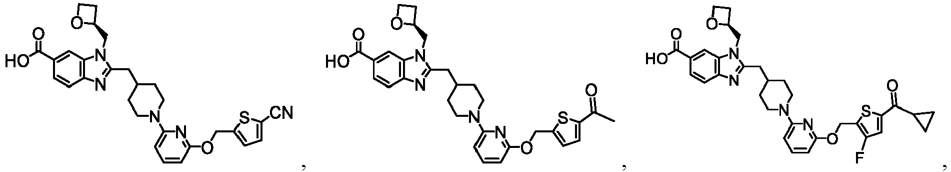 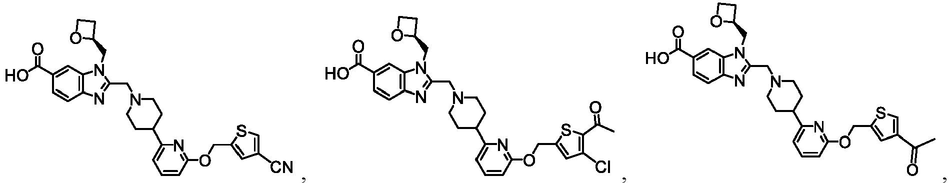 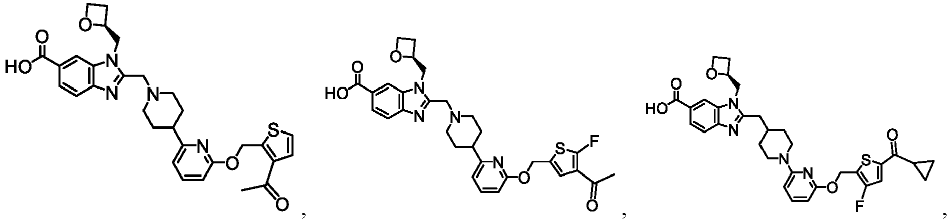 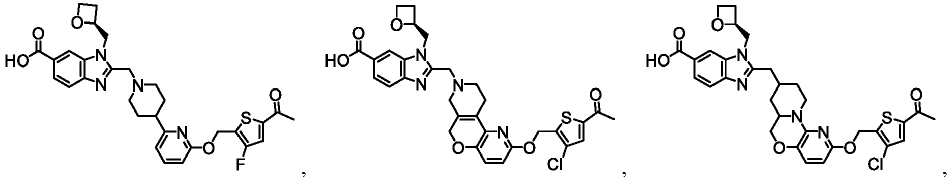 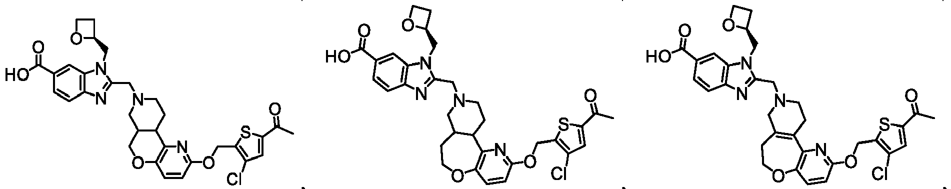 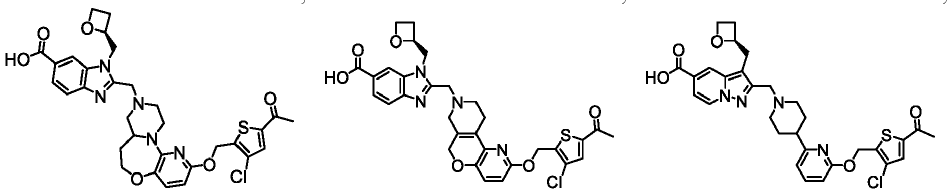 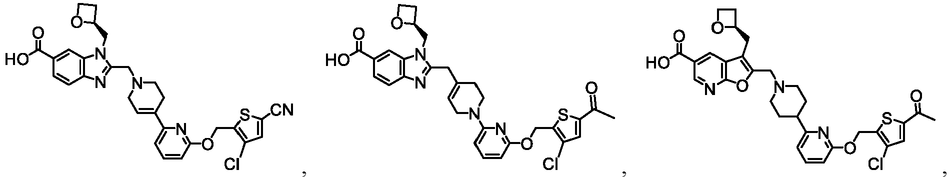 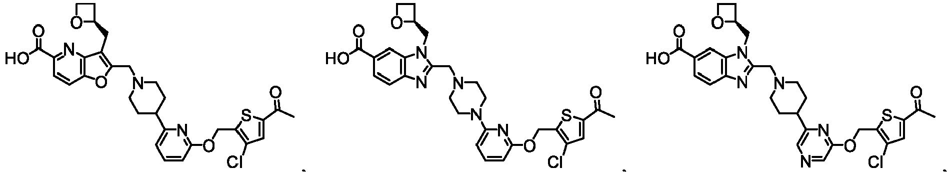 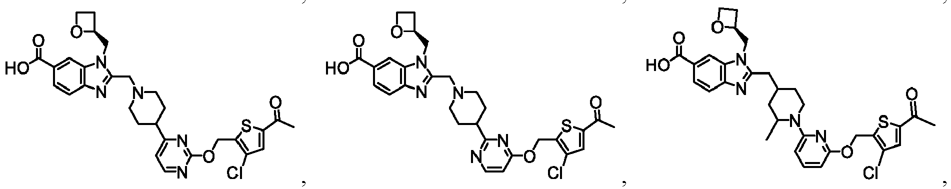 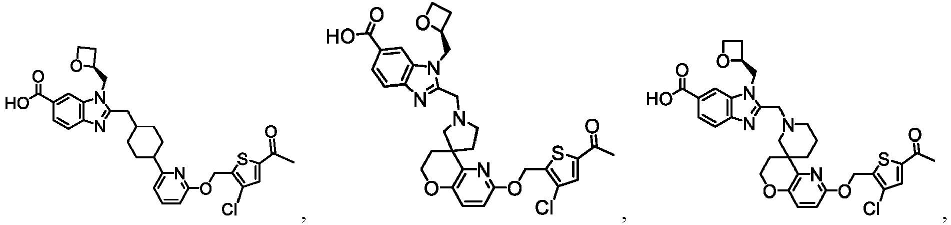 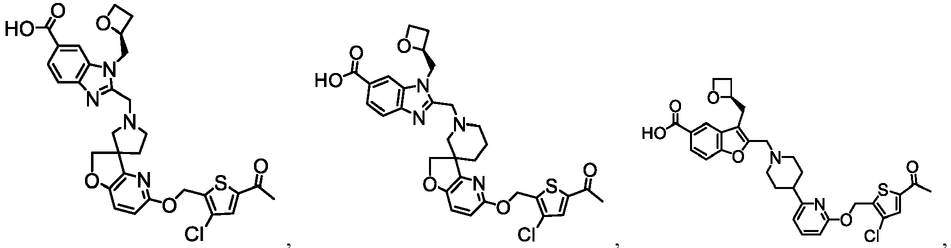 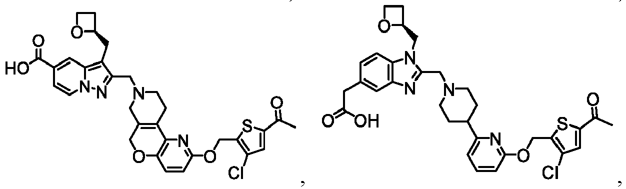 and a pharmaceutically acceptable salt thereof.

12. A pharmaceutical composition, comprising a compound of Formula I of any one of claims 1 to 11 or a pharmaceutically acceptable salt thereof, and a pharmaceutically acceptable pharmaceutical carrier.

13. Use of a compound of Formula I according to any one of claims 1 to 11 or a pharmaceutically acceptable salt thereof in the preparation of a medicament for the treatment of GLP-1 receptor agonist-mediated diseases or related diseases.

14. A method for preventing and/or treating GLP-1 mediated diseases or related diseases, comprising administering to a subject a therapeutically effective amount of a compound of Formula I according to any one of claims 1 to 11 or a pharmaceutically acceptable salt thereof, wherein the GLP-1 mediated diseases or related diseases include, but are not limited to diabetes, hyperglycemia, insulin resistance, glucose intolerance, diabetic nephropathy, diabetic neuropathy, diabetic retinopathy, adipocyte dysfunction, obesity, dyslipidemia, and hyperinsulinemia.
